# EUROPEAN PATENT APPLICATION

(11) **EP 4 063 386 A1**
(43) Date of publication of application: **28.09.2022**
(21) Application number: 20869539.5
(22) Date of filing: 28.09.2020
(51) Int. Cl.: C07K 16/28, A61K 39/395, C12N 15/63, A61P 35/00, G01N 33/574

(54) **ANTI-HUMAN CLAUDIN 18.2 ANTIBODY AND APPLICATION THEREOF**

(30) Priority: 29.09.2019 CN 201910929614
(71) Applicant: Mabwell (Shanghai) Bioscience Co., Ltd., Shanghai 201210 (CN)
(72) Inventor: LIN, Jian, Shanghai 201210 (CN); DENG, Xiaofang, Shanghai 201210 (CN); GAO, Pan, Shanghai 201210 (CN); XU, Xiaohong, Shanghai 201210 (CN); WANG, Lichun, Shanghai 201210 (CN); REN, Hongyuan, Shanghai 201210 (CN); BI, Jianjun, Shanghai 201210 (CN); WANG, Jin, Shanghai 201210 (CN)
(74) Representative: Schiweck Weinzierl Koch Patentanwälte Partnerschaft mbB
(86) International application number: PCT/CN2020/118424
(87) International publication number: WO 2021/058000

(57) **Abstract**

The present invention provides an antibody that binds to human Claudin 18.2 or a fragment thereof, as well as encoded nucleic acids and the like thereof. The anti-human Claudin 18.2 antibody of the present invention has strong affinity to an antigen Claudin 18.2 and significant complement-dependent cytotoxicity (CDC) activity and antibody-dependent cytotoxicity (ADCC) activity to target expression cells, and exhibits high specificity to human CLDN 18.2.

Sequence Listing

<110>  MABWELL (SHANGHAI) BIOSCIENCE CO., LTD.

<120>  ANTI-HUMAN CLAUDIN 18.2 ANTIBODY AND APPLICATION THEREOF

<130>  LC20210007P-AU

<150>  CN201910929614.0
<151>  2019-09-29

<160>  133

<170>  PatentIn version 3.3

<210>  1
<211>  119
<212>  PRT
<213>  Artificial

<220>
<223>  heavy chain variable region

<400>  1

Gln Val Gln Leu Lys Glu Ser Gly Pro Asp Leu Val Ala Pro Ser Gln
1               5                   10                  15

Ser Leu Ser Ile Thr Cys Thr Val Ser Gly Phe Ser Leu Thr Asn Tyr
            20                  25                  30

Gly Val His Trp Val Arg Gln Pro Pro Gly Lys Gly Leu Glu Trp Leu
        35                  40                  45

Val Val Ile Trp Ser Asp Gly Arg Ile Asn Tyr Asn Ser Ala Leu Lys
    50                  55                  60

Ser Arg Leu Ser Ile Thr Lys Asp Asn Ser Lys Arg Gln Val Phe Leu
65                  70                  75                  80

Lys Met Asn Ser Leu Gln Ile Asp Asp Thr Ala Ile Tyr Tyr Cys Val
                85                  90                  95

Arg His Pro Ala Phe Gly Pro His Ala Met Asp Tyr Trp Gly Gln Gly
            100                 105                 110

Ile Ser Val Thr Val Ser Ser
        115

<210>  2
<211>  112
<212>  PRT
<213>  Artificial

<220>

<223>  light chain variable region

<400>  2

Asp Ile Val Met Thr Gln Asp Ala Pro Ser Ile Pro Val Thr Pro Gly
1               5                   10                  15

Glu Ser Val Ser Ile Ser Cys Arg Ser Ser Lys Ser Leu Leu Asn Ser
            20                  25                  30

Asn Gly Asn Thr Tyr Leu Tyr Trp Phe Leu Gln Arg Pro Gly Gln Ser
        35                  40                  45

Pro His Leu Leu Leu Tyr Arg Met Ser Asn Pro Ala Ser Gly Ala Pro
    50                  55                  60

Asp Arg Phe Ser Gly Ser Gly Ser Gly Thr Glu Phe Thr Leu Arg Ile
65                  70                  75                  80

Ser Arg Val Glu Ala Glu Asp Val Gly Val Tyr Tyr Cys Met Gln Tyr
                85                  90                  95

Leu Glu Tyr Pro Pro Thr Phe Gly Ala Gly Thr Arg Leu Glu Leu Lys
            100                 105                 110

<210>  3
<211>  117
<212>  PRT
<213>  Artificial

<220>
<223>  heavy chain variable region

<400>  3

Glu Val Met Leu Val Glu Ser Gly Gly Gly Leu Val Arg Pro Gly Gly
1               5                   10                  15

Ser Leu Lys Leu Ser Cys Ala Gly Ser Gly Ile Thr Leu Ser Thr Tyr
            20                  25                  30

Ala Met Ser Trp Val Arg Gln Thr Pro Glu Arg Arg Leu Glu Trp Val
        35                  40                  45

Ala Ser Ile Ile Ser Gly Gly Ile Thr Tyr Tyr Leu Asp Ser Val Lys
    50                  55                  60

Gly Arg Phe Thr Ile Ser Arg Asp Asn Ala Arg Asn Ile Leu Tyr Leu
65                  70                  75                  80

Gln Met Ser Ser Leu Arg Ser Glu Asp Thr Ala Ile Tyr Tyr Cys Ala

85                  90                  95

Arg Lys Tyr His Gly Asn Ala Leu Asp Tyr Trp Gly Gln Gly Thr Ser
            100                 105                 110

Val Thr Val Ser Leu
        115

<210>  4
<211>  113
<212>  PRT
<213>  Artificial

<220>
<223>  light chain variable region

<400>  4

Asp Ile Val Met Thr Gln Ser Pro Ser Ser Leu Pro Val Thr Ala Gly
1               5                   10                  15

Glu Thr Val Thr Met Arg Cys Lys Ser Ser Gln Ser Leu Leu Asn Ser
            20                  25                  30

Gly Asn Gln Arg Asn Tyr Leu Thr Trp Tyr Gln Arg Lys Pro Gly Gln
        35                  40                  45

Pro Pro Lys Lys Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Gly Val Gln Ala Glu Asp Leu Ala Val Tyr Tyr Cys Gln Asn
                85                  90                  95

Asn Tyr Phe Tyr Pro Leu Thr Phe Gly Ala Gly Thr Lys Leu Glu Leu
            100                 105                 110

Lys

<210>  5
<211>  118
<212>  PRT
<213>  Artificial

<220>
<223>  heavy chain variable region

<400>  5

Gln Ile Gln Met Val Gln Ser Gly Pro Glu Leu Lys Lys Pro Gly Glu
1               5                   10                  15

Thr Val Arg Ile Ser Cys Lys Ala Ser Gly Tyr Ser Phe Thr Thr Ala
            20                  25                  30

Gly Met Gln Trp Val Arg Lys Met Pro Gly Glu Gly Leu Lys Trp Ile
        35                  40                  45

Gly Trp Ile Ile Ala His Ser Gly Glu Pro Lys Tyr Thr Glu Asp Phe
    50                  55                  60

Lys Gly Arg Phe Ala Phe Ser Leu Glu Thr Ser Ala Ser Thr Thr Tyr
65                  70                  75                  80

Leu Gln Ile Ser Asn Leu Lys Asn Glu Asp Thr Ala Thr Tyr Phe Cys
                85                  90                  95

Ala Arg Trp Gly Lys Gly Asn Thr Met Asp Tyr Trp Gly Gln Gly Thr
            100                 105                 110

Ser Val Ile Val Ser Ser
        115

<210>  6
<211>  113
<212>  PRT
<213>  Artificial

<220>
<223>  light chain variable region

<400>  6

Asp Ile Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Gly
            20                  25                  30

Gly Asn Gln Arg Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr His Phe Thr Leu Thr
65                  70                  75                  80

Ile Ser Ser Val Gln Ala Glu Asp Leu Ala Val Tyr Tyr Cys Gln Asn

85                  90                  95

Ala Tyr Phe Phe Pro Leu Thr Phe Gly Ala Gly Thr Lys Leu Glu Leu
            100                 105                 110

Lys

<210>  7
<211>  119
<212>  PRT
<213>  Artificial

<220>
<223>  heavy chain variable region

<400>  7

Asp Val Gln Leu Val Glu Ser Gly Gly Gly Leu Val Gln Pro Gly Gly
1               5                   10                  15

Ser Arg Lys Leu Ser Cys Ala Ala Ser Gly Phe Thr Phe Ser Ser Phe
            20                  25                  30

Gly Met His Trp Val Arg Gln Ala Pro Glu Lys Gly Leu Glu Trp Val
        35                  40                  45

Ala Tyr Ile Ser Ser Gly Ser Ser Thr Ile Tyr Tyr Pro Asp Thr Val
    50                  55                  60

Lys Gly Arg Phe Thr Val Ser Arg Asp Asn Pro Lys Asn Thr Leu Phe
65                  70                  75                  80

Leu Gln Met Thr Ser Leu Arg Ser Glu Asp Thr Ala Met Tyr Tyr Cys
                85                  90                  95

Val Arg Leu Gly Pro Arg Gly Asn Val Met Asp His Trp Gly Gln Gly
            100                 105                 110

Thr Ser Val Thr Val Ser Ser
        115

<210>  8
<211>  113
<212>  PRT
<213>  Artificial

<220>
<223>  light chain variable region

<400>  8

Asp Ile Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Arg Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Val Tyr Phe Tyr Pro Leu Thr Phe Gly Thr Gly Thr Lys Leu Glu Leu
            100                 105                 110

Arg

<210>  9
<211>  118
<212>  PRT
<213>  Artificial

<220>
<223>  heavy chain variable region

<400>  9

Asp Val Gln Leu Gln Glu Ser Gly Pro Asp Leu Val Lys Pro Ser Gln
1               5                   10                  15

Ser Leu Ser Leu Thr Cys Thr Val Ser Gly Tyr Ser Ile Ser Gly Ala
            20                  25                  30

Tyr Asn Trp His Trp Ile Arg Gln Phe Pro Gly Asn Lys Leu Glu Trp
        35                  40                  45

Leu Ala Tyr Met Gln Tyr Ser Gly Ser Ser Asn Tyr Asn Pro Ser Phe
    50                  55                  60

Lys Ser Arg Ile Ser Ile Ser Arg Asp Thr Ser Lys Asn Gln Phe Phe
65                  70                  75                  80

Leu Gln Leu Lys Ser Val Thr Thr Glu Asp Thr Ala Thr Tyr Tyr Cys

85                  90                  95

Ala Arg Met Tyr Asn Gly Asn Ser Phe Leu Tyr Trp Gly Gln Gly Thr
            100                 105                 110

Leu Val Thr Val Ser Ala
        115

<210>  10
<211>  113
<212>  PRT
<213>  Artificial

<220>
<223>  light chain variable region

<400>  10

Asp Ile Val Met Thr Gln Ser Pro Ser Ser Leu Thr Val Thr Ala Gly
1               5                   10                  15

Glu Lys Val Thr Met Asn Cys Lys Ser Ser Gln Ser Leu Phe Asn Ser
            20                  25                  30

Gly Asn Gln Lys Asn Tyr Leu Thr Trp Tyr Gln Gln Lys Pro Gly Gln
        35                  40                  45

Pro Pro Arg Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ser Leu Tyr Tyr Cys Gln Asn
                85                  90                  95

Ser Tyr Ser Tyr Pro Leu Thr Phe Gly Ala Gly Thr Lys Leu Glu Leu
            100                 105                 110

Lys

<210>  11
<211>  119
<212>  PRT
<213>  Artificial

<220>
<223>  heavy chain variable region

<400>  11

Gln Val Gln Leu Lys Glu Ser Gly Pro Asp Leu Val Ala Pro Ser Gln
1               5                   10                  15

Ser Leu Ser Leu Thr Cys Ser Val Ser Gly Phe Ser Leu Thr Ser Tyr
            20                  25                  30

Gly Ile His Trp Val Arg Gln Pro Pro Gly Lys Gly Leu Glu Trp Leu
        35                  40                  45

Val Val Ile Trp Ser Asp Gly Arg Thr Thr Tyr Asn Ser Gly Leu Lys
    50                  55                  60

Ser Arg Leu Ser Ile Ser Lys Asp Asn Ser Lys Ser Gln Val Leu Leu
65                  70                  75                  80

Lys Met Asn Ser Leu Arg Thr Asp Asp Thr Ala Ile Tyr Tyr Cys Val
                85                  90                  95

Arg His Pro Ala Phe Gly Pro His Ala Met Asp Tyr Trp Gly Gln Gly
            100                 105                 110

Thr Ser Val Thr Val Ser Ser
        115

<210>  12
<211>  112
<212>  PRT
<213>  Artificial

<220>
<223>  light chain variable region

<400>  12

Asp Ile Val Met Thr Gln Ala Ala Pro Ser Val Pro Val Thr Pro Gly
1               5                   10                  15

Glu Ser Val Ser Ile Ser Cys Arg Ser Ser Lys Ser Leu Leu Asn Ser
            20                  25                  30

Asn Gly Asn Thr Tyr Leu Tyr Trp Phe Leu Gln Arg Pro Gly Gln Ser
        35                  40                  45

Pro Gln Leu Leu Ile Tyr Arg Met Ser Asn Leu Ala Ser Gly Val Pro
    50                  55                  60

Asp Arg Phe Ser Gly Ser Gly Ser Gly Thr Asp Phe Thr Leu Arg Ile
65                  70                  75                  80

Ser Arg Val Glu Ala Gly Asp Val Gly Val Tyr Tyr Cys Met Gln Tyr

85                  90                  95

Leu Glu Tyr Pro Val Thr Phe Gly Ala Gly Thr Lys Leu Glu Leu Lys
            100                 105                 110

<210>  13
<211>  119
<212>  PRT
<213>  Artificial

<220>
<223>  heavy chain variable region

<400>  13

Asp Val Gln Leu Val Glu Ser Gly Gly Gly Leu Val Gln Pro Gly Gly
1               5                   10                  15

Ser Arg Lys Leu Ser Cys Ala Ala Ser Gly Phe Thr Phe Ser Arg Phe
            20                  25                  30

Gly Met His Trp Val Arg Gln Ala Pro Lys Lys Gly Leu Glu Trp Val
        35                  40                  45

Ala Tyr Ile Ser Ser Gly Ser Asn Thr Ile Tyr Tyr Ala Asp Thr Val
    50                  55                  60

Lys Gly Arg Phe Thr Ile Ser Arg Asp Asn Pro Lys Asn Thr Leu Phe
65                  70                  75                  80

Leu Gln Thr Thr Ser Leu Arg Ser Glu Asp Thr Ala Ile Tyr Tyr Cys
                85                  90                  95

Gly Arg Leu Gly Phe Tyr Gly Asn Ser Phe Asp His Trp Gly Gln Gly
            100                 105                 110

Thr Leu Val Thr Val Ser Ala
        115

<210>  14
<211>  113
<212>  PRT
<213>  Artificial

<220>
<223>  light chain variable region

<400>  14

Asn Ile Leu Met Thr Gln Ser Pro Ser Ser Leu Thr Val Thr Ala Gly
1               5                   10                  15

Glu Lys Val Thr Met Asn Cys Lys Ser Ser Gln Ser Leu Leu Asn Gly
            20                  25                  30

Gly Asn Gln Arg Asn Tyr Leu Thr Trp Tyr Gln Gln Lys Ala Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Gly Gly Ser Gly Thr Asp Phe Thr Leu Thr
65                  70                  75                  80

Ile Ser Ser Val Gln Ala Glu Asp Leu Ala Leu Tyr Tyr Cys Gln Asn
                85                  90                  95

Ser Tyr Tyr Tyr Pro Leu Thr Phe Gly Ala Gly Thr Lys Leu Glu Leu
            100                 105                 110

Lys

<210> 15
<211> 118
<212> PRT
<213> Artificial

<220>
<223> heavy chain variable region

<400> 15

Glu Val Gln Leu Arg Gln Ser Gly Pro Glu Leu Val Lys Pro Gly Ala
1               5                   10                  15

Ser Val Lys Met Ser Cys Lys Ala Ser Gly Tyr Thr Phe Thr Thr Tyr
            20                  25                  30

Ile Ile Asn Trp Val Lys Gln Lys Pro Gly Gln Gly Leu Glu Trp Ile
        35                  40                  45

Gly Tyr Ile Asn Pro Tyr Asn Asp Asp Thr Arg Tyr Asn Glu Arg Val
    50                  55                  60

Lys Gly Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Tyr Cys
                85                  90                  95

Ala Arg Phe Tyr Phe Gly Asn Ser Phe Thr Tyr Trp Gly Gln Gly Thr

            100                 105                 110

Leu Val Thr Val Ser Ala
        115

<210> 16
<211> 113
<212> PRT
<213> Artificial

<220>
<223> light chain variable region

<400> 16

Asp Ile Val Met Thr Gln Ser Pro Ser Ser Leu Pro Val Thr Val Gly
1               5                   10                  15

Glu Arg Val Thr Met Thr Cys Lys Ser Ser Gln Gly Leu Phe Asn Ser
            20                  25                  30

Gly Asn Gln Arg Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Ile Tyr Tyr Cys Gln Asn
                85                  90                  95

Asn Tyr Ile Tyr Pro Leu Thr Phe Gly Ala Gly Thr Lys Leu Glu Leu
            100                 105                 110

Lys

<210> 17
<211> 118
<212> PRT
<213> Artificial

<220>
<223> heavy chain variable region

<400> 17

Glu Val Gln Leu Arg Gln Ser Gly Pro Glu Leu Val Lys Pro Gly Ala
1               5                   10                  15

Ser Val Lys Met Ser Cys Lys Ala Ser Gly Tyr Thr Phe Thr Thr Tyr
            20                  25                  30

Ile Ile Asn Trp Val Lys Gln Lys Pro Gly Gln Gly Leu Glu Trp Ile
        35                  40                  45

Gly Tyr Ile Asn Pro Tyr Asn Asp Gly Thr Arg Tyr Asn Glu Arg Val
    50                  55                  60

Lys Gly Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Tyr Cys
                85                  90                  95

Ala Arg Phe His Phe Gly Asn Ser Phe Thr Tyr Trp Gly Gln Gly Thr
            100                 105                 110

Leu Val Thr Val Ser Ala
        115

<210>  18
<211>  113
<212>  PRT
<213>  Artificial

<220>
<223>  light chain variable region

<400>  18

Asp Ile Val Met Thr Gln Ser Pro Ser Ser Leu Pro Val Thr Thr Gly
1               5                   10                  15

Glu Lys Val Thr Met Thr Cys Lys Ser Ser Gln Gly Leu Phe Asn Asn
            20                  25                  30

Gly Asn Gln Arg Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ile Gly Ser Gly Ser Gly Thr Asp Phe Thr Leu Thr
65                  70                  75                  80

Ile Ser Ser Val Gln Ala Glu Asp Leu Ala Ile Tyr Tyr Cys Gln Asn
                85                  90                  95

Asn Tyr Ile Phe Pro Leu Thr Phe Gly Ala Gly Thr Lys Leu Glu Leu

            100                 105                 110

Lys

<210> 19
<211> 118
<212> PRT
<213> Artificial

<220>
<223> heavy chain variable region

<400> 19

Gln Ile Gln Leu Val Gln Ser Gly Pro Glu Leu Lys Lys Pro Gly Glu
1               5                   10                  15

Thr Val Lys Ile Ser Cys Lys Ala Ser Gly Tyr Thr Leu Thr Asn Tyr
            20                  25                  30

Gly Met Asn Trp Val Arg Gln Ala Pro Gly Lys Gly Leu Lys Trp Met
        35                  40                  45

Gly Trp Ile Arg Pro Asn Thr Gly Glu Pro Thr Tyr Ala Glu Asp Phe
    50                  55                  60

Lys Gly Arg Phe Val Phe Ser Leu Glu Thr Ser Ala Ala Thr Ala Tyr
65                  70                  75                  80

Leu Gln Ile Thr Asn Leu Lys Ser Glu Asp Thr Ser Thr Tyr Phe Cys
                85                  90                  95

Ala Arg Leu Tyr Arg Gly Asn Thr Leu Asp Asn Trp Gly Gln Gly Thr
            100                 105                 110

Ser Val Ile Val Ser Ser
        115

<210> 20
<211> 113
<212> PRT
<213> Artificial

<220>
<223> light chain variable region

<400> 20

Asp Ile Val Met Thr Gln Ser Pro Ser Ser Leu Thr Val Thr Thr Gly
1               5                   10                  15

Glu Lys Val Thr Met Ser Cys Lys Ser Ser Gln Asn Leu Leu Asn Ser
            20                  25                  30

Gly Asn Gln Arg Asn Tyr Leu Thr Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Tyr Arg Phe Thr Gly Ser Gly Ser Gly Thr Asp Phe Thr Leu Thr
65                  70                  75                  80

Ile Ser Ser Val Gln Thr Asp Asp Leu Ala Ile Tyr Tyr Cys Gln Asn
                85                  90                  95

Gly Tyr Ser Phe Pro Phe Thr Phe Gly Ser Gly Thr Lys Leu Glu Ile
            100                 105                 110

Lys

<210> 21
<211> 118
<212> PRT
<213> Artificial

<220>
<223> heavy chain variable region

<400> 21

Gln Val His Leu Gln Gln Ser Gly Ala Glu Leu Val Arg Pro Gly Ser
1               5                   10                  15

Ser Val Lys Ile Ser Cys Lys Ala Ser Gly Tyr Ala Phe Ser Asn Tyr
            20                  25                  30

Trp Met Asn Trp Val Arg Gln Arg Pro Gly Gln Gly Leu Glu Trp Ile
        35                  40                  45

Gly Gln Ile Tyr Pro Gly Asn Gly Asp Thr Lys Tyr Ser Gly Lys Phe
    50                  55                  60

Asn Ser Lys Asp Thr Leu Thr Ala Asp Lys Ser Ser Asn Thr Ala Tyr
65                  70                  75                  80

Met Gln Leu Asn Ser Leu Thr Ser Glu Asp Ser Ala Val Tyr Phe Cys
                85                  90                  95

Ala Arg Phe Tyr Tyr Gly Asn Val Met Asp Tyr Trp Gly Gln Gly Thr

            100                 105                 110

Ser Val Thr Val Ser Ser
        115

<210> 22
<211> 113
<212> PRT
<213> Artificial

<220>
<223> light chain variable region

<400> 22

Asp Ile Val Leu Thr Gln Ser Pro Ser Ser Leu Thr Val Thr Ala Gly
1               5                   10                  15

Glu Lys Val Thr Met Ser Cys Lys Ser Ser Gln Thr Leu Leu Asn Gly
            20                  25                  30

Gly Asn Gln Lys Asn Tyr Leu Thr Trp Tyr Gln Gln Lys Ser Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Gly Tyr Ser Tyr Pro Leu Thr Phe Gly Val Gly Thr Lys Leu Glu Leu
            100                 105                 110

Lys

<210> 23
<211> 117
<212> PRT
<213> Artificial

<220>
<223> heavy chain variable region

<400> 23

Glu Val Gln Leu Val Glu Ser Gly Gly Gly Leu Val Gln Pro Gly Gly
1               5                   10                  15

Ser Leu Arg Leu Ser Cys Ala Gly Ser Gly Ile Thr Leu Ser Thr Tyr
            20                  25                  30

Ala Met Ser Trp Val Arg Gln Ala Pro Gly Lys Gly Leu Glu Trp Val
        35                  40                  45

Ser Ser Ile Ile Ser Gly Gly Ile Thr Tyr Tyr Leu Asp Ser Val Lys
    50                  55                  60

Gly Arg Phe Thr Ile Ser Arg Asp Asn Ala Lys Asn Thr Leu Tyr Leu
65                  70                  75                  80

Gln Met Ser Ser Leu Arg Ala Glu Asp Thr Ala Val Tyr Tyr Cys Ala
                85                  90                  95

Arg Lys Tyr His Gly Asn Ala Leu Asp Tyr Trp Gly Gln Gly Thr Leu
            100                 105                 110

Val Thr Val Ser Ser
        115

<210> 24
<211> 113
<212> PRT
<213> Artificial

<220>
<223> light chain variable region

<400> 24

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Ser Ser
            20                  25                  30

Gly Asn Gln Arg Asn Tyr Leu Thr Trp Tyr Gln Gln Lys Pro Gly Gln
        35                  40                  45

Pro Pro Lys Lys Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Leu Ala Val Tyr Tyr Cys Gln Asn
                85                  90                  95

Asn Tyr Phe Tyr Pro Leu Thr Phe Gly Gln Gly Thr Lys Leu Glu Ile

            100                 105                 110

Lys

<210> 25
<211> 113
<212> PRT
<213> Artificial

<220>
<223> light chain variable region

<400> 25

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Ser Ser
            20                  25                  30

Gly Asn Gln Arg Asn Tyr Leu Thr Trp Tyr Gln Gln Lys Pro Gly Gln
        35                  40                  45

Pro Pro Lys Lys Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Leu Ala Val Tyr Tyr Cys Gln Ser
                85                  90                  95

Asn Tyr Phe Tyr Pro Leu Thr Phe Gly Gln Gly Thr Lys Leu Glu Ile
            100                 105                 110

Lys

<210> 26
<211> 113
<212> PRT
<213> Artificial

<220>
<223> light chain variable region

<400> 26

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Ser Ser
            20                  25                  30

Gly Asn Gln Arg Asn Tyr Leu Thr Trp Tyr Gln Gln Lys Pro Gly Gln
        35                  40                  45

Pro Pro Lys Lys Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Leu Ala Val Tyr Tyr Cys Gln Gln
                85                  90                  95

Asn Tyr Phe Tyr Pro Leu Thr Phe Gly Gln Gly Thr Lys Leu Glu Ile
            100                 105                 110

Lys

<210> 27
<211> 118
<212> PRT
<213> Artificial

<220>
<223> heavy chain variable region

<400> 27

Gln Ile Gln Leu Val Gln Ser Gly Ser Glu Leu Lys Lys Pro Gly Ala
1               5                   10                  15

Ser Val Lys Val Ser Cys Lys Ala Ser Gly Tyr Thr Leu Thr Asn Tyr
            20                  25                  30

Gly Met Asn Trp Val Arg Gln Ala Pro Gly Gln Gly Leu Glu Trp Met
        35                  40                  45

Gly Trp Ile Arg Pro Asn Thr Gly Glu Pro Thr Tyr Ala Glu Asp Phe
    50                  55                  60

Lys Gly Arg Phe Val Phe Ser Leu Asp Thr Ser Val Ala Thr Ala Tyr
65                  70                  75                  80

Leu Gln Ile Thr Ser Leu Lys Ala Glu Asp Thr Ala Val Tyr Tyr Cys
                85                  90                  95

Ala Arg Leu Tyr Arg Gly Asn Thr Leu Asp Asn Trp Gly Gln Gly Thr

            100                 105                 110

Leu Val Thr Val Ser Ser
        115

<210> 28
<211> 113
<212> PRT
<213> Artificial

<220>
<223> light chain variable region

<400> 28

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Asn Leu Leu Ser Ser
            20                  25                  30

Gly Asn Gln Arg Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Leu Ala Val Tyr Tyr Cys Gln Gln
                85                  90                  95

Gly Tyr Ser Phe Pro Phe Thr Phe Gly Gln Gly Thr Lys Leu Glu Ile
            100                 105                 110

Lys

<210> 29
<211> 118
<212> PRT
<213> Artificial

<220>
<223> heavy chain variable region

<400> 29

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Ala Phe Ser Asn Tyr
            20                  25                  30

Trp Met Asn Trp Val Arg Gln Ala Pro Gly Gln Gly Leu Glu Trp Met
        35                  40                  45

Gly Gln Ile Tyr Pro Gly Ser Gly Asp Thr Lys Tyr Ser Gly Lys Phe
    50                  55                  60

Gln Ser Arg Val Thr Ile Thr Ala Asp Lys Ser Thr Asn Thr Ala Tyr
65                  70                  75                  80

Met Glu Leu Ser Ser Leu Arg Ser Glu Asp Thr Ala Val Tyr Tyr Cys
                85                  90                  95

Ala Arg Phe Tyr Tyr Gly Asn Val Met Asp Tyr Trp Gly Gln Gly Thr
            100                 105                 110

Leu Val Thr Val Ser Ser
        115

<210>  30
<211>  113
<212>  PRT
<213>  Artificial

<220>
<223>  light chain variable region

<400>  30

Asp Ile Val Leu Thr Gln Ser Pro Asp Ser Leu Ala Val Ser Leu Gly
1               5                   10                  15

Glu Arg Ala Thr Ile Asn Cys Lys Ser Ser Gln Thr Leu Leu Ser Gly
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Leu Ala Val Tyr Tyr Cys Gln Gln
                85                  90                  95

Gly Tyr Ser Tyr Pro Leu Thr Phe Gly Gln Gly Thr Lys Leu Glu Ile

100                 105                 110

Lys

<210>  31
<211>  10
<212>  PRT
<213>  Artificial

<220>
<223>  heavy chain, CDR1

<400>  31

Gly Phe Ser Leu Thr Asn Tyr Gly Val His
1               5                   10

<210>  32
<211>  17
<212>  PRT
<213>  Artificial

<220>
<223>  heavy chain, CDR2

<400>  32

Val Val Ile Trp Ser Asp Gly Arg Ile Asn Tyr Asn Ser Ala Leu Lys
1               5                   10                  15

Ser

<210>  33
<211>  9
<212>  PRT
<213>  Artificial

<220>
<223>  heavy chain, CDR3

<400>  33

Ala Phe Gly Pro His Ala Met Asp Tyr
1               5

<210>  34
<211>  16
<212>  PRT
<213>  Artificial

<220>
<223>  light chain, CDR1

<400>  34

Arg Ser Ser Lys Ser Leu Leu Asn Ser Asn Gly Asn Thr Tyr Leu Tyr
1               5                   10                  15

<210>  35
<211>  7
<212>  PRT
<213>  Artificial

<220>
<223>  light chain, CDR2

<400>  35

Arg Met Ser Asn Pro Ala Ser
1               5

<210>  36
<211>  9
<212>  PRT
<213>  Artificial

<220>
<223>  light chain, CDR3

<400>  36

Met Gln Tyr Leu Glu Tyr Pro Pro Thr
1               5

<210>  37
<211>  10
<212>  PRT
<213>  Artificial

<220>
<223>  heavy chain, CDR1

<400>  37

Gly Ile Thr Leu Ser Thr Tyr Ala Met Ser
1               5                   10

<210>  38
<211>  16
<212>  PRT
<213>  Artificial

<220>
<223>  heavy chain, CDR2

<400>  38

Ser Ile Ile Ser Gly Gly Ile Thr Tyr Tyr Leu Asp Ser Val Lys Gly
1               5                   10                  15

<210>  39
<211>  9
<212>  PRT

<213>  Artificial

<220>
<223>  heavy chain, CDR3

<400>  39

Lys Tyr His Gly Asn Ala Leu Asp Tyr
1               5


<210>  40
<211>  17
<212>  PRT
<213>  Artificial

<220>
<223>  light chain, CDR1

<400>  40

Lys Ser Ser Gln Ser Leu Leu Asn Ser Gly Asn Gln Arg Asn Tyr Leu
1               5                   10                  15


Thr




<210>  41
<211>  7
<212>  PRT
<213>  Artificial

<220>
<223>  light chain, CDR2

<400>  41

Trp Ala Ser Thr Arg Glu Ser
1               5


<210>  42
<211>  9
<212>  PRT
<213>  Artificial

<220>
<223>  light chain, CDR3

<400>  42

Gln Asn Asn Tyr Phe Tyr Pro Leu Thr
1               5


<210>  43
<211>  10
<212>  PRT
<213>  Artificial

<220>

<223>  heavy chain, CDR1

<400>  43

Gly Tyr Ser Phe Thr Thr Ala Gly Met Gln
1               5                   10


<210>  44
<211>  18
<212>  PRT
<213>  Artificial

<220>
<223>  heavy chain, CDR2

<400>  44

Gly Trp Ile Ile Ala His Ser Gly Glu Pro Lys Tyr Thr Glu Asp Phe
1               5                   10                  15


Lys Gly



<210>  45
<211>  9
<212>  PRT
<213>  Artificial

<220>
<223>  heavy chain, CDR3

<400>  45

Trp Gly Lys Gly Asn Thr Met Asp Tyr
1               5


<210>  46
<211>  17
<212>  PRT
<213>  Artificial

<220>
<223>  light chain, CDR1

<400>  46

Lys Ser Ser Gln Ser Leu Leu Asn Gly Gly Asn Gln Arg Asn Tyr Leu
1               5                   10                  15


Thr



<210>  47
<211>  9
<212>  PRT
<213>  Artificial

<220>
<223>  light chain, CDR3

<400>  47

Gln Asn Ala Tyr Phe Phe Pro Leu Thr
1               5


<210>  48
<211>  10
<212>  PRT
<213>  Artificial

<220>
<223>  heavy chain, CDR1

<400>  48

Gly Phe Thr Phe Ser Ser Phe Gly Met His
1               5                   10


<210>  49
<211>  18
<212>  PRT
<213>  Artificial

<220>
<223>  heavy chain, CDR2

<400>  49

Ala Tyr Ile Ser Ser Gly Ser Ser Thr Ile Tyr Tyr Pro Asp Thr Val
1               5                   10                  15


Lys Gly



<210>  50
<211>  8
<212>  PRT
<213>  Artificial

<220>
<223>  heavy chain, CDR3

<400>  50

Pro Arg Gly Asn Val Met Asp His
1               5


<210>  51
<211>  9
<212>  PRT
<213>  Artificial

<220>
<223>  light chain, CDR3

<400>  51

Gln Asn Val Tyr Phe Tyr Pro Leu Thr
1               5

<210>  52
<211>  11
<212>  PRT
<213>  Artificial

<220>
<223>  heavy chain, CDR1

<400>  52

Gly Tyr Ser Ile Ser Gly Ala Tyr Asn Trp His
1               5                   10

<210>  53
<211>  17
<212>  PRT
<213>  Artificial

<220>
<223>  heavy chain, CDR2

<400>  53

Ala Tyr Met Gln Tyr Ser Gly Ser Ser Asn Tyr Asn Pro Ser Phe Lys
1               5                   10                  15

Ser

<210>  54
<211>  9
<212>  PRT
<213>  Artificial

<220>
<223>  heavy chain, CDR3

<400>  54

Met Tyr Asn Gly Asn Ser Phe Leu Tyr
1               5

<210>  55
<211>  17
<212>  PRT
<213>  Artificial

<220>
<223>  light chain, CDR1

<400>  55

Lys Ser Ser Gln Ser Leu Phe Asn Ser Gly Asn Gln Lys Asn Tyr Leu

1               5                   10                  15

Thr

<210>  56
<211>  9
<212>  PRT
<213>  Artificial

<220>
<223>  light chain, CDR3

<400>  56

Gln Asn Ser Tyr Ser Tyr Pro Leu Thr
1               5

<210>  57
<211>  10
<212>  PRT
<213>  Artificial

<220>
<223>  heavy chain, CDR1

<400>  57

Gly Phe Ser Leu Thr Ser Tyr Gly Ile His
1               5                   10

<210>  58
<211>  17
<212>  PRT
<213>  Artificial

<220>
<223>  heavy chain, CDR2

<400>  58

Val Val Ile Trp Ser Asp Gly Arg Thr Thr Tyr Asn Ser Gly Leu Lys
1               5                   10                  15

Ser

<210>  59
<211>  7
<212>  PRT
<213>  Artificial

<220>
<223>  light chain, CDR2

<400>  59

Arg Met Ser Asn Leu Ala Ser
1               5

<210>  60
<211>  9
<212>  PRT
<213>  Artificial

<220>
<223>  light chain, CDR3

<400>  60

Met Gln Tyr Leu Glu Tyr Pro Val Thr
1               5

<210>  61
<211>  10
<212>  PRT
<213>  Artificial

<220>
<223>  heavy chain, CDR1

<400>  61

Gly Phe Thr Phe Ser Arg Phe Gly Met His
1               5                   10

<210>  62
<211>  18
<212>  PRT
<213>  Artificial

<220>
<223>  heavy chain, CDR2

<400>  62

Ala Tyr Ile Ser Ser Gly Ser Asn Thr Ile Tyr Tyr Ala Asp Thr Val
1               5                   10                  15

Lys Gly

<210>  63
<211>  8
<212>  PRT
<213>  Artificial

<220>
<223>  heavy chain, CDR3

<400>  63

Phe Tyr Gly Asn Ser Phe Asp His
1               5

<210>  64
<211>  9
<212>  PRT
<213>  Artificial

<220>
<223>  light chain, CDR3

<400>  64

Gln Asn Ser Tyr Tyr Tyr Pro Leu Thr
1               5


<210>  65
<211>  10
<212>  PRT
<213>  Artificial

<220>
<223>  heavy chain, CDR1

<400>  65

Gly Tyr Thr Phe Thr Thr Tyr Ile Ile Asn
1               5                   10


<210>  66
<211>  18
<212>  PRT
<213>  Artificial

<220>
<223>  heavy chain, CDR2

<400>  66

Gly Tyr Ile Asn Pro Tyr Asn Asp Asp Thr Arg Tyr Asn Glu Arg Val
1               5                   10                  15


Lys Gly



<210>  67
<211>  9
<212>  PRT
<213>  Artificial

<220>
<223>  heavy chain, CDR3

<400>  67

Phe Tyr Phe Gly Asn Ser Phe Thr Tyr
1               5


<210>  68
<211>  17

<212>  PRT
<213>  Artificial

<220>
<223>  light chain, CDR1

<400>  68

Lys Ser Ser Gln Gly Leu Phe Asn Ser Gly Asn Gln Arg Asn Tyr Leu
1               5                   10                  15

Thr

<210>  69
<211>  9
<212>  PRT
<213>  Artificial

<220>
<223>  light chain, CDR3

<400>  69

Gln Asn Asn Tyr Ile Tyr Pro Leu Thr
1               5

<210>  70
<211>  18
<212>  PRT
<213>  Artificial

<220>
<223>  heavy chain, CDR2

<400>  70

Gly Tyr Ile Asn Pro Tyr Asn Asp Gly Thr Arg Tyr Asn Glu Arg Val
1               5                   10                  15

Lys Gly

<210>  71
<211>  9
<212>  PRT
<213>  Artificial

<220>
<223>  heavy chain, CDR3

<400>  71

Phe His Phe Gly Asn Ser Phe Thr Tyr
1               5

<210>  72

<211>  17
<212>  PRT
<213>  Artificial

<220>
<223>  light chain, CDR1

<400>  72

Lys Ser Ser Gln Gly Leu Phe Asn Asn Gly Asn Gln Arg Asn Tyr Leu
1               5                   10                  15

Thr

<210>  73
<211>  9
<212>  PRT
<213>  Artificial

<220>
<223>  light chain, CDR3

<400>  73

Gln Asn Asn Tyr Ile Phe Pro Leu Thr
1               5

<210>  74
<211>  10
<212>  PRT
<213>  Artificial

<220>
<223>  heavy chain, CDR1

<400>  74

Gly Tyr Thr Leu Thr Asn Tyr Gly Met Asn
1               5                   10

<210>  75
<211>  17
<212>  PRT
<213>  Artificial

<220>
<223>  heavy chain, CDR2

<400>  75

Trp Ile Arg Pro Asn Thr Gly Glu Pro Thr Tyr Ala Glu Asp Phe Lys
1               5                   10                  15

Gly

<210>  76
<211>  9
<212>  PRT
<213>  Artificial

<220>
<223>  heavy chain, CDR3

<400>  76

Leu Tyr Arg Gly Asn Thr Leu Asp Asn
1               5


<210>  77
<211>  17
<212>  PRT
<213>  Artificial

<220>
<223>  light chain, CDR1

<400>  77

Lys Ser Ser Gln Asn Leu Leu Asn Ser Gly Asn Gln Arg Asn Tyr Leu
1               5                   10                  15


Thr


<210>  78
<211>  9
<212>  PRT
<213>  Artificial

<220>
<223>  light chain, CDR3

<400>  78

Gln Asn Gly Tyr Ser Phe Pro Phe Thr
1               5


<210>  79
<211>  10
<212>  PRT
<213>  Artificial

<220>
<223>  heavy chain, CDR1

<400>  79

Gly Tyr Ala Phe Ser Asn Tyr Trp Met Asn
1               5                   10


<210>  80
<211>  18
<212>  PRT

<213>  Artificial

<220>
<223>  heavy chain, CDR2

<400>  80

Gly Gln Ile Tyr Pro Gly Asn Gly Asp Thr Lys Tyr Ser Gly Lys Phe
1               5                   10                  15

Asn Ser

<210>  81
<211>  9
<212>  PRT
<213>  Artificial

<220>
<223>  heavy chain, CDR3

<400>  81

Phe Tyr Tyr Gly Asn Val Met Asp Tyr
1               5

<210>  82
<211>  17
<212>  PRT
<213>  Artificial

<220>
<223>  light chain, CDR1

<400>  82

Lys Ser Ser Gln Thr Leu Leu Asn Gly Gly Asn Gln Lys Asn Tyr Leu
1               5                   10                  15

Thr

<210>  83
<211>  9
<212>  PRT
<213>  Artificial

<220>
<223>  light chain, CDR3

<400>  83

Gln Asn Gly Tyr Ser Tyr Pro Leu Thr
1               5

<210>  84
<211>  119

<212>  PRT
<213>  Artificial

<220>
<223>  heavy chain variable region

<400>  84

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Ser Leu Thr Asn Tyr
            20                  25                  30

Gly Val His Trp Val Arg Gln Ala Pro Gly Lys Gly Leu Glu Trp Val
        35                  40                  45

Ala Val Ile Trp Ser Asp Gly Arg Ile Asn Tyr Asn Ser Ala Leu Lys
    50                  55                  60

Ser Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Arg His Pro Ala Phe Gly Pro His Ala Met Asp Tyr Trp Gly Gln Gly
            100                 105                 110

Thr Leu Val Thr Val Ser Ser
        115


<210>  85
<211>  17
<212>  PRT
<213>  Artificial

<220>
<223>  light chain, CDR1

<400>  85

Lys Ser Ser Gln Ser Leu Leu Ser Ser Gly Asn Gln Arg Asn Tyr Leu
1               5                   10                  15

Thr


<210>  86
<211>  9
<212>  PRT
<213>  Artificial

<220>
<223>  light chain, CDR3

<400>  86

Gln Ser Asn Tyr Phe Tyr Pro Leu Thr
1               5


<210>  87
<211>  9
<212>  PRT
<213>  Artificial

<220>
<223>  light chain, CDR3

<400>  87

Gln Gln Asn Tyr Phe Tyr Pro Leu Thr
1               5


<210>  88
<211>  112
<212>  PRT
<213>  Artificial

<220>
<223>  light chain variable region

<400>  88

Asp Ile Val Met Thr Gln Ser Pro Leu Ser Leu Pro Val Thr Pro Gly
1               5                   10                  15


Glu Pro Ala Ser Ile Ser Cys Arg Ser Ser Lys Ser Leu Leu Asn Ser
            20                  25                  30


Asn Gly Asn Thr Tyr Leu Tyr Trp Tyr Leu Gln Lys Pro Gly Gln Ser
        35                  40                  45


Pro Gln Leu Leu Ile Tyr Arg Met Ser Asn Pro Ala Ser Gly Val Pro
    50                  55                  60


Asp Arg Phe Ser Gly Ser Gly Ser Gly Thr Asp Phe Thr Leu Lys Ile
65                  70                  75                  80


Ser Arg Val Glu Ala Glu Asp Val Gly Val Tyr Tyr Cys Met Gln Tyr
                85                  90                  95


Leu Glu Tyr Pro Pro Thr Phe Gly Ala Gly Thr Lys Leu Glu Ile Lys
            100                 105                 110


<210>  89
<211>  17

<212>  PRT
<213>  Artificial

<220>
<223>  light chain, CDR1

<400>  89

Lys Ser Ser Gln Asn Leu Leu Ser Ser Gly Asn Gln Arg Asn Tyr Leu
1               5                   10                  15

Thr

<210>  90
<211>  9
<212>  PRT
<213>  Artificial

<220>
<223>  light chain, CDR3

<400>  90

Gln Gln Gly Tyr Ser Phe Pro Phe Thr
1               5

<210>  91
<211>  17
<212>  PRT
<213>  Artificial

<220>
<223>  heavy chain, CDR2

<400>  91

Gln Ile Tyr Pro Gly Ser Gly Asp Thr Lys Tyr Ser Gly Lys Phe Gln
1               5                   10                  15

Ser

<210>  92
<211>  17
<212>  PRT
<213>  Artificial

<220>
<223>  light chain, CDR1

<400>  92

Lys Ser Ser Gln Thr Leu Leu Ser Gly Gly Asn Gln Lys Asn Tyr Leu
1               5                   10                  15

Thr

<210>  93
<211>  9
<212>  PRT
<213>  Artificial

<220>
<223>  light chain, CDR3

<400>  93

Gln Gln Gly Tyr Ser Tyr Pro Leu Thr
1               5


<210>  94
<211>  357
<212>  DNA
<213>  Artificial

<220>
<223>  heavy chain variable region

<400>  94
caggtgcagc tgaaggagtc aggacctgac ctggtggcgc cctcacagag cctgtccatc      60

acatgcaccg tctcagggtt ctccttaacc aactatggtg tacactgggt tcgccagcct     120

ccaggaaagg gtctggaatg gctggtagtg atatggagtg atggaagaat aaactataat     180

tcagctctca aatccagact gagcatcacc aaggacaatt ccaagaggca agttttccta     240

aaaatgaaca gtctccaaat tgatgacaca gccatttatt attgtgtcag acatcccgcc     300

ttcggtcccc atgctatgga ctactggggt caaggaatct cagtcaccgt ctcctca        357


<210>  95
<211>  336
<212>  DNA
<213>  Artificial

<220>
<223>  light chain variable region

<400>  95
gatattgtga tgactcagga tgcaccctct atacctgtca ctcctggaga gtcagtatcc      60

atctcctgca ggtctagtaa gagtctcctg aatagtaatg gcaacactta cttatattgg     120

ttcttgcaga ggccaggcca gtctcctcac ctcctgcttt atcggatgtc caaccctgcc     180

tcaggagccc cagacaggtt cagtggcagt gggtcaggga ctgaattcac actgagaatc     240

agtagagtgg aggctgaaga tgtgggtgtt tattactgta tgcaatatct ggaatatccg     300

cccacgttcg gtgctgggac caggctggag ctgaaa                               336


<210>  96
<211>  352

<212>  DNA
<213>  Artificial

<220>
<223>  heavy chain variable region

<400>  96
gaagtgatgc tggtggagtc tgggggaggc ttagtgaggc ctggagggtc cctgaaactc      60

tcctgtgcag gctctggaat cactctcagt acctatgcca tgtcttgggt tcgccagact     120

ccagagagga ggctggagtg ggtcgcatcc attattagtg gtggtatcac ctattattta     180

gacagtgtga agggccgctt caccatctcc agagataatg ccaggaacat cctgtacctg     240

caaatgagca gtctgaggtc tgaggacacg gccatttatt actgtgcaag aaaataccac     300

gggaatgctt tggactactg gggtcaagga acctcagtca ccgtctcttt ag             352


<210>  97
<211>  340
<212>  DNA
<213>  Artificial

<220>
<223>  light chain variable region

<400>  97
gacattgtga tgacacagtc tccatcgtcc ctgcctgtga cagcaggaga gacggtcact      60

atgcgctgca agtccagtca gagtctttta aacagtggaa atcaaaggaa ctacttgacc     120

tggtaccagc ggaaaccagg acagcctcct aagaaactga tctactgggc atccactagg     180

gaatctgggg tccctgatcg cttcacaggc agtggatctg gaacagattt cactctcacc     240

atcagtggtg tgcaggctga agacctggca gtttattact gtcagaataa ttatttttat     300

ccgctcacgt tcggtgctgg gaccaagctg gagctgaaac                           340


<210>  98
<211>  354
<212>  DNA
<213>  Artificial

<220>
<223>  heavy chain variable region

<400>  98
cagatccaga tggtgcagtc tggacctgag ctgaagaagc ctggagagac agtcaggatc      60

tcctgcaagg cttctgggta ttccttcaca actgctggaa tgcagtgggt gcgaaagatg     120

cctggagagg gtttgaagtg gattggctgg ataatcgccc actctggaga gccaaaatat     180

acagaagact tcaagggacg gtttgccttc tctttggaaa cctctgccag cactacatat     240

ttacagataa gtaacctcaa aaatgaggac acggctacgt atttctgtgc gagatgggga     300

aagggaaata ctatggacta ttggggtcaa ggaacctcag tcatcgtctc ctca           354

<210>  99
<211>  339
<212>  DNA
<213>  Artificial

<220>
<223>  light chain variable region

<400>  99
gacattgtga tgacacagtc gccatcctcc ctgactgtga cagcaggaga gaaggtcact      60

atgagctgca agtccagtca gagtctgtta aacggtggaa atcagaggaa ctacttgacc     120

tggtaccagc agaaaccagg acagcctcct aaacttttga tctactgggc atccactagg     180

gaatctgggg tccctgatcg cttcacaggc agtggatctg gaacacattt cactctcacc     240

atcagcagtg tgcaggctga agacctggca gtttattact gtcagaatgc ttattttttt     300

ccgctcacgt tcggtgctgg gaccaagcta gagctgaaa                            339


<210>  100
<211>  358
<212>  DNA
<213>  Artificial

<220>
<223>  heavy chain variable region

<400>  100
gatgtgcagc tggtggagtc tgggggaggc ttagtgcagc ctggagggtc ccggaaactc      60

tcctgtgcag cctctggatt cactttcagt agctttggaa tgcactgggt tcgtcaggct     120

ccagagaagg ggctggagtg ggtcgcatac attagtagtg gcagtagtac catctactat     180

ccagacacag tgaagggccg attcaccgtc tccagagaca atcccaagaa caccctgttc     240

ctgcaaatga ccagtctaag gtctgaggac acggccatgt attactgtgt aagattgggt     300

ccacggggga atgttatgga ccattggggt caaggaacct cagtcaccgt ctcctcag       358


<210>  101
<211>  339
<212>  DNA
<213>  Artificial

<220>
<223>  light chain variable region

<400>  101
gacattgtga tgacacagtc tccatcctcc ctgactgtga cagcaggaga gaaggtcact      60

atgagctgca agtccagtca gagtctgtta aacagtggaa atcaaaggaa ctacttgacc     120

tggtaccagc agaaaccagg gcagcctcct aaactgttga tctactgggc atccactagg     180

gaatctgggg tccctgatcg cttcacaggc agtggatctg gaacagattt cactctcacc     240

atcagcagtg tgcaggctga agacctggca gtttattact gtcagaatgt ttatttttat     300

ccactcacgt tcggtactgg gaccaagctg gagctgaga 339

<210> 102
<211> 354
<212> DNA
<213> Artificial

<220>
<223> heavy chain variable region

<400> 102
gatgtgcagc ttcaggagtc aggacctgac ctggtaaaac cctctcagtc actttcactc 60

acctgcactg tcagtggcta ctccatcagc ggtgcttata actggcactg gatccggcag 120

tttccaggaa acaaactgga gtggctggcc tacatgcagt acagtggtag ctctaactac 180

aacccatctt tcaaaagtcg aatctctatc agtcgagaca cgtccaagaa ccaattcttc 240

ctgcagttga aatctgtgac tactgaagac acagccacat attactgtgc aagaatgtat 300

aacggaaact cgtttcttta ctggggccaa gggactctgg tcactgtctc tgca 354

<210> 103
<211> 339
<212> DNA
<213> Artificial

<220>
<223> light chain variable region

<400> 103
gacattgtga tgacacagtc tccatcctcc ctgactgtga cagcaggcga gaaggtcact 60

atgaactgca agtccagtca gagtctgttt aacagtggaa atcaaaagaa ctatttgacc 120

tggtatcagc aaaaaccagg gcagcctcct agattgttga tctattgggc atccactagg 180

gaatctgggg tccctgatcg cttcacaggc agtggatctg gaacagattt cactctcacc 240

atcagcagtg tgcaggctga agacctgtca ctttattact gtcagaattc ttatagttat 300

cctctcacgt tcggtgctgg gaccaagctg gagttgaaa 339

<210> 104
<211> 357
<212> DNA
<213> Artificial

<220>
<223> heavy chain variable region

<400> 104
caggtgcagc tgaaggagtc aggacctgac ctggtggcgc cctcacagag cctgtccctc 60

acatgctccg tctcagggtt ctccttaacc agctatggta ttcactgggt tcgccagcct 120

ccaggaaagg gtctggagtg gctggtggtg atatggagtg atggaagaac aacctataat 180

tcaggtctca aatccagact gagcatcagc aaggacaact ccaagagcca agttctctta 240

aaaatgaaca gtctccgaac tgatgacaca gccatttact actgtgtcag acatcccgcc 300

ttcggtcccc atgctatgga ctactggggt caaggaacct cagtcaccgt ctcctca 357

<210> 105
<211> 336
<212> DNA
<213> Artificial

<220>
<223> light chain variable region

<400> 105
gatattgtga tgactcaggc tgcaccctct gtacctgtca ctcctggaga gtcagtatcc 60

atctcctgca ggtctagtaa gagtctcctg aatagtaatg gcaacactta cttgtattgg 120

ttcctgcaga ggccaggcca gtctcctcag ctcctgatat atcggatgtc caaccttgcc 180

tcaggagtcc cagacaggtt cagtggcagt gggtcaggaa ctgatttcac actgagaatc 240

agtagagtgg aggctgggga tgtgggtgtt tattactgta tgcaatatct agaatatccg 300

gtcacgttcg gtgctgggac caagctggag ctgaaa 336

<210> 106
<211> 357
<212> DNA
<213> Artificial

<220>
<223> heavy chain variable region

<400> 106
gatgtgcagc tggtggagtc tgggggaggc ttagtgcagc ctggagggtc ccggaaactc 60

tcctgtgcag cctctggatt cactttcagt cgctttggaa tgcactgggt tcgtcaggct 120

ccaaaaaagg gactggagtg ggtcgcatac attagtagtg gcagtaatac catctactat 180

gcagacacag tgaagggccg attcaccatc tccagagaca atcccaagaa caccctgttc 240

ctgcaaacga ccagtctaag gtctgaggac acggccatat attactgtgg aagattgggc 300

ttctatggta actcgtttga tcactggggc caagggactc tggtcactgt ctctgca 357

<210> 107
<211> 339
<212> DNA
<213> Artificial

<220>
<223> light chain variable region

<400> 107
aacattttga tgacacagtc tccatcctcc ctgactgtga cagcgggaga gaaggtcact 60

atgaactgca agtccagtca gagtctgtta aacggtggaa atcaaaggaa ctacttgacc 120

tggtaccagc agaaagcagg gcagcctccc aaactgttga tctactgggc atccactagg 180

gagtctgggg tccctgatcg cttcacaggc ggtggatctg gaacagattt cactctcacc 240

atcagcagtg tgcaggctga agacctggca ctttattact gtcagaattc ttattattat 300

ccgctcacgt tcggtgctgg taccaagctg gagctgaaa 339

<210> 108
<211> 354
<212> DNA
<213> Artificial

<220>
<223> heavy chain variable region

<400> 108
gaggtccagc tgcggcagtc tggacctgag ctggtaaagc ctggggcttc agtgaagatg 60

tcctgcaagg cttctggata cacattcact acctatatta taaactgggt gaagcagaag 120

cctgggcagg gccttgagtg gattggatat attaatcctt acaatgatga tacaaggtac 180

aatgagaggg tcaaaggcaa ggccacactg acttcagaca aatcctccag cactgcctac 240

atggagctca gcagcctgac ctctgaagac tctgcggtct attactgtgc aagattttac 300

tttggtaact cgtttactta ctggggccaa gggactctgg tcactgtctc tgca 354

<210> 109
<211> 339
<212> DNA
<213> Artificial

<220>
<223> light chain variable region

<400> 109
gacattgtga tgacacagtc tccgtcctcc ctgcctgtga cagtaggaga gagggtcact 60

atgacctgca agtccagtca gggtctgttt aacagtggaa atcaaaggaa ctacttgacc 120

tggtaccaac agaaaccagg gcagcctcct aaactgttga tctactgggc atccactagg 180

gaatctgggg tccctgatcg cttcacaggc agtggatctg gaacagattt cactctcacc 240

atcagcagtg tgcaggctga agacctggca atttattact gtcagaataa ttatatttat 300

ccgctcacgt tcggtgctgg gaccaagttg gagctgaaa 339

<210> 110
<211> 354
<212> DNA
<213> Artificial

<220>
<223> heavy chain variable region

<400> 110

gaggtccagc tgcggcagtc tggacctgag ctggtaaagc ctggggcttc agtgaagatg 60

tcctgcaagg cttctggata cacattcact acctatatta taaactgggt gaagcagaag 120

cctgggcagg gccttgagtg gattggatat attaatcctt acaatgatgg tactaggtac 180

aatgagaggg tcaaaggcaa ggccacactg acttcagaca aatcctccag tacagcctac 240

atggagctca gcagcctgac ctctgaggac tctgcggtct actactgtgc aagatttcac 300

tttggtaatt cgtttactta ctggggccaa gggactctgg tcactgtctc tgca 354

<210> 111
<211> 339
<212> DNA
<213> Artificial

<220>
<223> light chain variable region

<400> 111
gacattgtga tgacacagtc tccgtcctcc ctgcctgtga caacaggaga gaaggtcact 60

atgacttgca agtccagtca gggtctgttt aacaatggaa atcaaaggaa ctacttgacc 120

tggtaccagc agaaaccagg gcagcctcct aaactgttga tctactgggc atccactagg 180

gaatctgggg tccctgatcg cttcataggc agtggatctg gaacagattt cactctcacc 240

atcagcagtg tgcaggctga agacctggca atttattact gtcagaataa ttatattttt 300

ccgctcacgt tcggtgctgg gaccaagttg gagctgaaa 339

<210> 112
<211> 354
<212> DNA
<213> Artificial

<220>
<223> heavy chain variable region

<400> 112
cagatccagt tggtgcagtc tggacctgag ctgaagaagc ctggagagac agtcaagatc 60

tcctgcaagg cttctggata taccctcaca aactatggaa tgaattgggt gaggcaggct 120

ccaggaaagg gtttaaagtg gatgggctgg ataaggccca acactggaga gccaacatat 180

gctgaagact tcaagggacg atttgtcttc tctttggaaa cctctgccgc cactgcctat 240

ttacagatca ccaacctcaa aagtgaggac acgtctacat atttctgtgc aagactgtac 300

aggggaaata ctttggacaa ctggggtcaa ggaacctcag tcatcgtctc ctca 354

<210> 113
<211> 339
<212> DNA
<213> Artificial

<220>

<223>  light chain variable region

<400>  113
gacattgtga tgacacagtc tccatcctcc ctgactgtga caacagggga gaaggtcact      60

atgagctgca agtccagtca gaatctgtta aacagtggaa atcaaaggaa ctacttgacc     120

tggtaccagc agaagccagg acagtctcct aaactattga tctactgggc atccactagg     180

gaatctgggg tcccttatcg cttcacaggc agtggatctg gaacagattt cactctcacc     240

atcagcagtg tacagactga tgacctggct atttattact gtcagaatgg ttatagtttt     300

ccattcacgt tcggctcggg gacaaagttg gaaataaaa                            339


<210>  114
<211>  354
<212>  DNA
<213>  Artificial

<220>
<223>  heavy chain variable region

<400>  114
caggttcacc tgcagcagtc tggggctgaa ctggtgaggc ctgggtcctc agtgaagatt      60

tcctgcaagg cttctggcta tgcattcagt aattactgga tgaactgggt gaggcagagg     120

cctggacagg gtcttgagtg gattggacag atttatcctg gaaatggtga tactaagtac     180

agtggaaagt tcaacagtaa agacacactg actgcagaca aatcctccaa cacagcctac     240

atgcaactca acagcctaac atctgaggac tctgcggtct atttctgtgc aagattttac     300

tacggtaatg ttatggacta ctggggtcaa ggaacctcag tcaccgtctc ctcg           354


<210>  115
<211>  339
<212>  DNA
<213>  Artificial

<220>
<223>  light chain variable region

<400>  115
gacattgtgt tgacacagtc tccatcctcc ctgactgtga cagcaggaga gaaggtcact      60

atgagctgca agtccagtca gactctgtta aacggtggaa atcaaaagaa ctacttgacc     120

tggtaccagc agaaatcagg gcagcctcct aaactgttga tctattgggc atcaactagg     180

gaatctgggg tccctgatcg cttcacaggc agtggatctg gaacagattt cactctcacc     240

attagcagtg tccaggctga agacctggca gtttattact gtcagaatgg ttatagttat     300

ccgctcacgt tcggtgttgg gaccaagctg gagctgaaa                            339


<210>  116
<211>  351
<212>  DNA

<213>  Artificial

<220>
<223>  heavy chain variable region

<400>  116
gaggtgcagc tggtggagag cgggggggga ctggtgcagc caggaggaag cctgagactg      60

agctgtgccg gcagtggcat taccctgagc acatatgcca tgagctgggt gagacaggcc     120

cctggcaaag gactggagtg ggtgagcagc attatcagcg gaggaatcac ctactatctg     180

gatagcgtga agggacggtt cacaatcagc agggataacg ccaagaacac actgtatctg     240

cagatgagct ctctgagggc cgaagacacc gccgtgtatt attgcgccag aaagtaccac     300

ggaaacgccc tggactactg gggccagggg accctggtga cagtgagctc c              351


<210>  117
<211>  339
<212>  DNA
<213>  Artificial

<220>
<223>  light chain variable region

<400>  117
gacattgtga tgacccagag ccccgactcc ctggccgtga gcctgggaga aagagccacc      60

atcaactgca agagcagcca gagcctgctg agcagcggca accagagaaa ctacctgacc     120

tggtaccagc agaaacccgg ccagcccccc aaaaagctga tctactgggc ctccacccgg     180

gaatccggcg tgccagacag attctccggc tccggctccg gaaccgactt caccctgacc     240

atcagctccc tgcaggctga ggacctggcc gtgtactact gccagaacaa ctacttctac     300

cccctgacct tcggccaggg caccaaactg gagatcaag                            339


<210>  118
<211>  339
<212>  DNA
<213>  Artificial

<220>
<223>  light chain variable region

<400>  118
gacattgtga tgacccagag ccccgactcc ctggccgtga gcctgggaga aagagccacc      60

atcaactgca agagcagcca gagcctgctg agcagcggca accagagaaa ctacctgacc     120

tggtaccagc agaaacccgg ccagcccccc aaaaagctga tctactgggc ctccacccgg     180

gaatccggcg tgccagacag attctccggc tccggctccg gaaccgactt caccctgacc     240

atcagctccc tgcaggctga ggacctggcc gtgtactact gccagagcaa ctacttctac     300

cccctgacct tcggccaggg caccaaactg gagatcaag                            339

<210>  119
<211>  339
<212>  DNA
<213>  Artificial

<220>
<223>  light chain variable region

<400>  119
gacattgtga tgacccagag ccccgactcc ctggccgtga gcctgggaga aagagccacc      60

atcaactgca agagcagcca gagcctgctg agcagcggca accagagaaa ctacctgacc     120

tggtaccagc agaaacccgg ccagcccccc aaaaagctga tctactgggc ctccacccgg     180

gaatccggcg tgccagacag attctccggc tccggctccg gaaccgactt caccctgacc     240

atcagctccc tgcaggctga ggacctggcc gtgtactact gccagcagaa ctacttctac     300

cccctgacct tcggccaggg caccaaactg gagatcaag                            339


<210>  120
<211>  354
<212>  DNA
<213>  Artificial

<220>
<223>  heavy chain variable region

<400>  120
cagatccagc tggtgcagag cggcagcgaa ctgaagaagc ccggcgccag cgtgaaagtg      60

agctgcaagg ccagcggcta caccctgacc aactacggca tgaactgggt gagacaggct     120

cccggccagg ggctggaatg gatgggatgg atcagaccca acaccggaga gccaacctac     180

gccgaggact tcaagggcag attcgtgttc agcctggaca cctctgtggc caccgcttac     240

ctgcagatca ccagcctgaa agccgaggac accgccgtgt actactgtgc ccgcctgtat     300

agaggaaaca ccctggacaa ctggggccag ggcactctgg tgaccgtgag ctcc           354


<210>  121
<211>  339
<212>  DNA
<213>  Artificial

<220>
<223>  light chain variable region

<400>  121
gacattgtga tgacccagag ccccgactcc ctggccgtga gcctgggaga aagagccacc      60

atcaactgca agagcagcca gaacctgctg agcagcggca accagagaaa ctacctgacc     120

tggtaccagc agaaacccgg ccagcccccc aaactgctga tctactgggc ctccaccaga     180

gaaagcggcg tgcccgacag attctccgga agcggcagcg gcaccgactt caccctgact     240

atcagcagcc tgcaggctga ggacctggcc gtgtactact gccagcaggg ctacagcttc     300

cccttcacct tcggccaggg caccaaactg gagatcaaa 339

<210> 122
<211> 354
<212> DNA
<213> Artificial

<220>
<223> heavy chain variable region

<400> 122
caggtgcagc tggtgcagag cggcgccgag gtgaaaaagc ccggctcctc agtgaaagtg 60

agctgcaagg ccagcggcta cgccttctcc aactactgga tgaactgggt gagacaggcc 120

ccaggccagg gcctggaatg gatgggacag atctaccccg gcagcggcga tacaaagtac 180

agcggaaagt tccagagcag agtgaccatc accgccgaca aaagcaccaa caccgcctac 240

atggagctga gcagcctgag aagtgaggac accgccgtgt actactgcgc cagattctac 300

tacggcaacg tgatggacta ttggggccag ggaaccctgg tgaccgtgag ctct 354

<210> 123
<211> 339
<212> DNA
<213> Artificial

<220>
<223> light chain variable region

<400> 123
gacattgtgc tgacccagag ccccgactcc ctggctgtga gcctgggaga gagagccacc 60

atcaactgca agagcagcca gaccctgctg agtggaggaa accagaaaaa ctacctgact 120

tggtaccagc agaaacccgg ccagcccccc aaactgctga tctactgggc ctccacaaga 180

gaaagcggag tgcccgacag attcagcggc agcggcagcg gaaccgactt caccctgact 240

atcagcagcc tgcaggctga ggacctggcc gtgtactact gccagcaggg ctacagctac 300

ccactgacct tcggacaggg caccaagctg gagattaaa 339

<210> 124
<211> 330
<212> PRT
<213> Artificial

<220>
<223> heavy chain constant region

<400> 124

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe

        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210> 125
<211> 107
<212> PRT
<213> Artificial

<220>
<223> light chain constant region

<400> 125

<210> 126
<211> 448
<212> PRT
<213> Artificial

<220>
<223> IMAB362£¬heavy chain

<400> 126

Gln Val Gln Leu Gln Gln Pro Gly Ala Glu Leu Val Arg Pro Gly Ala
1               5                   10                  15

Ser Val Lys Leu Ser Cys Lys Ala Ser Gly Tyr Thr Phe Thr Ser Tyr
            20                  25                  30

Trp Ile Asn Trp Val Lys Gln Arg Pro Gly Gln Gly Leu Glu Trp Ile
        35                  40                  45

Gly Asn Ile Tyr Pro Ser Asp Ser Tyr Thr Asn Tyr Asn Gln Lys Phe
    50                  55                  60

Lys Asp Lys Ala Thr Leu Thr Val Asp Lys Ser Ser Ser Thr Ala Tyr
65                  70                  75                  80

Met Gln Leu Ser Ser Pro Thr Ser Glu Asp Ser Ala Val Tyr Tyr Cys
                85                  90                  95

Thr Arg Ser Trp Arg Gly Asn Ser Phe Asp Tyr Trp Gly Gln Gly Thr
            100                 105                 110

Thr Leu Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210> 127
<211> 220
<212> PRT
<213> Artificial

<220>
<223> IMAB362£¬light chain

<400> 127

Asp Ile Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Ser Tyr Pro Phe Thr Phe Gly Ser Gly Thr Lys Leu Glu Ile
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Val Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
                165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Cys
    210                 215                 220

<210>  128
<211>  113
<212>  PRT
<213>  Artificial

<220>
<223>  light chain variable region

<400>  128

Asp Ile Val Met Thr Gln Ser Pro Asp Ser Leu Ala Val Ser Leu Gly

1               5                   10                  15

Glu Arg Ala Thr Ile Asn Cys Lys Ser Ser Gln Ser Leu Leu Asn Ser
            20                  25                  30

Gly Asn Gln Arg Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Asn
                85                  90                  95

Asn Tyr Phe Tyr Pro Leu Thr Phe Gly Gln Gly Thr Lys Leu Glu Ile
            100                 105                 110

Lys

<210>  129
<211>  117
<212>  PRT
<213>  Artificial

<220>
<223>  heavy chain variable region

<400>  129

Glu Val Gln Leu Val Glu Ser Gly Gly Gly Leu Val Gln Pro Gly Gly
1               5                   10                  15

Ser Leu Arg Leu Ser Cys Ala Ala Ser Gly Ile Thr Leu Ser Thr Tyr
            20                  25                  30

Ala Met Ser Trp Val Arg Gln Ala Pro Gly Lys Gly Leu Glu Trp Val
        35                  40                  45

Ser Ser Ile Ile Ser Gly Gly Ile Thr Tyr Tyr Leu Asp Ser Val Lys
    50                  55                  60

Gly Arg Phe Thr Ile Ser Arg Asp Asn Ala Lys Asn Ser Leu Tyr Leu
65                  70                  75                  80

Gln Met Asn Ser Leu Arg Ala Glu Asp Thr Ala Val Tyr Tyr Cys Ala
                85                  90                  95

Arg Lys Tyr His Gly Asn Ala Leu Asp Tyr Trp Gly Gln Gly Thr Leu
            100                 105                 110

Val Thr Val Ser Ser
        115

<210>  130
<211>  113
<212>  PRT
<213>  Artificial

<220>
<223>  light chain variable region

<400>  130

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Asn Leu Leu Asn Ser
            20                  25                  30

Gly Asn Gln Arg Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Asn
                85                  90                  95

Gly Tyr Ser Phe Pro Phe Thr Phe Gly Gln Gly Thr Lys Leu Glu Ile
            100                 105                 110

Lys

<210>  131
<211>  118
<212>  PRT
<213>  Artificial

<220>
<223>  heavy chain variable region

<400>  131

Gln Val Gln Leu Val Gln Ser Gly Ser Glu Leu Lys Lys Pro Gly Ala

1               5                   10                  15

Ser Val Lys Val Ser Cys Lys Ala Ser Gly Tyr Thr Leu Thr Asn Tyr
            20                  25                  30

Gly Met Asn Trp Val Arg Gln Ala Pro Gly Gln Gly Leu Glu Trp Met
        35                  40                  45

Gly Trp Ile Arg Pro Asn Thr Gly Glu Pro Thr Tyr Ala Glu Asp Phe
    50                  55                  60

Lys Gly Arg Phe Val Phe Ser Leu Asp Thr Ser Val Ser Thr Ala Tyr
65                  70                  75                  80

Leu Gln Ile Cys Ser Leu Lys Ala Glu Asp Thr Ala Val Tyr Tyr Cys
                85                  90                  95

Ala Arg Leu Tyr Arg Gly Asn Thr Leu Asp Asn Trp Gly Gln Gly Thr
            100                 105                 110

Leu Val Thr Val Ser Ser
        115

<210>  132
<211>  113
<212>  PRT
<213>  Artificial

<220>
<223>  light chain variable region

<400>  132

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Thr Leu Leu Asn Gly
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Asn
                85                  90                  95

Gly Tyr Ser Tyr Pro Leu Thr Phe Gly Gln Gly Thr Lys Leu Glu Ile
            100                 105                 110

Lys

<210> 133
<211> 118
<212> PRT
<213> Artificial

<220>
<223> heavy chain variable region

<400> 133

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Ala Phe Ser Asn Tyr
            20                  25                  30

Trp Met Asn Trp Val Arg Gln Ala Pro Gly Gln Gly Leu Glu Trp Met
        35                  40                  45

Gly Gln Ile Tyr Pro Gly Asn Gly Asp Thr Lys Tyr Ser Gly Lys Phe
    50                  55                  60

Asn Ser Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Phe Tyr Tyr Gly Asn Val Met Asp Tyr Trp Gly Gln Gly Thr
            100                 105                 110

Leu Val Thr Val Ser Ser
        115

## Description

The present application claims the priority benefit of Chinese Patent Application No. CN201910929614.0 filed on 29 September 2019, which is hereby incorporated by reference in its entirety.

### TECHNICAL FIELD

The present disclosure belongs to the field of biomedicine, and relates to a novel anti-human Claudin18.2 antibody or a functional fragment thereof. The present disclosure also relates to the use of the antibody or functional fragment thereof.

### BACKGROUND OF THE INVENTION

Human tight junction protein Claudin18 (CLDN18) belongs to the family of tight junction proteins that biologically function in the construction of tight cell junctions, the maintenance of cell barrier functions and the involvement in intercellular molecular trafficking. Claudin18 protein has a molecular weight of about 26 KD and can be changed by alternative splicing to Claudin subtypes having different properties: CLDN18.1 and CLDN18.2. CLDN18.1 and CLDN18.2 each comprises 261 amino acids and has four transmembrane domains, i.e., NH2 terminal domain and COOH terminal domain intracellularly located, and two extracellular loops (ECL1, ECL2). The extracellular regions 1 of CLDN18.1 and CLDN18.2 differ from each other by 8 amino acids.

CLDN18.2 is expressed in primary lesions and metastases of a number of human epithelial tumor types and is expressed continuously in diffuse carcinoma cells. Expression levels of CLDN18.2 are even more significantly increased in esophageal, pancreatic, lung and gastric cancer cells. A study by Sahin et al suggested CLDN18.2 to be an important biomarker of gastric cancer, present in 70% of gastric cancer patients among which 90% to 95% were diagnosed with adenocarcinomas and another 5-10% comprised lymphomas, gastrointestinal stromal tumors (GISTs) and carcinoid tumors.

IMAB362, a monoclonal antibody against CLDN18.2, was developed by Ganymed, a biotechnological company in Germany, and is currently in phase III clinical trials for the treatment of patients with advanced gastroesophageal cancer. The binding of IMAB362 antibody alone leads to an inhibition of proliferation of target cells expressing CLDN18.2 both in vitro and in vivo, which inhibits tumor growth and eliminates the cancer cells via action mechanisms of Complement Dependent Cytotoxicity (CDC) and Antibody Dependent Cytotoxicity (ADCC), with various modes of action being shown to be independent but synergistic. IMAB362 antibody is directed against indications including metastatic esophageal cancer, and metastatic gastric cancer, and is currently in phase III clinical trials in the US and the EU and is expected to file for marketing approval in 2021.

IMAB362 is a chimeric antibody, however, with a relatively more murine amino acids and a relatively high probability of causing a rejection immune response in human. Thus, there remains a need in the art for an antibody, in particular a humanized antibody, having a higher specificity, affinity and biological activity for human CLDN18.2. Furthermore, the two subtypes of Claudin18 have highly similar structures but different functions. The high homology between CLDN18.1 and CLDN18.2 and the higher structure of ECL1 make it more difficult to screen an antibody against CLDN18.2 of high quality, and a high degree of uncertainty with regard to antibody specificity exists.

### SUMMARY OF THE INVENTION

The technical problem to be solved by the present invention is to obtain an antibody capable of specifically binding to human CLDN18.2 with high affinity through hybridoma screening and humanization, by optimizing the preparation method of monoclonal antibodies and improving the screening efficiency of Claudin 18.2 specific antibodies, wherein the antibody has the fewest murine amino acids by humanization design and is expected to have better in vivo safety and application potential.

For the technical problem as described above, with respect to an immunogen, murine-derived cells expressing human CLDN18.2 are utilized in the present disclosure. In one aspect, the murine-derived cells as host are non-immunogenic or have very low immunogenicity to mice, thereby avoiding the shadowing effect of the host cells on the immunogen of interest CLDN18.2 and avoiding the adverse effect on the production efficiency of CLDN18.2 specific hybridomas caused by introduction of a heterologous immunogen. In another aspect, expression of human CLDN18.2 on the surface of cell membrane of the murine-derived cells can be achieved, such that a monoclonal antibody obtained by the screening specifically binds to the extracellular region of CLDN18.2, ensuring the biological activity of the CLDN18.2 specific antibody.

With respect to screening strategy, a strategy which combines positive screening and negative screening is used in the present disclosure. Firstly, a positive screening is performed using recombinant cells expressing human CLDN18.2, to obtain monoclonal antibodies having high affinity to the cells; and then a negative screening is performed using recombinant cells expressing human CLDN18.1 on the antibody clones capable of binding to recombinant cells expressing human CLDN18.2, to exclude monoclonal antibodies capable of specifically binding to a common extracellular region structure of CLDN18.2 and CLDN 18.1. Furthermore, the immunogen, the positive screening antigen, and the negative screening antigen used in the present invention are all the same murine-derived cell platform, not only simplifying the process, avoiding the introduction of xenoantigens, but enabling the negative screening step to eliminate antibodies generated by allogeneic antigen effect of murine derived cells on individual mice, and further improving the production efficiency of monoclonal antibodies specifically binding extracellular region 2 of CLDN18.2.

A further object of the present disclosure is to provide an antibody or fragment thereof specifically binding human CLDN18.2 and to provide uses thereof. Fragment of the antibody according to the present disclosure encompasses, inter alia, various functional fragments of the antibody, such as antigen binding portions thereof, e.g. Fab, F(ab')₂ or scFv fragments.

The present disclosure provides the following technical solutions.

In one aspect, the present disclosure provides a preparation method of an anti-human Claudin 18.2 antibody, the preparation method including steps as follows:
(1) immunizing an animal with cells expressing human Claudin 18.2 protein on surface as an immunogen;
(2) preparing cell clones capable of producing antibodies using the animals immunized in step (1);
(3) using cells expressing human Claudin 18.2 protein on surface as a positive screening antigen, screening antibodies having a binding activity to the positive screening antigen and cells producing the antibodies;
(4) using the cells expressing human Claudin 18.1 protein on surface as a negative screening antigen, excluding antibodies having a binding activity to the negative screening antigen and cells producing the antibody.

Preferably, in the preparation method according to the present disclosure, the cells expressing the human Claudin 18.2 protein in step (1) are derived from the same species as the animal immunized; preferably, the cells expressing the human Claudin 18.2 protein are mouse cells, and the animal immunized is a mouse.

Preferably, in the preparation method according to the present disclosure, the cell clones capable of producing antibodies in step (2) are prepared by a technique selected from the group consisting of hybridoma technique and single cell amplification technique.

Preferably, in the preparation method according to the present disclosure, the positive screening antigen in step (3) is the same as the immunogen in step (1); and the negative screening antigen in step (4) differs from the immunogen in step (1) only in that the protein expressed is human Claudin 18.1 protein.

Preferably, in the preparation method according to the present disclosure, step (3) and step (4) each is conducted by a method selected from the group consisting of Enzyme-Linked Immunosorbent Assay (ELISA) and Fluorescence Resonance Energy Transfer (FRET).

In another aspect, the present disclosure provides an anti-human Claudin 18.2 antibody obtained according to the preparation method of the present disclosure.

Preferably, the anti-human Claudin 18.2 antibody according to the present disclosure specifically binds the N-terminus of human Claudin 18.2, preferably extracellular region at the N-terminus of human Claudin 18.2 comprising the first Extracellular Loop (ECL1).

Preferably, the anti-human Claudin 18.2 antibody according to the present disclosure specifically has a specific binding to human CLDN18.2 at nM scale; and which has no significant difference in binding to human CLDN18.1 as compared to an isotype negative antibody (or an unrelated antibody).

In a further aspect, the present disclosure provides an antibody or fragment thereof comprising a heavy chain variable region (VH) and a light chain variable region (VL), wherein the heavy chain variable region (VH) and the light chain variable region (VL) comprise a combination of CDRs (VH-CDR1, VH-CDR2, VH-CDR3; and VL-CDR1, VL-CDR2, VL-CDR3) selected from the group consisting of:
(1) VH-CDR1 as shown in SEQ ID NO. 31, VH-CDR2 as shown in SEQ ID NO. 32, and VH-CDR3 as shown in SEQ ID NO. 33; and VL-CDR1 as shown in SEQ ID NO. 34, VL-CDR2 as shown in SEQ ID NO. 35, and VL-CDR3 as shown in SEQ ID NO. 36;
(2) VH-CDR1 as shown in SEQ ID NO. 37, VH-CDR2 as shown in SEQ ID NO. 38, and VH-CDR3 as shown in SEQ ID NO. 39; and VL-CDR1 as shown in SEQ ID NO. 40, VL-CDR2 as shown in SEQ ID NO. 41, and VL-CDR3 as shown in SEQ ID NO. 42;
(3) VH-CDR1 as shown in SEQ ID NO. 43, VH-CDR2 as shown in SEQ ID NO. 44, and VH-CDR3 as shown in SEQ ID NO. 45; and VL-CDR1 as shown in SEQ ID NO. 46, VL-CDR2 as shown in SEQ ID NO. 41, and VL-CDR3 as shown in SEQ ID NO. 47;
(4) VH-CDR1 as shown in SEQ ID NO. 48, VH-CDR2 as shown in SEQ ID NO. 49, and VH-CDR3 as shown in SEQ ID NO. 50; and VL-CDR1 as shown in SEQ ID NO. 40, VL-CDR2 as shown in SEQ ID NO. 41, and VL-CDR3 as shown in SEQ ID NO. 51;
(5) VH-CDR1 as shown in SEQ ID NO. 52, VH-CDR2 as shown in SEQ ID NO. 53, and VH-CDR3 as shown in SEQ ID NO. 54; and VL-CDR1 as shown in SEQ ID NO. 55, VL-CDR2 as shown in SEQ ID NO. 41, and VL-CDR3 as shown in SEQ ID NO. 56;
(6) VH-CDR1 as shown in SEQ ID NO. 57, VH-CDR2 as shown in SEQ ID NO. 58, and VH-CDR3 as shown in SEQ ID NO. 33; and VL-CDR1 as shown in SEQ ID NO. 34, VL-CDR2 as shown in SEQ ID NO. 59, and VL-CDR3 as shown in SEQ ID NO. 60;
(7) VH-CDR1 as shown in SEQ ID NO. 61, VH-CDR2 as shown in SEQ ID NO. 62, and VH-CDR3 as shown in SEQ ID NO. 63; and VL-CDR1 as shown in SEQ ID NO. 46, VL-CDR2 as shown in SEQ ID NO. 41, and VL-CDR3 as shown in SEQ ID NO. 64;
(8) VH-CDR1 as shown in SEQ ID NO. 65, VH-CDR2 as shown in SEQ ID NO. 66, and VH-CDR3 as shown in SEQ ID NO. 67; and VL-CDR1 as shown in SEQ ID NO. 68, VL-CDR2 as shown in SEQ ID NO. 41, and VL-CDR3 as shown in SEQ ID NO. 69;
(9) VH-CDR1 as shown in SEQ ID NO. 65, VH-CDR2 as shown in SEQ ID NO. 70, and VH-CDR3 as shown in SEQ ID NO. 71; and VL-CDR1 as shown in SEQ ID NO. 72, VL-CDR2 as shown in SEQ ID NO. 41, and VL-CDR3 as shown in SEQ ID NO. 73;
(10) VH-CDR1 as shown in SEQ ID NO. 74, VH-CDR2 as shown in SEQ ID NO. 75, and VH-CDR3 as shown in SEQ ID NO. 76; and VL-CDR1 as shown in SEQ ID NO. 77, VL-CDR2 as shown in SEQ ID NO. 41, and VL-CDR3 as shown in SEQ ID NO. 78;
(11) VH-CDR1 as shown in SEQ ID NO. 79, VH-CDR2 as shown in SEQ ID NO. 80, and VH-CDR3 as shown in SEQ ID NO. 81; and VL-CDR1 as shown in SEQ ID NO. 82, VL-CDR2 as shown in SEQ ID NO. 41, and VL-CDR3 as shown in SEQ ID NO. 83;
(12) VH-CDR1 as shown in SEQ ID NO. 37, VH-CDR2 as shown in SEQ ID NO. 38, and VH-CDR3 as shown in SEQ ID NO. 39; and VL-CDR1 as shown in SEQ ID NO. 85, VL-CDR2 as shown in SEQ ID NO. 41, and VL-CDR3 as shown in SEQ ID NO. 42;
(13) VH-CDR1 as shown in SEQ ID NO. 37, VH-CDR2 as shown in SEQ ID NO. 38, and VH-CDR3 as shown in SEQ ID NO. 39; and VL-CDR1 as shown in SEQ ID NO. 85, VL-CDR2 as shown in SEQ ID NO. 41, and VL-CDR3 as shown in SEQ ID NO. 86;
(14) VH-CDR1 as shown in SEQ ID NO. 37, VH-CDR2 as shown in SEQ ID NO. 38, and VH-CDR3 as shown in SEQ ID NO. 39; and VL-CDR1 as shown in SEQ ID NO. 85, VL-CDR2 as shown in SEQ ID NO. 41, and VL-CDR3 as shown in SEQ ID NO. 87;
(15) VH-CDR1 as shown in SEQ ID NO. 74, VH-CDR2 as shown in SEQ ID NO. 75, and VH-CDR3as shown in SEQ ID NO. 76; and VL-CDR1 as shown in SEQ ID NO. 89, VL-CDR2 as shown in SEQ ID NO. 41, and VL-CDR3 as shown in SEQ ID NO. 90, and
(16) VH-CDR1 as shown in SEQ ID NO. 79, VH-CDR2 as shown in SEQ ID NO. 91, and VH-CDR3 as shown in SEQ ID NO. 81; and VL-CDR1 as shown in SEQ ID NO. 92, VL-CDR2 as shown in SEQ ID NO. 41, and VL-CDR3 as shown in SEQ ID NO. 93.

Preferably, in the antibody or fragment thereof according to the present disclosure, the heavy chain variable region comprises an amino acid sequence as shown in any one of SEQ ID NO. 1, SEQ ID NO. 3, SEQ ID NO. 5, SEQ ID NO. 7, SEQ ID NO. 9, SEQ ID NO. 11, SEQ ID NO. 13, SEQ ID NO. 15, SEQ ID NO. 17, SEQ ID NO. 19, SEQ ID NO. 21, SEQ ID NO. 23, SEQ ID NO. 27, and SEQ ID NO. 29 or an amino acid sequence having at least 75% identity to the amino acid sequence as shown; and/or the light chain variable region comprises an amino acid sequence as shown in any one of SEQ ID NO. 2, SEQ ID NO. 4, SEQ ID NO. 6, SEQ ID NO. 8, SEQ ID NO. 10, SEQ ID NO. 12, SEQ ID NO. 14, SEQ ID NO. 16, SEQ ID NO. 18, SEQ ID NO. 20, SEQ ID NO. 22, SEQ ID NO. 24, SEQ ID NO. 25, SEQ ID NO. 26, SEQ ID NO. 28, and SEQ ID NO. 30 or an amino acid sequence having at least 75% identity to the amino acid sequence as shown

According to a particular embodiment of the present disclosure, the antibody or fragment thereof comprises a heavy chain variable region and a light chain variable region selected from the group consisting of:
(1) an amino acid sequence as shown in SEQ ID NO. 1 or an amino acid sequence having at least 75% identity to the amino acid sequence as shown in SEQ ID NO. 1; and an amino acid sequence as shown in SEQ ID NO. 2 or an amino acid sequence having at least 75% identity to the amino acid sequence as shown in SEQ ID NO. 2;
(2) an amino acid sequence as shown in SEQ ID NO. 3 or an amino acid sequence having at least 75% identity to the amino acid sequence as shown in SEQ ID NO. 3; and an amino acid sequence as shown in SEQ ID NO. 4 or an amino acid sequence having at least 75% identity to the amino acid sequence as shown in SEQ ID NO. 4;
(3) an amino acid sequence as shown in SEQ ID NO. 5 or an amino acid sequence having at least 75% identity to the amino acid sequence as shown in SEQ ID NO. 5; and an amino acid sequence as shown in SEQ ID NO. 6 or an amino acid sequence having at least 75% identity to the amino acid sequence as shown in SEQ ID NO. 6;
(4) an amino acid sequence as shown in SEQ ID NO. 7 or an amino acid sequence having at least 75% identity to the amino acid sequence as shown in SEQ ID NO. 7; and an amino acid sequence as shown in SEQ ID NO. 8 or an amino acid sequence having at least 75% identity to the amino acid sequence as shown in SEQ ID NO. 8;
(5) an amino acid sequence as shown in SEQ ID NO. 9 or an amino acid sequence having at least 75% identity to the amino acid sequence as shown in SEQ ID NO. 9; and an amino acid sequence as shown in SEQ ID NO. 10 or an amino acid sequence having at least 75% identity to the amino acid sequence as shown in SEQ ID NO. 10;
(6) an amino acid sequence as shown in SEQ ID NO. 11 or an amino acid sequence having at least 75% identity to the amino acid sequence as shown in SEQ ID NO. 11; and an amino acid sequence as shown in SEQ ID NO. 12 or an amino acid sequence having at least 75% identity to the amino acid sequence as shown in SEQ ID NO. 12;
(7) an amino acid sequence as shown in SEQ ID NO. 13 or an amino acid sequence having at least 75% identity to the amino acid sequence as shown in SEQ ID NO. 13; and an amino acid sequence as shown in SEQ ID NO. 14 or an amino acid sequence having at least 75% identity to the amino acid sequence as shown in SEQ ID NO. 14;
(8) an amino acid sequence as shown in SEQ ID NO. 15 or an amino acid sequence having at least 75% identity to the amino acid sequence as shown in SEQ ID NO. 15; and an amino acid sequence as shown in SEQ ID NO. 16 or an amino acid sequence having at least 75% identity to the amino acid sequence as shown in SEQ ID NO. 16;
(9) an amino acid sequence as shown in SEQ ID NO. 17 or an amino acid sequence having at least 75% identity to the amino acid sequence as shown in SEQ ID NO. 17; and an amino acid sequence as shown in SEQ ID NO. 18 or an amino acid sequence having at least 75% identity to the amino acid sequence as shown in SEQ ID NO. 18;
(10) an amino acid sequence as shown in SEQ ID NO. 19 or an amino acid sequence having at least 75% identity to the amino acid sequence as shown in SEQ ID NO. 19; and an amino acid sequence as shown in SEQ ID NO. 20 or an amino acid sequence having at least 75% identity to the amino acid sequence as shown in SEQ ID NO. 20;
(11) an amino acid sequence as shown in SEQ ID NO. 21 or an amino acid sequence having at least 75% identity to the amino acid sequence as shown in SEQ ID NO. 21; and an amino acid sequence as shown in SEQ ID NO. 22 or an amino acid sequence having at least 75% identity to the amino acid sequence as shown in SEQ ID NO. 22;
(12) an amino acid sequence as shown in SEQ ID NO. 23 or an amino acid sequence having at least 75% identity to the amino acid sequence as shown in SEQ ID NO. 23; and an amino acid sequence as shown in SEQ ID NO. 24 or an amino acid sequence having at least 75% identity to the amino acid sequence as shown in SEQ ID NO. 24;
(13) an amino acid sequence as shown in SEQ ID NO. 23 or an amino acid sequence having at least 75% identity to the amino acid sequence as shown in SEQ ID NO. 23; and an amino acid sequence as shown in SEQ ID NO. 25 or an amino acid sequence having at least 75% identity to the amino acid sequence as shown in SEQ ID NO. 25;
(14) an amino acid sequence as shown in SEQ ID NO. 23 or an amino acid sequence having at least 75% identity to the amino acid sequence as shown in SEQ ID NO. 23; and an amino acid sequence as shown in SEQ ID NO. 26 or an amino acid sequence having at least 75% identity to the amino acid sequence as shown in SEQ ID NO. 26;
(15) an amino acid sequence as shown in SEQ ID NO. 27 or an amino acid sequence having at least 75% identity to an amino acid sequence as set forth in SEQ ID NO. 27; and an amino acid sequence as shown in SEQ ID NO. 28 or an amino acid sequence having at least 75% identity to the amino acid sequence as shown in SEQ ID NO. 28;
(16) an amino acid sequence as shown in SEQ ID NO. 29 or an amino acid sequence having at least 75% of identity to the amino acid sequence as shown in SEQ ID NO. 29; and an amino acid sequence as shown in SEQ ID NO. 30 or an amino acid sequence having at least 75% of identity to the amino acid sequence as shown in SEQ ID NO. 30.

The at least 75% identity in the context of the present disclosure is any percent identity greater than or equal to 75%, such as at least 80%, preferably at least 85%, more preferably at least 90%, even more preferably at least 91%, 92%, 93%, 94%, 95%, 96%, 97%, 98%, or even 99% identity.

The antibody or fragment thereof according to the present disclosure is in any form, e.g., a monoclonal antibody, a single chain antibody, a diabody, a single domain antibody, a nanobody, a fully or partially humanized antibody, or a chimeric antibody and the like; alternatively, the antibody or fragment thereof is a half-antibody or an antigen-binding fragment of the half-antibody, e.g., single-chain variable fragment (scFv), bivalent single-chain variable fragment (BsFv), disulfide-stabilized Fv fragment (dsFv), (disulfide-stabilized Fv fragment)₂ (dsFv)₂, antigen-binding fragment (Fab), Fab' fragment, F(ab')₂ fragment, or variable fragment (Fv). With respect to the fragment provided by the present disclosure, preferably, the antibody or fragment thereof is any fragment of the antibody capable of binding to human Claudin 18.2.

Preferably, the antibody or fragment thereof further comprises a human or murine constant region, preferably a human or murine light chain constant region (CL) and/or heavy chain constant region (CH); more preferably, the antibody or fragment thereof comprises a heavy chain constant region selected from the group consisting of IgG, IgA, IgM, IgD and IgE and/or a kappa or lambda type light chain constant region.

Preferably, the antibody is a monoclonal antibody, preferably a murine, chimeric, or humanized monoclonal antibody; preferably, the heavy chain constant region of the monoclonal antibody is of IgG1 or IgG4 subtype and the light chain constant region of the monoclonal antibody is of kappa type.

According to a particular embodiment of the present disclosure, the heavy chain constant region of the monoclonal antibody comprises an amino acid sequence as shown in SEQ ID NO: 124 or an amino acid sequence having at least 75% identity to the amino acid sequence as shown. According to a particular embodiment of the present disclosure, the light chain constant region of the monoclonal antibody comprises an amino acid sequence as shown in SEQ ID NO: 125 or an amino acid sequence having at least 75% identity to the amino acid sequence as shown.

In yet another aspect, the present disclosure provides a nucleic acid molecule comprising a nucleotide sequence encoding the antibody or fragment thereof according to the present disclosure, or encoding a heavy chain CDR, a light chain CDR, a heavy chain variable region, a light chain variable region, a heavy chain or a light chain comprised in the antibody or fragment thereof. According to a particular embodiment of the present disclosure, the nucleic acid molecule comprising a nucleotide sequence encoding the heavy chain variable region or the light chain variable region of the antibody according to the present disclosure. For example, the nucleic acid molecule comprises the nucleotide sequence as shown in any one of SEQ ID NOs. 96-125.

The nucleic acid molecule according to the present disclosure can be cloned into a vector which in turn transfects or transforms a host cell. In yet another aspect, the present disclosure provides a vector comprising the nucleic acid molecule of the present disclosure. The vector can be a eukaryotic expression vector, a prokaryotic expression vector, an artificial chromosome, a phage vector and the like.

The vector or nucleic acid molecule of the present disclosure may be used to transform or transfect a host cell or in any way enter a host cell for antibody preservation or expression, etc. Thus, in a further aspect, the present disclosure provides a host cell comprising the nucleic acid molecule and/or vector according to the present disclosure, or transformed or transfected with the nucleic acid molecule and/or vector according to the present disclosure. The host cell may be any prokaryotic or eukaryotic cell, such as a bacterial or insect, fungus, plant or animal cell.

The antibody according to the present disclosure can be obtained using any conventional techniques known in the art. For example, the heavy chain variable region and/or the light chain variable region of the antibody or the heavy chain and/or the light chain of the antibody may be obtained from the nucleic acid molecule provided by the present disclosure, and then the antibody is obtained by assembling them with optional other domains of the antibody; alternatively, the host cell provided by the present disclosure is cultured under conditions that allow the host cell to express the heavy chain variable region and/or the light chain variable region of the antibody or the heavy chain and/or the light chain of the antibody and assemble them into an antibody. Optionally, the method further includes a step of recovering the produced antibody.

The antibody or fragment thereof provided by the present disclosure may also be combined with other moieties, for example, a cell surface receptor, a small molecule compound such as amino acids and carbohydrates, a small molecule polymer, or any other moiety that modifies the antibody of the present disclosure, or even an active protein or polypeptide. Thus, in another aspect, the present invention provides a conjugate or fusion protein comprising an antibody or fragment thereof provided by the present disclosure. For example, the conjugate or fusion protein can be a bispecific antibody comprising an antibody or fragment thereof according to the present disclosure.

The antibody or fragment thereof, the nucleic acid molecule, the vector, the host cell, and/or the conjugate or fusion protein provided by the present disclosure may be contained in a pharmaceutical composition, more particularly, a pharmaceutical preparation, to be used for various purposes as actually needed. Thus, in a further aspect, the present disclosure also provides a pharmaceutical composition comprising an antibody or fragment thereof, a nucleic acid molecule, a vector, a host cell, and/or a conjugate or fusion protein according to the present disclosure, and optionally a pharmaceutically acceptable excipient.

For any purpose of use, the present disclosure also provides a kit comprising an antibody or fragment thereof, a nucleic acid molecule, a vector, a host cell, a pharmaceutical composition and/or a conjugate or fusion protein of the present disclosure.

In another aspect, the present disclosure also provides use of the antibody or fragment thereof, the nucleic acid molecule, the vector, the host cell, the pharmaceutical composition, and/or the conjugate or fusion protein as described above in the manufacture of a medicament for the prevention and/or treatment of cancer. Preferably, the cancer is selected from the group consisting of pancreatic cancer, gastric cancer, colon cancer, esophageal cancer, liver cancer, ovarian cancer, lung cancer, gallbladder cancer, and head and neck cancer.

In a further aspect, the present disclosure also provides use of the antibody or fragment thereof, the nucleic acid molecule, the vector, the host cell, the pharmaceutical composition, and/or the conjugate or fusion protein as described above in the manufacture of a reagent for the diagnosis of cancer. Preferably, the cancer is selected from the group consisting of pancreatic cancer, gastric cancer, colon cancer, esophageal cancer, liver cancer, ovarian cancer, lung cancer, gallbladder cancer, and head and neck cancer.

In another aspect, the present invention also provides a method of preventing and/or treating cancer, the method including administering to a subject in need thereof the antibody or fragment thereof, the nucleic acid molecule, the vector, the host cell, the pharmaceutical composition, and/or the conjugate or fusion protein according to the present disclosure, and optionally other drug or means. The optional other drug or means refers to other drug or means that can be administered in combination with the antibody or fragment thereof, the nucleic acid molecule, the vector, the host cell, the pharmaceutical composition, and/or the conjugate or fusion protein of the present disclosure, such as a small molecule drug, a targeted drug, a recombinant protein drug such as an antibody, a vaccine, an ADC, an oncolytic virus, a gene or nucleic acid therapy drug and radiotherapy. The co-administration of the two may be in any way, including simultaneously, sequentially or at intervals.

Preferably, the cancer is selected from the group consisting of pancreatic cancer, gastric cancer, colon cancer, esophageal cancer, liver cancer, ovarian cancer, lung cancer, gallbladder cancer, and head and neck cancer. Preferably, the subject is a mammal; more preferably, the subject is a human.

Alternatively, the present invention also provides a method for diagnosing cancer, the method including contacting the antibody or fragment thereof, the nucleic acid molecule, the vector, the host cell, the pharmaceutical composition, and/or the conjugate or fusion protein according to the present disclosure with a sample from a subject. Preferably, the cancer is selected from the group consisting of pancreatic cancer, stomach cancer, colon cancer, esophageal cancer, liver cancer, ovarian cancer, lung cancer, gallbladder cancer, and head and neck cancer. Preferably, the subject is a mammal; more preferably, the subject is a human.

The genes encoding the anti-human Claudin 18.2 antibody according to the present disclosure were transduced onto primary T cells from a healthy donor through a lentiviral vector, and the prepared CAR-T cells were found to be efficiently activated by cells expressing human Claudin 18.2. Thus, in a further aspect, the present invention also provides the use of the antibody or fragment thereof, the nucleic acid molecule, the vector, the host cell, the pharmaceutical composition, and/or the conjugate or fusion protein according to the present disclosure in the manufacture of CAR-T cells.

The present invention provides a novel antibody capable of specifically binding to human Claudin 18.2. In contrast to existing anti-human Claudin 18.2 antibodies, the antibody according to the present disclosure has the following characteristics.

The anti-human Claudin 18.2 antibody provided by the present disclosure exhibited a strong affinity for the antigen Claudin 18.2, and had significant Complement Dependent Cytotoxicity (CDC) activity and antibody dependent cytotoxicity (ADCC) activity on target expressing cells, both of which were stronger than or comparable to IMAB362. Furthermore, the anti-human Claudin 18.2 antibody of the present disclosure exhibited a higher specificity to human CLDN18.2 as compared to IMAB362.

In addition, the genes encoding the anti-human Claudin 18.2 antibody according to the present disclosure were transduced onto primary T cells from a healthy donor through a lentiviral vector, and CAR-T cells were prepared. Upon the stimulation of target cells, the prepared CAR-T cells were detected for activating protein CD25 on the surface of CAR-T cells and for the secreted IFNγ, and were found to be efficiently activated by cells expressing human Claudin 18.2, demonstrating the target specificity of the antibody according to the present disclosure for human CLDN 18.2.

### Definitions

Unless otherwise defined, the meaning of scientific and technical terms used herein is the meaning commonly understood by those skiledl in the art. The nomenclatures and techniques used in cell and tissue culture, molecular biology, and protein and oligo or polynucleotide chemistry and hybridization described herein are well known and commonly used in the art. Standard techniques are used for recombinant DNA, oligonucleotide synthesis, and tissue culture and transformation (e.g., electroporation, lipid transfection). Enzymatic reaction and purification techniques are performed according to the manufacturer's specifications or procedures commonly used in the art or described herein. The foregoing techniques and procedures are used as described in the many comprehensive and more specific literatures commonly known in the art and cited and discussed in this specification. See, e.g., Sambrook et al, Molecular Cloning: A Laboratory Manual (2nd ed., Cold Spring Harbor Laboratory Press, Cold Spring Harbor, N.Y. (1989)). The nomenclatures and laboratory procedures and techniques used in analytical chemistry, synthetic organic chemistry, and medical and pharmaceutical chemistry described herein are well known and commonly used in the art.

As used herein, term "antibody" refers to an immunoglobulin molecule that generally consists of two pairs of polypeptide chains (each pair with one "light" (L) chain and one "heavy" (H) chain). In a general sense, the heavy chain can be interpreted as a polypeptide chain with a larger molecular weight in an antibody, and the light chain refers to a polypeptide chain with a smaller molecular weight in an antibody. Light chains are classified as κ and λ light chains. Heavy chains are generally classified as µ, δ, γ, α, or ε, and isotypes of antibodies are defined as IgM, IgD, IgG, IgA, and IgE, respectively. In light chains and heavy chains, the variable region and constant region are linked by a "J" region of about 12 or more amino acids, and the heavy chain also comprises a "D" region of about 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain CL. The constant region of the antibody can mediate the binding of immunoglobulins to host tissues or factors, including the binding of various cells of the immune system (e.g., effector cells) to the first component (C1q) of classical complement system. The VH and VL regions can be further subdivided into highly variable regions (called complementarity determining regions (CDRs)), and between which conservative regions called framework regions (FRs) are distributed. Each VH and VL consists of 3 CDRs and 4 FRs arranged from amino terminus to carboxyl terminus in the following order: FR1, CDR1, FR2, CDR2, FR3, CDR3, FR4. The variable regions (VH and VL) of each heavy chain/light chain pair form an antibody binding site, respectively. The assignment of amino acids to each region or domain follows the definition of Kabat Sequences of Proteins of Immunological Interest (National Institutes of Health, Bethesda, Md. (1987 and 1991)), or Chothia & Lesk (1987) J. Mol. Biol. 196:901-917 ; Chothia et al. (1989) Nature 342:878-883 . In particular, the heavy chain may also comprise more than 3 CDRs, such as 6, 9, or 12. For example, in the bispecific antibody of the present invention, the heavy chain may be a ScFv with the N-terminus of the heavy chain of IgG antibody linked to another antibody, and in this case, the heavy chain comprises 9 CDRs.

As used herein, term "antigen binding fragment" refers to the polypeptide comprising the fragment of a full-length antibody, which maintains the ability to specifically bind to the same antigen to which the full-length antibody binds, and/or competing with the full-length antibody for the specific binding to antigen, which is also known as the "antigen binding portion". See generally, Fundamental Immunology, Ch. 7 (Paul, W., ed., 2nd edition, Raven Press, N.Y. (1989), which is incorporated herein by reference in its entirety for all purposes. Antigen binding fragment of the antibody can be produced by recombinant DNA technology or by enzymatic or chemical cleavage of intact antibodies. In some cases, the antigen binding fragment includes a Fab, a Fab', an F(ab')2, an Fd, an Fv, a dAb, a complementarity determining region (CDR) fragment, a single chain antibody fragment (e.g., scFv), a chimeric antibody, a diabody and such polypeptide, which comprises at least a portion of the antibody sufficient to impart specific antigen binding ability to a polypeptide.

As used herein, term "Fv fragment" refers to an antibody fragment consisting of the VL and VH domains of a single arm of an antibody; term "Fab fragment" refers to an antibody fragment consisting of VL, VH, CL, and CHI (or CH) domains; and term "F(ab')2" refers to an antibody fragment comprising two Fab fragments linked by the disulfide bridge on a hinge region.

In some cases, the antigen binding fragment of the antibody is a single chain binding fragment (e.g., scFv) in which the VL and VH domains are paired to form a monovalent molecule via a linker that enables them to produce a single polypeptide chain (see, e.g., Bird et al., Science 242:423-426 (1988) and Huston et al., Proc. Natl. Acad. Sci. USA 85:5879-5883 (1988)). Such scFv molecules may have a general structure: NH2-VL-linker-VH-COOH or NH2-VH-linker-VL-COOH. An appropriate prior art linker consists of a repeating G4S amino acid sequence or a variant thereof. For example, a linker having the amino acid sequence (G4S)4 can be used, but variants thereof can also be used (Holliger et al. (1993), Proc. Natl. Acad. Sci. USA 90: 6444-6448).

Antigen binding fragments (e.g., the above mentioned antibody fragments) of antibodies can be obtained from given antibodies by using conventional techniques known to those skilled in the art (e.g., recombinant DNA technology or enzymatic or chemical cleavage), and the antigen binding fragments of the antibodies are screened for specificity in the same way as for intact antibodies.

As used herein, unless otherwise clearly defined in the context, when referring to term "antibody", it includes not only intact antibodies but also antigen binding fragments of antibodies.

As used herein, term "isolated" refers to a state obtained from natural state by artificial means. If a certain "isolated" substance or component is present in nature, it is possible because its natural environment changes, or the substance is isolated from natural environment, or both. For example, a certain unisolated polynucleotide or polypeptide naturally exsits in a certain living animal body, and the same polynucleotide or polypeptide with a high purity isolated from such a natural state is called isolated polynucleotide or polypeptide. Term "isolated" does not exclude mixed artificial or synthesized substance or other unpure substance that does not affect the activity of the isolated substance.

Term "host cell" refers to a cell into which a vector can be introduced, including but not limited to prokaryotic cells such as Escherichia coli cells, fungal cells such as yeast cells, insect cells such as Drosophila melanogaster (S2) cells or Spodoptera frugiperda (Sf9) cells, or animal cells such as fibroblasts, CHO cells, COS cells, NSO cells, HeLa cells, BHK cells, HEK293 cells or human cells, etc.

Term "KD" refers to the equilibrium dissociation constant (KD) of a specific antibody-antigen interaction, which is used to describe the binding affinity between the antibody and the antigen. The smaller the equilibrium dissociation constant, the closer the antibody-antigen binding, and the higher the affinity between the antibody and the antigen. In general, an antibody binds to an antigen at an equilibrium dissociation constant less than about 10-5 M, for example, less than about 10-6 M, 10-7 M, 10-8 M, 10-9 M or 10-10 M or less, for example, as determined by surface plasmon resonance (SPR) in a BIACORE instrument. For example, the affinity of an antibody with a cell is detected on a KINEXA 400 instrument using KINEXA method.

Term "Specific binding" means that an antibody reacts with one or more antigenic determinants of an antigen but does not react with other polypeptides, or it binds to other polypeptides with very low affinity (Kd >10-6). The antibody include but is not limited to polyclonal, monoclonal, chimeric, dAb (domain antibody), single chain, Fab, Fab' and F(ab')2 fragment, Fv, scFv and Fab expression library. A monoclonal antibody (mAb) is an antibody obtained from a monoclonal cell line, and the cell line is not limited to eukaryotic, prokaryotic or phage clonal cell lines. A monoclonal antibody or antigen-binding fragment thereof can be obtained by recombination using, for example, hybridoma technology, recombination technology, phage display technology, and synthesis techniques such as CDR grafting, or other known techniques.

The "murine antibody" of the present disclosure is a monoclonal antibody against human CLDN18.2 produced according to the knowledge and skills in the art. During the production, the test subject is injected with the CLDN18.2 antigen, and then the hybridomas expressing the antibody with the desired sequence or functional property are isolated.

The "chimeric antibody" of the present disclosure is an antibody formed by fusing the variable regions of a murine-derived antibody with the constant regions of a human antibody, which can reduce the immune response induced by the murine-derived antibody. To establish a chimeric antibody, it is necessary to establish a hybridoma secreting murine-derived specific monoclonal antibodies first, clone the variable region genes from the mouse hybridoma cells, and then clone the constant region genes of the human antibody as needed, and chimeric genes formed by linking the mouse variable region with the human constant region genes are inserted into an expression vector. Finally, the chimeric antibody is expressed in a eukaryotic system or a prokaryotic system.

The "humanized antibody" of the present disclosure is also called a CDR grafted antibody, which is the antibody produced by grafting mouse CDR sequences into the variable region framework (FR) of a human antibody. Such variable region framework sequences can be obtained from public DNA databases or public references, for example, from the ImMunoGeneTics (IMGT) website http://imgt.cines.fr or from the Journal of Immunoglobulin, 2001ISBN012441351.

Term "CLDN18.2" includes isoforms, mammalian (e.g. human) CLDN18.2, species homologs of human CLDN18.2, and analogs having at least one epitope common with human CLDN18.2. The amino acid sequence of CLDN18.2 (e.g. human CLDN18.2) is known in the art, for example as shown in NCBI database.

Term "CLDN18.1" includes isoforms, mammalian (e.g. human) CLDN18.1, species homologs of human CLDN18.1, and analogs having at least one epitope common with human CLDN18.1. The amino acid sequence of CLDN18.1 (e.g. human CLDN18.1) is known in the art, for example as shown in NCBI database.

"Optional", "optionally", "any", or "any one" means that the following event or situation may but not necessarily occur, and the description includes the instances in which the event or situation does or does not occur. For example, "optionally contains 1 antibody heavy chain variable region" means the antibody heavy chain variable region with a specific sequence may be, but not necessarily be, present.

Term "pharmaceutical composition" refers to a mixture containing one or more compounds of the present disclosure or a physiologically/pharmaceutically acceptable salt or prodrug thereof together with other chemical components, as well as other components such as a physiologically/pharmaceutically acceptable carrier and excipient. The pharmaceutical composition is used to facilitate administration to an organism, and facilitate absorption of the active ingredient to exert biological activities. A therapeutic composition should generally be sterile and stable under conditions of manufacture and storage. The composition can be formulated as a solution, a microemulsion, a dispersion, a liposome or other ordered structure suitable for a high antibody concentration. A sterile injectable solution can be prepared by incorporating the active compound (i.e., the antibody or antibody portion) in a required amount in an appropriate solvent with one ingredient or a combination of ingredients as listed above, and as required followed by being filtered for sterilization.

The method, composition, and combination therapy according to the present disclosure can be combined with other active agents or therapeutic methods. The method includes the administration of the CLDN18.2 antibody molecule of the present disclosure to a subject in an amount effective for treatment or prevention of a disease (e.g., a cancer), optionally, in combination with one or more inhibitors selected from PD-1, PD-L1, PD-L2, LAG-3, CTLA-4, or Tim-3 antibody (immunotherapy) or other tumor therapeutic antibodies, such as Her-2, EGFR, VEGF, VEGFR antibodies, etc., as well as ADC (antibody drug conjugate, such as T-DM1), bispecific antibody, chemotherapy drug, etc. The method also includes the administration of the CLDN18.2 antibody molecule, additional active agents or all can be administered in such amount or dose that is higher, lower, or equal to the amount or dose of each active agent used alone (e.g., as a monotherapy). The administered amount or dose of the CLDN18.2 antibody molecule, the additional active agents or all of them is lower (e.g., at least 20%, at least 30%, at least 40%, or at least 50%) than the amount or dose of each active agent used alone (e.g., as a monotherapy).

Furthermore, the anti-CLDN 18.2 antibody of the present disclosure can bind to CLDN18.2 to induce apoptosis of target cells (tumor cells), inhibit growth of tumor cells, increase ADCC against tumor cells by effector cells in vivo, and CDC killing effect for treating cancer patients. Accordingly, in certain embodiments, the anti-CLDN 18.2 antibody molecule described herein exhibits anti-tumor effect of the antibody of the present disclosure through these mechanisms. And a method of inhibiting growth of tumor cells is provided, which includes administering a therapeutically effective amount of the anti-CLDN 18.2 antibody molecule described herein to a subject. The method is suitable for in vivo treatment of cancer. To obtain a target specific therapeutic effect, the anti-CLDN 18.2 antibody molecule may be administered together with other antibodies. When the CLDN18.2 antibody is administered in combination with one or more active agents, the combination may be administered to a cancer type, in particular to a patient having a CLDN18.2 expressing tumor, in any order or simultaneously. In certain aspects, a method of treating (e.g., reducing or alleviating) a hyperproliferative condition or disease (e.g., cancer), such as a solid tumor, hematological cancer, soft tissue tumor or metastatic lesion, in a subject is provided. The method includes administering one or more anti-CLDN 18.2 antibody molecules described herein to a subject alone or in combination with other active agents or treatments.

As used herein, terms "carcinoma", "cancer", "cancer patient" are intended to include all types of cancerous growth or tumorigenic process, metastatic tissue or malignant transformed cells, tissues or organs, regardless of the histopathological type or aggressive stage. Examples include but are not limited to solid tumors, hematological cancers, soft tissue tumors, and metastatic lesions. Examples of solid tumors include malignancies, e.g. sarcomas and carcinomas of multiple organ systems (including adenocarcinomas and squamous cell carcinomas), such as those invading liver, lung, breast, lymph, gastrointestinal tract (e.g. colon), genitourinary tract (e.g. kidney, bladder epithelial cells), prostate and pharynx. Adenocarcinomas include malignancies such as non-small cell carcinomas in the majority of colon, rectal, stomach, renal cell, liver, and lung cancers, small intestine cancer and esophagus cancer. Squamous cell carcinomas include malignancies, such as in the lung, esophagus, skin, head and neck region, oral cavity, anus and cervix. Metastatic lesions of the aforementioned cancers may also be treated or prevented using the methods and compositions of the present disclosure. The antibody molecule directed to CLDN18.2 may be combined with an immunogenic agent such as cancer cells, purified tumor antigens (including recombinant proteins, peptides and sugar molecules), cells and cells transfected with a gene encoding an immunostimulatory cytokine.

As used herein, term "antibody-dependent cell-mediated cytotoxicity" (ADCC) describes the cell killing ability of effector cells (in particular lymphocytes) which preferably requires the target cell being marked by an antibody. ADCC preferably occurs when antibodies bind to antigens on tumor cells and the antibody Fc domains engage Fc receptors (FcR) on the surface of immune effector cells. Several families of Fc receptors have been identified, and specific cell populations characteristically express defined Fc receptors. ADCC can be viewed as a mechanism to directly induce a variable degree of immediate tumor destruction that leads to antigen presentation and the induction of tumor-directed T-cell responses. Preferably, in vivo induction of ADCC will lead to tumor- directed T-cell responses and host-derived antibody responses.

As used herein, term "Complement-dependent cytotoxicity" (CDC) is another cell killing method that can be directed by antibodies. IgM is the most effective isotype for complement activation. IgG1 and IgG3 are also both very effective at directing CDC via the classical complement activation pathway. Preferably, in this cascade, the formation of antigen-antibody complexes results in the uncloaking of multiple C1q binding sites in close proximity on the CH2 domains of participating antibody molecules such as IgG molecules (C1q is one of three subcomponents of complement CI). Preferably these uncloaked C1q binding sites convert the previously low-affinity C1q-IgG interaction to one of high avidity, which triggers a cascade of events involving a series of other complement proteins and leads to the proteolytic release of the effector-cell chemotactic/activating agents C3a and C5a. Preferably, the complement cascade ends in the formation of a membrane attack complex, which creates pores in the cell membrane that facilitate free passage of water and solutes into and out of the cell.

It has been surprisingly found that the antibody-drug conjugates prepared with the anti-CLDN 18.2 antibodies herein are capable of mediating killing of cells, in particular of cells expressing CLDN18.2, such as cancer cells, by inducing Complement Dependent Cytotoxicity (CDC) mediated lysis and/or Antibody Dependent Cellular Cytotoxicity (ADCC) mediated lysis. Accordingly, in one embodiment, the antibody-drug conjugates of the present invention mediate cell killing by inducing Complement Dependent Cytotoxicity (CDC) mediated lysis and/or Antibody Dependent Cellular Cytotoxicity (ADCC) mediated lysis, preferably by inducing CDC mediated lysis and ADCC mediated lysis.

### BRIEF DESCRIPTION OF THE DRAWINGS

Embodiments of the present invention are described in detail below with reference to the attached figures, in which:
FIG. 1 shows the results of cell binding assay in vitro of anti-human CLDN18.2 chimeric antibodies.
FIG. 2 shows the results of Complement Dependent Cytotoxicity (CDC) assay of anti-human CLDN18.2 humanized antibodies, in which panel 2A: CHOK1; panel 2B: BxPC3; panel 2C: NCI-N87.
FIG. 3 shows the results of Antibody Dependent Cellular Cytotoxicity (ADCC) assay of anti-human CLDN18.2 humanized antibodies, in which panel 3A: CHOK1; panel 3B: BxPC3; panel 3C: NCI-N87.
FIG. 4 shows the result of analysis of binding characteristics of the anti-human CLDN18.2 humanized antibodies to proteins of the same family.
FIG. 5 shows the binding of anti-human CLDN18.2 humanized antibodies at different concentrations to human CLDN18.1 expressing cells.
FIG. 6 shows the results of analysis of species cross binding characteristics of anti-human CLDN18.2 humanized antibodies.
FIG. 7 shows the results of detected positive percentages of CAR-T cells prepared in different groups.
FIG. 8 shows different antigen expression profiles of different CHO cell strains constructed.
FIG. 9 shows the results of detected IFN-γ in supernatants after the incubation of CAR-T cells in different groups with target cells.
FIG. 10 shows the results of detected CD25 expression on CAR-T cells in different groups after incubation with target cells.

### DETAILED DESCRIPTION OF PREFERRED EMBODIMENTS

The present invention is illustrated below with reference to specific examples. It will be understood by those skilled in the art that these examples are merely illustrative of the present invention and do not limit the scope of the present invention in any way.

Experimental procedures in the following examples are all conventional ones, unless otherwise specified. Raw materials and reagents used in the following examples are all commercially available products, unless otherwise specified.

The heavy and light chain sequences of IMAB362 are as shown in SEQ ID NO. 126 and SEQ ID NO. 127.

Antigen human CLDN18.2 is as shown in NP_001002026.1, and antigen human CLDN18.1 is as shown in NP_057453.1.

The negative control (Isotype control) in the figures is anti-CD33 IgG antibody Lintuzumab in full length.

### Example 1 Screening for murine monoclonal antibodies

Balb/c mice were immunized with CHOK1 cells stably expressing human CLDN18.2 protein on cell surfaces. One month later, sera from the mice were analyzed by flow cytometry (FACS), and spleens were taken from mice having high antibody titers in sera. The spleen cells isolated by standard method were fused with myeloma cells P3X63Ag8.653 using PEG or electrofusion methods. The fused hybridoma cells were seeded in 384 well plates and after 10-14 days of culture, supernatants obtained were analyzed by FACS for antibody secretion by the hybridoma cells. Several clones that were able to bind to CHOK1 cells stably expressing human CLDN18.2 protein on cell surfaces and were not able to bind to CHOK1 cells stably expressing human CLDN18.1 protein on cell surfaces were obtained. Single cells of the obtained clones were obtained by Limiting Dilution, and each of monoclonal hybridoma cell clones obtained after diluted for three times secreted only one antibody.

The monoclonal hybridoma cells secreting anti human CLDN18.2 were subjected to expansion culture, and total RNA of the cells was extracted using RNAfast200 Kit (Shanghai Flytech Biotechnology Co., Ltd.) according to the steps described in Kit instructions; the total RNA of the hybridoma cells obtained was reverse transcribed to cDNA using 5×PrimeScript RT Master Mix (Takara); and sequences of antibody light chain variable region IgVL (κ) and heavy chain variable region VH were amplified using degenerate primers (Anke Krebber., 1997) and Extaq PCR reagents (Takara). PCR amplification products were purified using PCR Clean-up Gel Extraction Kit (Macherey-Nagel GmbH & Co.); and linked to T-vector using pClone007 Simple Vector Kit (Tsingke Biotechnology Co., Ltd.) according to Kit instructions, and transformed into competent Escherichia coli cells. Variable region sequences of the monoclonal antibodies were obtained by DNA sequencing after strain amplification and plasmid extraction.

**Table 1. Light and heavy chain variable regions of murine antibodies**

| Murine antibody | Heavy chain variable region (VH) | Light chain variable region (VL) |
|---|---|---|
| 11M23 | SEQ ID NO: 1 | SEQ ID NO: 2 |
| 16K15 | SEQ ID NO: 3 | SEQ ID NO: 4 |
| 18B21 | SEQ ID NO: 5 | SEQ ID NO: 6 |
| 20L17 | SEQ ID NO: 7 | SEQ ID NO: 8 |
| 43B5 | SEQ ID NO: 9 | SEQ ID NO: 10 |
| 43L6 | SEQ ID NO: 11 | SEQ ID NO: 12 |
| 46J05 | SEQ ID NO: 13 | SEQ ID NO: 14 |
| 48G12 | SEQ ID NO: 15 | SEQ ID NO: 16 |
| 50C14 | SEQ ID NO: 17 | SEQ ID NO: 18 |
| 52E22 | SEQ ID NO: 19 | SEQ ID NO: 20 |
| 8K13 | SEQ ID NO: 21 | SEQ ID NO: 22 |

>11M23_vh (SEQ ID NO: 1) 
QVQLKESGPDLVAPSQSLSITCTVSGFSLTNYGVHWVRQPPGKGLEWLVVIWSDGRINYN
SALKSRLSITKDNSKRQVFLKMNSLQIDDTAIYYCVRHPAFGPHAMDYWGQGISVTVSS
>11M23_vl(SEQ ID NO: 2) 
DIVMTQDAPSIPVTPGESVSISCRSSKSLLNSNGNTYLYWFLQRPGQSPHLLLYRMSNPAS
GAPDRFSGSGSGTEFTLRISRVEAEDVGVYYCMQYLEYPPTFGAGTRLELK
>16K15 vh (SEQ ID NO: 3) 
EVMLVESGGGLVRPGGSLKLSCAGSGITLSTYAMSWVRQTPERRLEWVASIISGGITYYL
DSVKGRFTISRDNARNILYLQMSSLRSEDTAIYYCARKYHGNALDYWGQGTSVTVSL
>16K15 _vl(SEQ ID NO: 4) 
DIVMTQSPSSLPVTAGETVTMRCKSSQSLLNSGNQRNYLTWYQRKPGQPPKKLIYWASTR
ESGVPDRFTGSGSGTDFTLTISGVQAEDLAVYYCQNNYFYPLTFGAGTKLELK
>18B21_vh (SEQ ID NO: 5) 
QIQMVQSGPELKKPGETVRISCKASGYSFTTAGMQWVRKMPGEGLKWIGWIIAHSGEPK
YTEDFKGRFAFSLETSASTTYLQISNLKNEDTATYFCARWGKGNTMDYWGQGTSVIVSS
>18B21_vl (SEQ ID NO: 6) DIVMTQSPSSLTVTAGEKVTMSCKSSQSLLNGGNQRNYLTWYQQKPGQPPKLLIYWAST RESGVPDRFTGSGSGTHFTLTISSVQAEDLAVYYCQNAYFFPLTFGAGTKLELK
>20L17 _vh (SEQ ID NO: 7) 
DVQLVESGGGLVQPGGSRKLSCAASGFTFSSFGMHWVRQAPEKGLEWVAYISSGSSTIYY
PDTVKGRFTVSRDNPKNTLFLQMTSLRSEDTAMYYCVRLGPRGNVMDHWGQGTSVTVS
S
>20L17 _vl (SEQ ID NO: 8) 
DIVMTQSPSSLTVTAGEKVTMSCKSSQSLLNSGNQRNYLTWYQQKPGQPPKLLIYWASTR
ESGVPDRFTGSGSGTDFTLTISSVQAEDLAVYYCQNVYFYPLTFGTGTKLELR
>43B5 _vh(SEQ ID NO: 9) 
DVQLQESGPDLVKPSQSLSLTCTVSGYSISGAYNWHWIRQFPGNKLEWLAYMQYSGSSN
YNPSFKSRISISRDTSKNQFFLQLKSVTTEDTATYYCARMYNGNSFLYWGQGTLVTVSA
>43B5 _vl(SEQ ID NO: 10) 
DIVMTQSPSSLTVTAGEKVTMNCKSSQSLFNSGNQKNYLTWYQQKPGQPPRLLIYWAST
RESGVPDRFTGSGSGTDFTLTISSVQAEDLSLYYCQNSYSYPLTFGAGTKLELK
>43L6_vh (SEQ ID NO: 11) 
QVQLKESGPDLVAPSQSLSLTCSVSGFSLTSYGIHWVRQPPGKGLEWLVVIWSDGRTTYN
SGLKSRLSISKDNSKSQVLLKMNSLRTDDTAIYYCVRHPAFGPHAMDYWGQGTSVTVSS
>43L6_vl (SEQ ID NO: 12) 
DIVMTQAAPSVPVTPGESVSISCRSSKSLLNSNGNTYLYWFLQRPGQSPQLLIYRMSNLAS
GVPDRFSGSGSGTDFTLRISRVEAGDVGVYYCMQYLEYPVTFGAGTKLELK
>46J05_vh (SEQ ID NO: 13) 
DVQLVESGGGLVQPGGSRKLSCAASGFTFSRFGMHWVRQAPKKGLEWVAYISSGSNTIY
YADTVKGRFTISRDNPKNTLFLQTTSLRSEDTAIYYCGRLGFYGNSFDHWGQGTLVTVSA
>46J05_vl (SEQ ID NO: 14) 
NILMTQSPSSLTVTAGEKVTMNCKSSQSLLNGGNQRNYLTWYQQKAGQPPKLLIYWAST
RESGVPDRFTGGGSGTDFTLTISSVQAEDLALYYCQNSYYYPLTFGAGTKLELK
>48G12 vh (SEQ ID NO: 15) 
EVQLRQSGPELVKPGASVKMSCKASGYTFTTYIINWVKQKPGQGLEWIGYINPYNDDTRY
NERVKGKATLTSDKSSSTAYMELSSLTSEDSAVYYCARFYFGNSFTYWGQGTLVTVSA
>48G12 _vl (SEQ ID NO: 16) 
DIVMTQSPSSLPVTVGERVTMTCKSSQGLFNSGNQRNYLTWYQQKPGQPPKLLIYWASTR
ESGVPDRFTGSGSGTDFTLTISSVQAEDLAIYYCQNNYIYPLTFGAGTKLELK
>50C14_vh (SEQ ID NO: 17) 
EVQLRQSGPELVKPGASVKMSCKASGYTFTTYIINWVKQKPGQGLEWIGYINPYNDGTRY
NERVKGKATLTSDKSSSTAYMELSSLTSEDSAVYYCARFHFGNSFTYWGQGTLVTVSA
>50C14_vl(SEQ ID NO: 18) 
DIVMTQSPSSLPVTTGEKVTMTCKSSQGLFNNGNQRNYLTWYQQKPGQPPKLLIYWAST
RESGVPDRFIGSGSGTDFTLTISSVQAEDLAIYYCQNNYIFPLTFGAGTKLELK
>52E22 vh (SEQ ID NO: 19) 
QIQLVQSGPELKKPGETVKISCKASGYTLTNYGMNWVRQAPGKGLKWMGWIRPNTGEPT
YAEDFKGRFVFSLETSAATAYLQITNLKSEDTSTYFCARLYRGNTLDNWGQGTSVIVSS
>52E22_vl (SEQ ID NO: 20) 
DIVMTQSPSSLTVTTGEKVTMSCKSSQNLLNSGNQRNYLTWYQQKPGQSPKLLIYWASTR
ESGVPYRFTGSGSGTDFTLTISSVQTDDLAIYYCQNGYSFPFTFGSGTKLEIK
>8K13_vh (SEQ ID NO: 21) 
QVHLQQSGAELVRPGSSVKISCKASGYAFSNYWMNWVRQRPGQGLEWIGQIYPGNGDT
KYSGKFNSKDTLTADKSSNTAYMQLNSLTSEDSAVYFCARFYYGNVMDYWGQGTSVTV
SS
>8K13_vl (SEQ ID NO: 22)

DIVLTQSPSSLTVTAGEKVTMSCKSSQTLLNGGNQKNYLTWYQQKSGQPPKLLIYWASTR
ESGVPDRFTGSGSGTDFTLTISSVQAEDLAVYYCQNGYSYPLTFGVGTKLELK

### Example 2 Preparation of anti-human CLDN18.2 chimeric antibodies

The heavy chain variable region sequence of each murine anti-human CLDN18.2 monoclonal antibody and the heavy chain constant region sequence of published human monoclonal antibody IgG1 subclass (see SEQ ID NO: 124) were spliced together and constructed into a mammalian cell expression vector; and the light chain variable region sequence of each murine anti-human CLDN18.2 monoclonal antibody and the light chain constant region sequence of published human monoclonal antibody kappa subclass (see SEQ ID NO: 125) were spliced together and constructed into a mammalian cell expression vector. The constructed heavy chain and light chain vectors of the anti-human CLDN18.2 chimeric antibodies were mixed in pairs, HEK293 cells were transfected with the vectors using Polyethyleneimine (PEI), and cell supernatants were collected about 7 days later. Anti-human CLDN18.2 chimeric antibody proteins were obtained via Protein A purification.

The chimeric antibodies of the present disclosure were named following a format "murine antibody abbreviation-xiIgG".

### Example 3 Cell binding assay in vitro of anti-human CLDN18.2 chimeric antibodies

The chimeric anti-human CLDN18.2 antibodies were diluted 2-fold in gradient from an initial concentration of 100 nM and solutions of each antibody of 16 concentrations were obtained totally. 10 µl of the solutions of each antibody of each concentration was added to a 384-well plate. CHOK1 cells expressing CLDN18.2 on cell surface were collected by centrifugation for 5 min at 100 g at room temperature, and the cells were washed with PBS containing 0.5% BSA once and then were centrifuged for 5 min at 100 g at room temperature. The cells were resuspended at a density of about 2×10⁶ cells/ml, and 10 µl was added to each well of the 384-well plate to which the antibody had been added. After incubation for 1 hour at 4 °C, fluorescently labeled secondary goat anti-human IgG antibody was added. After continued incubation for 1 hour at 4 °C, the mean fluorescence readings of the cell populations were analyzed by a flow cytometer.

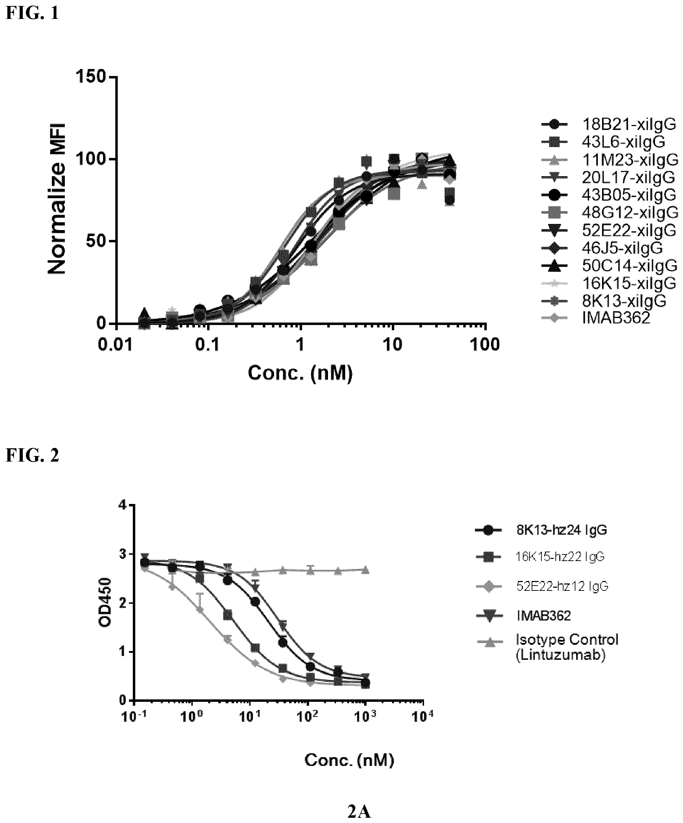

The results showed that the chimeric antibodies had specific binding to the cells expressing human CLDN18.2 at nM scale. See FIG. 1.

### Example 4 Humanization of anti-human CLDN18.2 murine antibodies

Based on a comprehensive analysis of Kabat, and Chothia antibody coding schemes, amino acid sequence regions of 6 complementarity-determining regions (CDRs) and framework regions supporting the conserved three-dimensional conformation of the heavy and light chains of each murine antibody were determined. Subsequently, the heavy chain variable region of human antibody which mostly resembles to the murine antibody, such as IGHV1 | IGHJ4^{∗}01, was searched for and selected in known human antibody sequences. The framework region sequences were selected as a template, and the heavy chain CDRs of the murine antibody were combined with the framework regions of the human antibody, and a humanized heavy chain variable region sequence was ultimately produced. In the same manner, a humanized light chain variable region sequence was produced.

An Antibody with murine CDRs grafted directly to its human framework regions often exhibits a dramatic decrease in binding activity, thus requiring the conversion of individual amino acids in the framework regions from being human back to murine. In order to determine which positions need to be reverted to original murine residues, the designed humanized antibody sequence and the original murine antibody sequence should be compared to check for differences in the amino acids, and to check whether those different amino acids are important for supporting the antibody structure or for binding to the antigen. The sequences obtained by humanization design need to be checked for potential post-Translational Modification Sites (PTMs), such as an N (asparagine) glycosylation site, an N-deamidation site, a D (aspartic acid) isomerization site, etc.

Gene of each humanized heavy chain variable region sequence was constructed into a mammalian cell expression vector comprising gene of the heavy chain constant region sequence of human monoclonal antibody IgG1 subclass; and gene of each humanized light chain variable region sequence was constructed into a mammalian cell expression vector comprising gene of the light chain constant region sequence of human monoclonal antibody kappa subclass. The constructed heavy chain and light chain vectors of the humanized anti-human CLDN18.2 antibodies were mixed in pairs, HEK293 cells were transfected with the vectors using Polyethyleneimine (PEI), and cell supernatants were collected about 7 days later. Anti-human CLDN18.2 humanized antibody proteins were obtained via Protein A purification.

Humanized antibodies of the present disclosure were named following a format "murine antibody abbreviation-hz". Antibodies having CDRs from murine antibodies grafted directly to their human framework regions were named following a format "murine antibody abbreviation-hzOO"; and antibodies further engineered were numbered with the numbers of the humanized sequences.

Binding kinetic parameters of the chimeric antibodies and the humanized antibodies thereof to the antigen human CLDN18.2 were analyzed by Fortebio (BLITZ pro1.1.0.28) instrument. Before performing the assay, an NTA bioprobe was soaked in PBS for 10 min; and the probe was then placed in PBS containing 100 nM antigen for 300 s to capture the His-tagged antigen. The probe was further subjected to a binding reaction with 100 nM antibody for a binding time of 400 s; and the probe was then transferred to PBS and subjected to a dissociation reaction for 600 s. When the assay was finished, data from which the response value of blank control had been deducted were fitted to a 1:1 Langmuir binding model using software, and then kinetic constants for antigen-antibody binding were calculated. The results are shown in Table 2.

**Table 2. Comparison of binding kinetics parameters following the humanization of murine antibodies**

| Ab ID | PTM | Response | K_{D}(M) | Kₒₙ | k_{off}(s⁻¹) | K_{off}/ |
|---|---|---|---|---|---|---|
| | | | | (1/Ms) | | K_{off}(Xi-IgG) |
| 8K13-xiIgG | Yes | 0.134 | 3.82E-10 | 2.98E+05 | 1.14E-04 | 1.00 |
| 8K13-hz00 | Yes | 0.133 | 7.44E-10 | 2.66E+05 | 1.98E-04 | 1.74 |
| 8K13-hz11 | Yes | 0.139 | 2.97E-10 | 2.27E+05 | 6.73E-05 | 0.59 |
| 8K13-hz24 | No | 0.133 | 1.51E-09 | 2.64E+05 | 3.99E-04 | 3.50 |
| 11M23-xiIgG | Yes | 0.184 | 2.93E-10 | 3.66E+05 | 1.07E-04 | 1.00 |
| 11M23-hz00 | Yes | 0.164 | 1.49E-09 | 2.18E+05 | 3.24E-04 | 3.03 |
| 11M23-hz11 | Yes | 0.174 | 1.29E-09 | 3.56E+05 | 4.60E-04 | 4.30 |
| 11M23-hz22 | No | 0.136 | 2.92E-09 | 6.70E+05 | 1.95E-03 | 18.22 |
| 16K15-xiIgG | Yes | 0.261 | 5.57E-10 | 2.76E+05 | 1.54E-04 | 1.00 |
| 16K15-hz00 | Yes | 0.176 | 8.95E-11 | 2.40E+05 | 2.15E-05 | 0.14 |
| 16K15-hz11 | Yes | 0.17 | 5.35E-10 | 1.88E+05 | 1.00E-04 | 0.65 |
| 16K15-hz22 | No | 0.266 | 1.10E-09 | 2.59E+05 | 2.86E-04 | 1.86 |
| 52E22-xiIgG | Yes | 0.223 | 9.96E-11 | 3.57E+05 | 3.55E-05 | 1.00 |
| 52E22-hz00 | Yes | 0.188 | 3.09E-10 | 1.98E+05 | 6.11E-05 | 1.72 |
| 52E22-hz11 | Yes | 0.238 | 5.42E-10 | 3.63E+05 | 1.97E-04 | 5.55 |
| 52E22-hz12 | No | 0.226 | 3.60E-10 | 2.18E+05 | 7.83E-05 | 2.21 |

The results showed that the dissociation constant of 1 1M23-hz22, relative to the murine chimeric antibody, was raised more than 10 fold after humanization and removal of critical post translational modification site (PTM) and was therefore eliminated from the list of candidates. The dissociation constants of the remaining 8K13-hz24, 16K15-hz22 and 52E22-hz12 were raised 1 to 4 folds, compared to original murine chimeric antibodies and could be used as lead molecules for subsequent studies. The light chain amino acid sequence of 16K15-hz22 contained two consecutive "NN" residues, and further optimized mutant antibodies 16K15-hz22_2 and 16K15-hz22_3 were obtained by changing the "NN" residues to "SN" and "QN" residues, respectively.

**Table 3. Light and heavy chain variable regions of humanized antibodies**

| Murine antibody | Humanized light chain variable region | Humanized heavy chain variable region | Humanized antibody |
|---|---|---|---|
| 16K15 | SEQ ID NO. 128 | SEQ ID NO. 129 | 16K15-hz00 |
| | SEQ ID NO. 128, having reverse mutations at positions 52 and 89 | SEQ ID NO. 129, having reverse mutation at position 24 | 16K15-hz11 |
| | SEQ ID NO. 24 | SEQ ID NO. 23 | 16K15-hz22 |
| | SEQ ID NO. 25 | | 16K15-hz22_2 |
| | SEQ ID NO. 26 | | 16K15-hz22_3 |
| 52E22 | SEQ ID NO. 130 | SEQ ID NO. 131 | 52E22-hz00 |
| | SEQ ID NO. 130, having reverse mutation at position 89 | SEQ ID NO. 27 | 52E22-hz11 |
| | SEQ ID NO. 28 | SEQ ID NO. 27 | 52E22-hz12 |
| 8K13 | SEQ ID NO. 132 | SEQ ID NO. 133 | 8K13-hz00 |
| | SEQ ID NO. 132, having reverse mutations at positions 4 and 89 | SEQ ID NO. 133, having reverse mutations at positions 74 and 77 | 8K13-hz11 |
| | SEQ ID NO. 30 | SEQ ID NO. 29 | 8K13-hz24 |
| 11M23 | SEQ ID NO. 88 | SEQ ID NO. 84 | 11M23-hz00 |
| | SEQ ID NO. 88, having reverse mutation at position 41 | SEQ ID NO. 84, having reverse mutation at position 71 | 11M23-hz11 |
| | SEQ ID NO. 88, having reverse mutation at position 41 as well as N31S and N33S | SEQ ID NO. 84, having reverse mutation at position 71 as well as D54E and N60Q | 11M23-hz22 |

>16K15_vl_hz2 (SEQ ID NO: 24) 
DIVMTQSPDSLAVSLGERATINCKSSQSLLSSGNQRNYLTWYQQKPGQPPKKLIYWASTR
ESGVPDRFSGSGSGTDFTLTISSLQAEDLAVYYCQNNYFYPLTFGQGTKLEIK
>16K15_vl_hz2_N-S (SEQ ID NO: 25) 
DIVMTQSPDSLAVSLGERATINCKSSQSLLSSGNQRNYLTWYQQKPGQPPKKLIYWASTR
ESGVPDRFSGSGSGTDFTLTISSLQAEDLAVYYCQSNYFYPLTFGQGTKLEIK
>16K15_vl_hz2_N-Q (SEQ ID NO: 26) 
DIVMTQSPDSLAVSLGERATINCKSSQSLLSSGNQRNYLTWYQQKPGQPPKKLIYWASTR
ESGVPDRFSGSGSGTDFTLTISSLQAEDLAVYYCQQNYFYPLTFGQGTKLEIK
>16k15_vh_hz2 (SEQ ID NO: 23) 
EVQLVESGGGLVQPGGSLRLSCAGSGITLSTYAMSWVRQAPGKGLEWVSSIISGGITYYL
DSVKGRFTISRDNAKNTLYLQMSSLRAEDTAVYYCARKYHGNALDYWGQGTLVTVSS
>52E22_vl_hz2 (SEQ ID NO: 28) 
DIVMTQSPDSLAVSLGERATINCKSSQNLLSSGNQRNYLTWYQQKPGQPPKLLIYWASTR
ESGVPDRFSGSGSGTDFTLTISSLQAEDLAVYYCQQGYSFPFTFGQGTKLEIK
>52E22_vh_hz1 (SEQ ID NO: 27) 
QIQLVQSGSELKKPGASVKVSCKASGYTLTNYGMNWVRQAPGQGLEWMGWIRPNTGEP
TYAEDFKGRFVFSLDTSVATAYLQITSLKAEDTAVYYCARLYRGNTLDNWGQGTLVTVS
S
>8K13_vl_hz4_N-S_N-Q (SEQ ID NO: 30) 
DIVLTQSPDSLAVSLGERATINCKSSQTLLSGGNQKNYLTWYQQKPGQPPKLLIYWASTR
ESGVPDRFSGSGSGTDFTLTISSLQAEDLAVYYCQQGYSYPLTFGQGTKLEIK
>8K13_vh_hz2 (SEQ ID NO: 29)

QVQLVQSGAEVKKPGSSVKVSCKASGYAFSNYWMNWVRQAPGQGLEWMGQIYPGSGD
TKYSGKFQSRVTITADKSTNTAYMELSSLRSEDTAVYYCARFYYGNVMDYWGQGTLVT
VSS

### Example 5 Kinetic experiment of binding affinity in vitro of anti-human CLDN18.2 humanized antibodies

Antibody-antigen interactions were measured using BIAcore S200 from GE Healthcare.

Referring to the instructions provided in Human Antibody Capture Kit (cat No. BR-1008-39, Lot 10261753) from GE Healthcare, the analytical channel and the control sample channel on CM5 sensor chip were first saturated and coupled with maximum amount of anti-human Fc antibody, then buffers containing anti-human CLDN18.2 chimeric antibodies, humanized antibodies or control antibody IMAB362 at 7.5 µg/ml were allowed to flow through the analytical channel and uniformly distribute; and finally antigen samples diluted in gradient (the initial concentration was 20 nM and diluted by 1:3 to get 8 concentrations, and the concentration 0.741 nM was set to be repeated) were allowed to flow through both the analytical channel and the sample channel, and the photoreactions upon the binding of antibody to antigen were measured. Subsequently, the association constant Kon and dissociation constant Koff and affinity constant KD of each antibody were finally obtained by instrument software fitting analysis.

The results showed that the in vitro binding affinity constants of the anti-human CLDN18.2 humanized antibodies were not significantly different from those of the original murine antibodies, while were lower than that of IMAB362 one order of magnitude. See Table 4.

**Table 4. Binding kinetics of antibodies of the present disclousre**

| Antibody | ka (M-1s-1) | kd (s-1) | KD (M) |
|---|---|---|---|
| 8K13-xiIgG | 8.19E+04 | 5.73E-04 | 6.99E-09 |
| 8K13-hz24 | 7.09E+04 | 2.02E-03 | 2.85E-08 |
| 16K15-xiIgG | 1.07E+05 | 1.44E-03 | 1.35E-08 |
| 16K15-hz22 | 5.71E+04 | 5.63E-04 | 9.85E-09 |
| 52E22-xiIgG | 4.90E+05 | 5.45E-04 | 1.11E-09 |
| 52E22-hz12 | 1.46E+05 | 1.72E-04 | 1.18E-09 |
| IMAB362 | 1.48E+06 | 0.201 | 1.36E-07 |

### Example 6 Cytology assay in vitro of anti-human CLDN18.2 humanized antibodies

### 6.1 Complement Dependent Cytotoxicity (CDC)

The humanized antibodies of the present disclosure were analyzed for their ability to induce complement-dependent cytotoxicity (CDC) on CHOK1, BxPC3 and NCI-N87 cells stably expressing human CLDN18.2 using a commercial human serum whole complement available by Quidel, Inc.

Cells were mixed with the antibody to be tested at a final concentration ranged from 250 µg/ml to 3.8 ng/ml; whole human serum at a concentration of 6.25% dissolved in cell culture medium RPMI-1640 was added into the mixture which was then incubated for 3 hours at 37 °C. Cytotoxicity was measured by a CCK-8 kit; finally, absorbance at 450 nm was measured by MD plate reader. EC50 values of the samples were calculated by plotting 4-parameter fitting curves with the absorbance values using the softmax pro7 software.

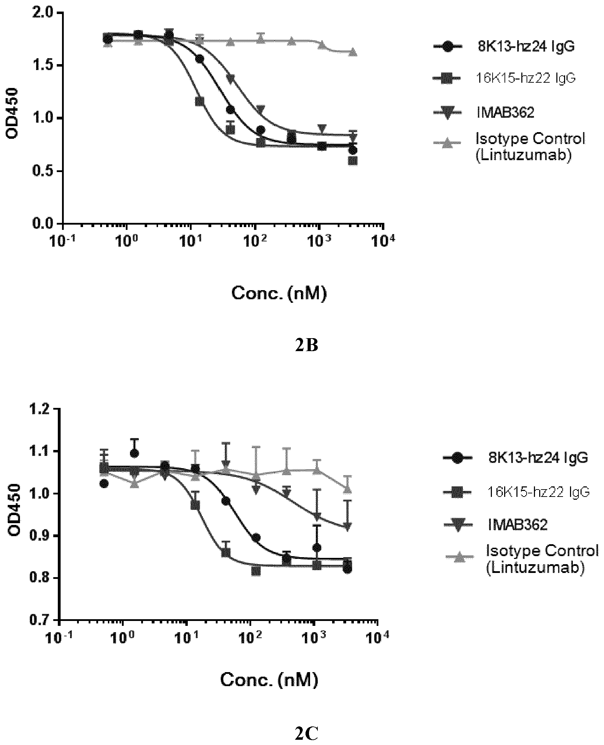

The results showed that the anti-human CLDN18.2 humanized antibodies had specific Complement Dependent Cytotoxicity (CDC) against target expressing cells and their cell killing activities were significantly superior to that of IMAB362. See FIG. 2 and Table 5.

**Table 5. CDC of the antibodies of the present disclosure**

| Target expressing cells | a. CHOK1 | b. BxPC3 | c. NCI-N87 |
|---|---|---|---|
| Antibody | EC50 (nM) | EC50 (nM) | EC50 (nM) |
| 8K13-hz24 IgG | 21.27 | 28.53 | 57.7 |
| 16K15-hz22 IgG | 5.2 | 12.22 | 17.36 |
| 52E22-hz12 IgG | 2.08 | NA | NA |
| IMAB362 | 30.45 | 52.23 | 474.2 |

### 6.2 Antibody Dependent Cellular Cytotoxicity (ADCC)

Engineered Jurkat cells stably expressing FcγRIIIa-FcεRIaγ hybrid receptor and expressing firefly Luciferase under the driving by NFAT response element were used as effector cells. The biological activity of an antibody in ADCC mechanism is quantified by Luciferase produced by activation of NFAT pathway. 1.5E5 effector cells were mixed with the antibody to be tested at a final concentration ranged from 33 µg/ml to 85 pg/ml, and then 2.5E4 target cells were added into the mixture (effector to target E: T ratio was 6:1) which was then incubated for 16 hours at 37 °C. Cytotoxicity was measured by a kit Bio-Glo^{™} Luciferase Assay System from Promega; and LUM values were finally determined by a MD plate reader.

The data were processed as follows: Fold induction = (reading value of the well detected - background value)/(reading value of the negative control well - background value). EC50 values of the samples were calculated by plotting 4-parameter fitting curves with the data.

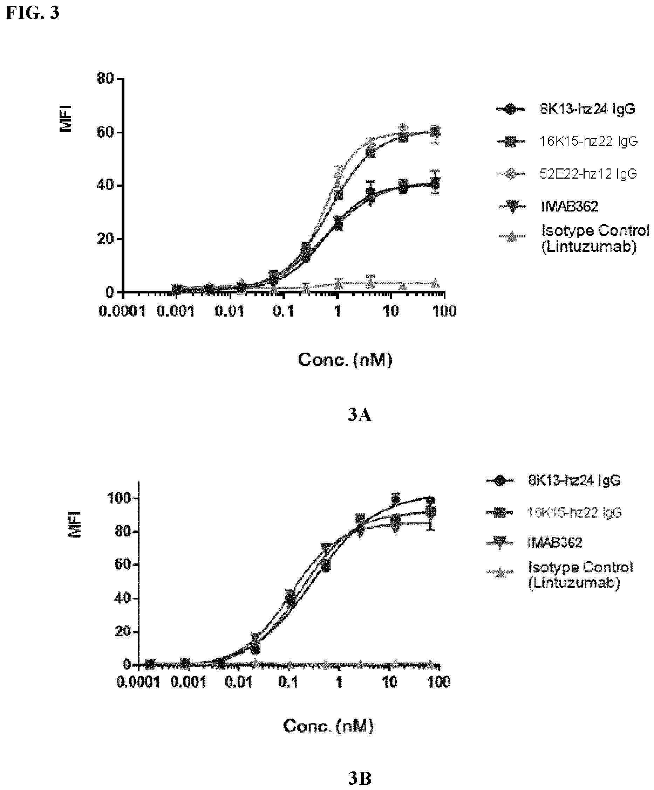

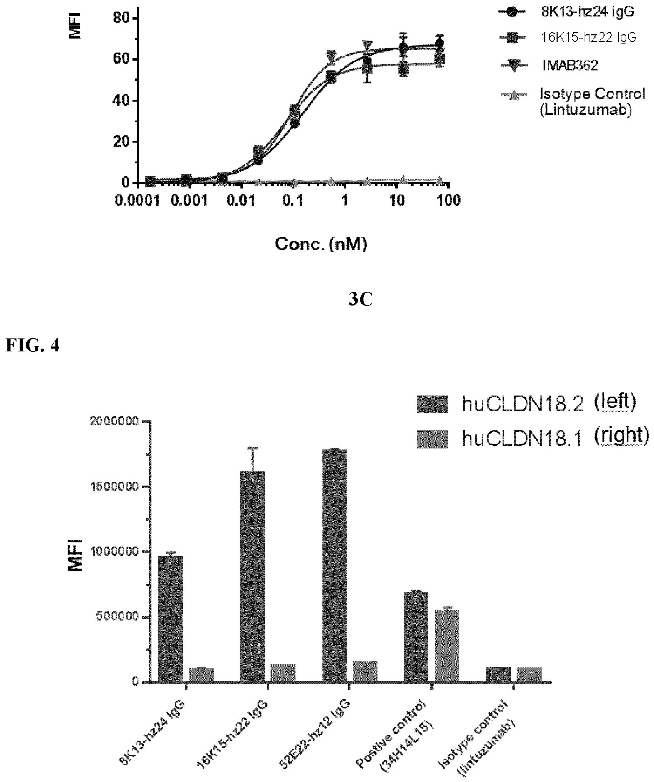

The results showed that the anti-human CLDN18.2 humanized antibodies had specific Antibody Dependent Cellular Cytotoxicity (ADCC) against target expressing cells and their cell killing activities were comparable to that of IMAB362. See FIG. 3 and Table 6.

**Table 6. ADCC of the antibodies of the present disclosure**

| Target expressing cells | a. CHOK1 | b. BxPC3 | c. NCI-N87 |
|---|---|---|---|
| Antibody | EC50 ( nM ) | EC50 ( nM ) | EC50 (nM ) |
| 8K13-hz24 IgG | 0.5894 | 0.3045 | 0.1499 |
| 16K15-hz22 IgG | 0.7122 | 0.1759 | 0.0677 |
| 52E22-hz12 IgG | 0.5672 | NA | NA |
| IMAB362 | 0.5929 | 0.1056 | 0.0909 |

### Example 7 Analysis of binding characteristics of the anti-human CLDN18.2 humanized antibodies to proteins of the same family

The genes of human CLDN18.2 and CLDN18.1 were constructed respectively into eukaryotic expression vectors and HEK293 cells were transfected with the vectors using Polyethyleneimine (PEI). Three days after the transfection, the cells were collected through centrifuging, washed with PBS once, and resuspended at a cell density of 2×10⁶/ml. 10 µl was added to each wells of a 384-well plate and then humanized antibodies at different concentration were added into the wells. After incubation for 1 hour at 4 °C, fluorescently labeled secondary goat anti-human IgG antibody was added. After continued incubation for 1 hour at 4 °C, the mean fluorescence readings of the 384-well plate were read by a flow cytometer. The data were analyzed to obtain the binding characteristics of the cells to the anti-human CLDN18.2 humanized antibodies. The positive control used for the experiment was the commercial anti-human CLDN18 rabbit mAb 34H14L15 (available from Abcam corporation) and the negative Isotype control was the anti-CD33 IgG antibody Lintuzumab in full-length.

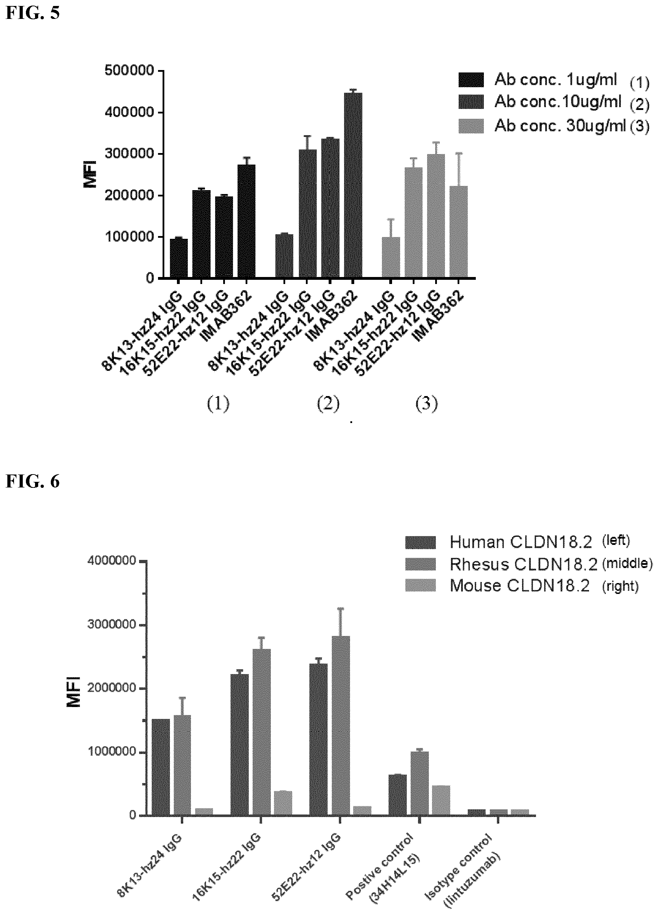

The results showed that the anti-human CLDN18.2 humanized antibodies exhibited characteristics of binding to human CLDN18.2 but not to human CLDN18.1. See FIG. 4. And, the non-specific binding to human CLDN18.1 cells at low, medium and high concentrations was all lower than that of IMAB362. See FIG. 5.

### Example 8 Analysis of human, monkey, and murine species cross binding characteristics of the anti-human CLDN18.2 humanized antibodies

The genes of human, murine and Rhesus CLDN18.2 were constructed respectively into eukaryotic expression vectors, and HEK293 cells were transfected with the vectors using Polyethyleneimine (PEI). The cells were collected 2 days later. The binding specificity of the cells to anti-human CLDN18.2 humanized antibodies was analyzed using flow cytometry. See Example 7 for the procedure of flow cytometry.

The results showed that the anti-human CLDN18.2 humanized antibody 16K15-22 IgG bound to CLDN18.2 of all three species, and 8K13-24 IgG and 52E22-12 IgG bound to Rhesus CLDN18.2 but not to mouse CLDN18.2. The results are shown in FIG. 6.

### Example 9 Preparation of CAR-T cells using anti-human CLDN18.2 humanized antibodies and activation thereof

### 9.1 Lentivirus packaging

Lentivirus packaging was performed according to the grouping shown in Table 7. Lentiviral vector plasmids pLTR containing different antibody genes were first constructed, and their plasmid DNA was extracted using a plasmid extraction kit from Qiagen after the DNA was confirmed correct by sequencing. The plasmid DNA was dissolved in sterile TE, and its concentration and purity were determined by UV light absorption, ensuring A260/A280 of the extracted plasmid DNA was between 1.8 and 2.0. The DNA of two helper packaging element plasmids, pCMV-VSV-G and pCMV-dR8.2, was extracted too. HEK293T cells for transfection were prepared to obtain freshly passaged cells which should grow to a confluency of about 60%. Three plasmids were co-transferred into HEK293T cells using calcium phosphate as transfection reagent. 48 hours after transfection, cell supernatant containing the packaged virus was collected by centrifugation at low temperature, and cell debris was removed using a 0.45 µm filter. The virus was concentrated using an ultrafiltration centrifuge tube, and sub-packaged and stored in a refrigerator at -80 °C. A small amount of the virus concentrate was taken and the virus titer was determined by FACS.

**Table 7. Lentivirus groupings**

| No. | Virus | Virus titer | Group |
|---|---|---|---|
| 135 | LV-135.N | 8.75E+06 | Negative control |
| 417 | LV-417.N | 1.65E+07 | 16k15 (16K15-hz22) scfv^{∗} |
| 418 | LV-418.N | 1.38E+07 | 52E22 (52E22-hz12) scfv^{∗} |
| 419 | LV-419.N | 5.13E+07 | 8K13 (8K13-hz24) scfv^{∗} |
| 420 | LV-420.N | 1.50E+07 | Positive control |

The negative control shown in Table 7 was another antibody not binding CLDN18.2 and the positive control was antibody hu8E5 from Carsgen Therapeutics (see WO2018006882A1). ^{∗}: The single chain antibody was formed by linking humanized VH and VL via a short peptide (GSTSGGGSGGGSGGGGSS).

### 9.2 Preparation of CAR-T cells

A healthy donor detected negative for HBV, HCV and HIV was selected. 100 ml of blood was collected from the antecubital vein, and Ficoll density gradient centrifugation was performed to isolate the white layer containing PBMCs. CD3+ T cells were isolated using DynaBeads CD3/CD28 (LifeTechnologies, Cat. No. 40203D) at a ratio of 3:1 of DynaBeads/CD3+ T cells. 24 hours after activation, percentage of CD25+ CD69+ T cells was detected by flow cytometry. When activated, the CD3+ T cells were transduced with the lentiviruses at an MOI of 5. A 24-well plate was coated with Novonectin for 2 hours at 37 °C; the cell suspension obtained by the procedure above was formulated into a suspension for transduction with various lentiviruses (MOI = 5), Synperonic^{®} F108 (Sigma, Cat. No. 07579-250G-F), and Tscm (2 U/ml). The suspension for transduction was added into the 24-well plate with the density of cells adjusted to 1.0E +06/ml. The plate was centrifuged at 500 g for 30 min, and placed in an incubator at 37 °C, 5% CO₂ for static culture for 48 h. After the transduction, the cells were cultured in 5% FBS X-vivo15 medium (LONZA, Cat. No. 04-418Q), supplemented with Tscm (at a final concentration of 2 U/ml) on alternate days. The cells were counted, adjusted to 0.5E +06/ml, and harvested on Day 8-10 of culture.

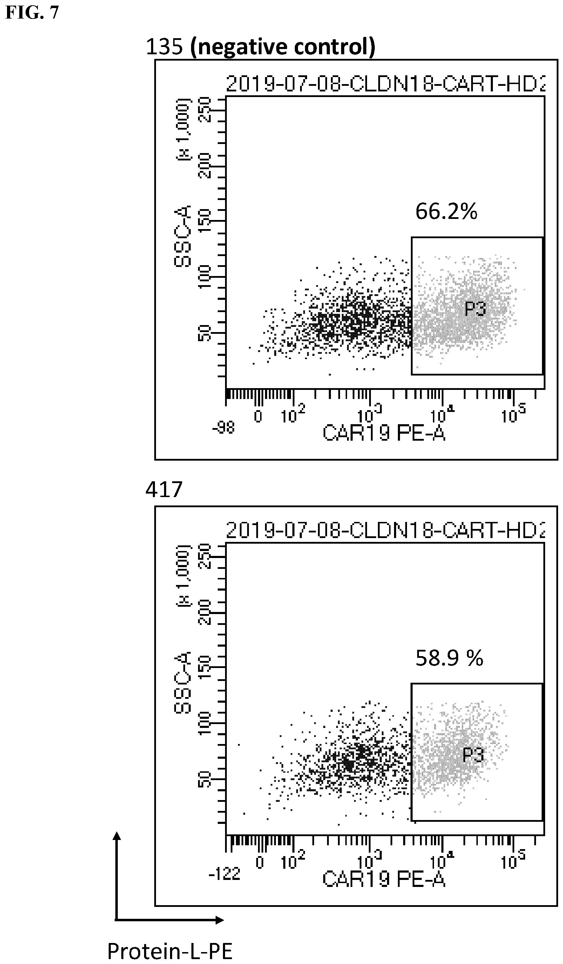

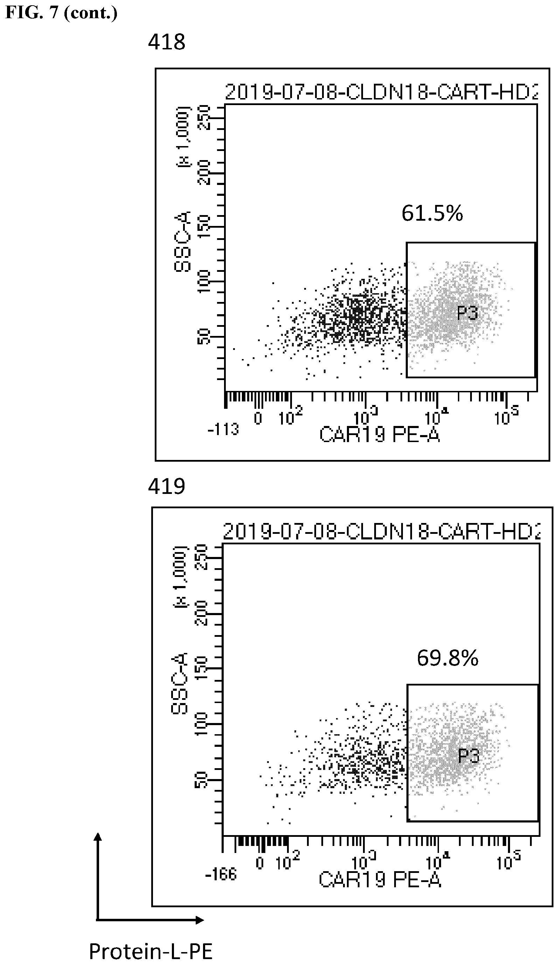

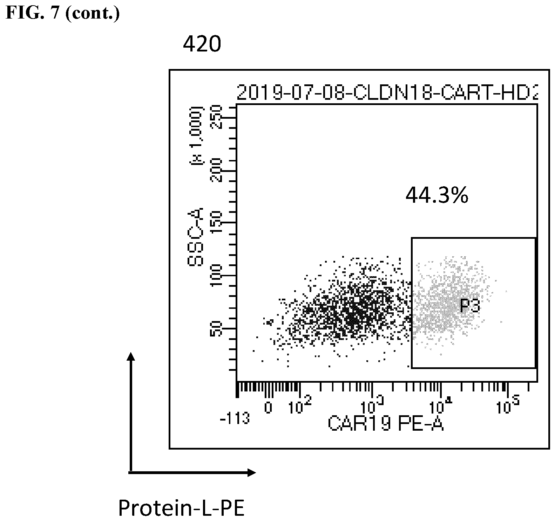

The results of positive percentages of CAR-T cells in each group obtained are shown in FIG. 7.

### 9.3 Activation of CAR-T cells

CHO cells expressing antigen human CLDN18.1 and antigen human CLDN18.2 were constructed and used as target cells.

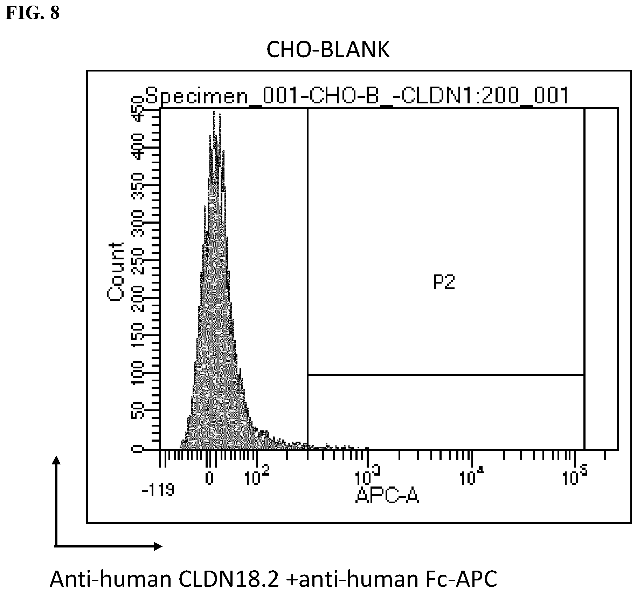

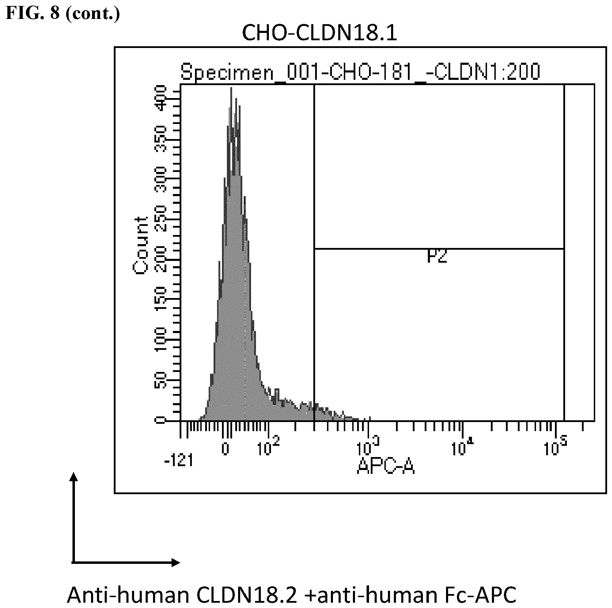

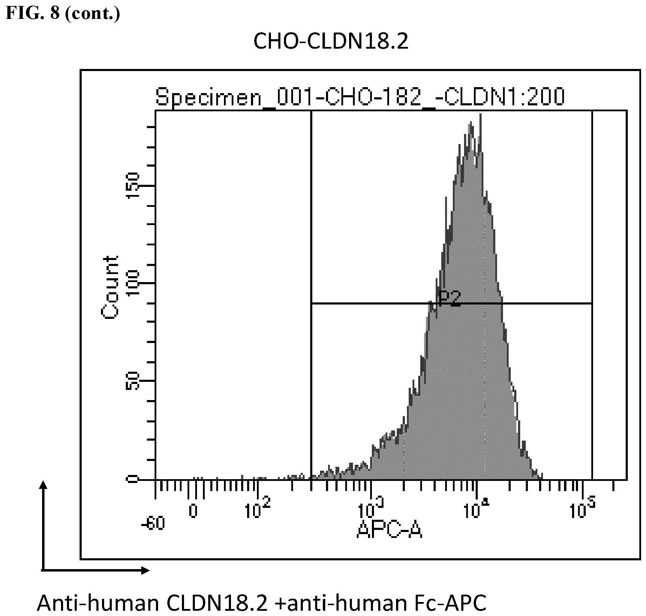

Detection results of antigen expression of the cells are shown in FIG. 8. CHO-BLANK (blank control) and CHO-CLDN18.1 had no expression of antigen CLDN18.2, and CHO-CLDN18.2 had a high expression of antigen CLDN18.2.

The effector cells with density adjusted described above, including each group of CAR-T cells and T cells without lentivirus transduction were mixed with the target cells in 1.5 mL centrifuge tubes respectively at an effective to target ratio of 16:1. Total volumes were replenished to 200 µL using T cell broth X-vivo15 (free of autologous serum and Tscm); and then 200 µL of the systems was respectively transferred into 96-well plate V-bottom for a 24-hour co-incubation.

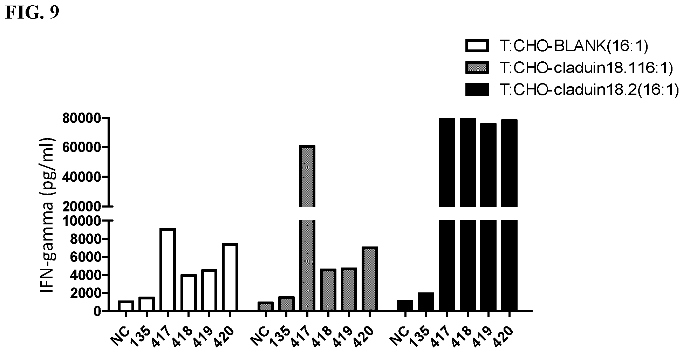

After the incubation, supernatant of each culture system was taken for the detection of human IFNγ. A significant increase in IFNγ release was found in the CAR-T cells in group 417 after incubatation with CHO-CLDN18.1 and CHO-CLDN18.2; and an increase in IFNγ release was found in the CAR-T cells in groups 418, 419, and 420 only after incubation with CHO-CLDN18.2. The results are shown in Tables 8 and FIG. 9.

**Table 8. Human IFNγ detected in the supernatants of the culture systems**

| | | | |
|---|---|---|---|
| Group | T: CHO-BLANK | T: CHO-claduin18.1 | T: CHO-claduin18.2 |
| nc | 1020.1 | 912 | 1095.2 |
| 135 | 1441.2 | 1458.6 | 1877 |
| 417 | 9016 | 60328.2 | 79178.6 |
| 418 | 3940.8 | 4540 | 78903.4 |
| 419 | 4486.2 | 4670.6 | 75516.8 |
| 420 | 7381.1 | 6973.2 | 78247 |

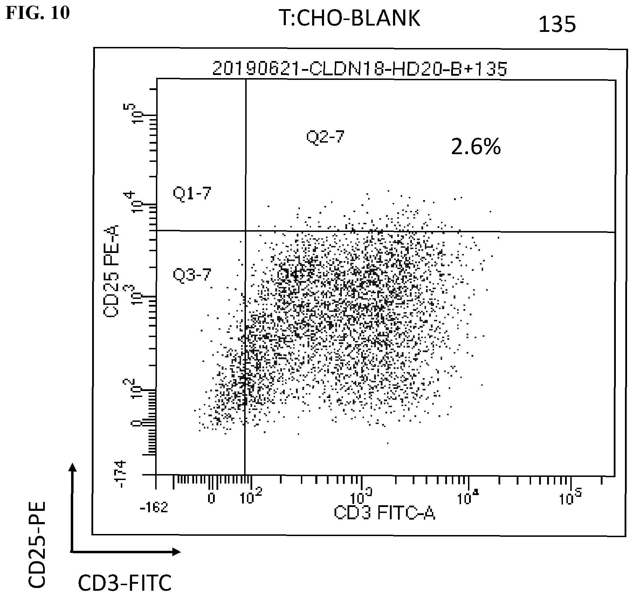

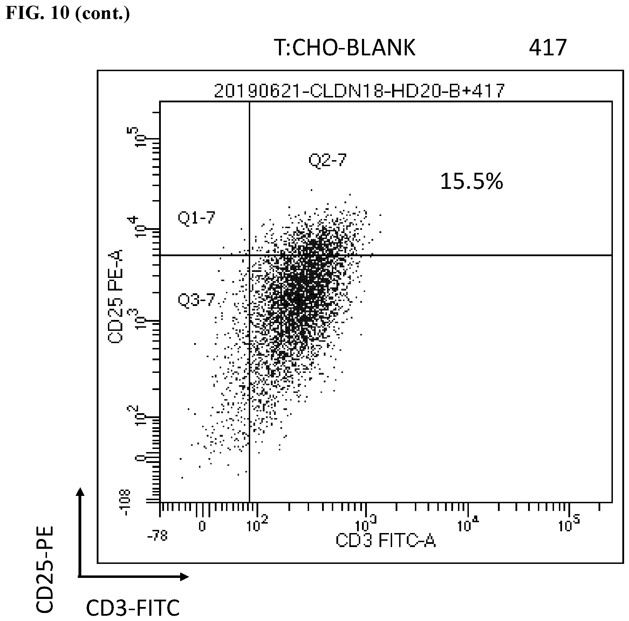

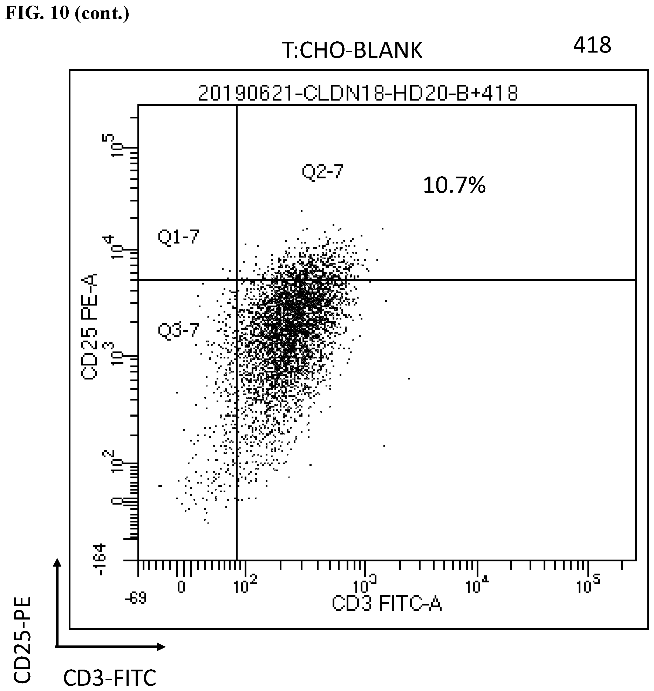

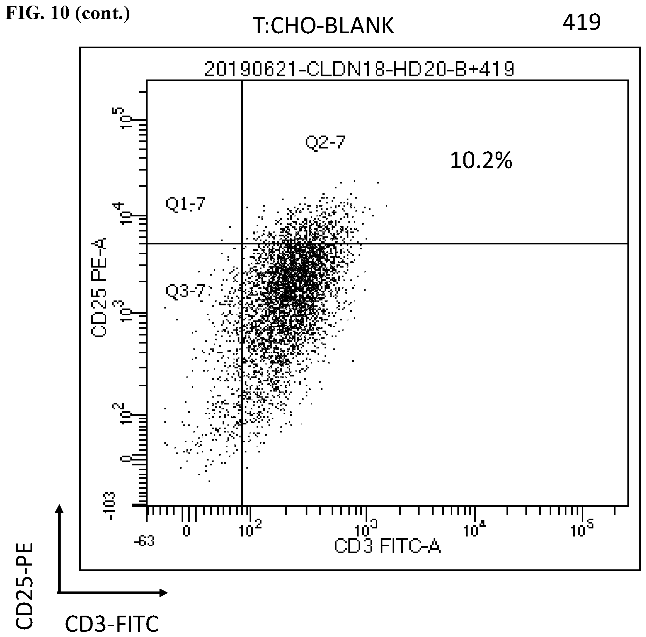

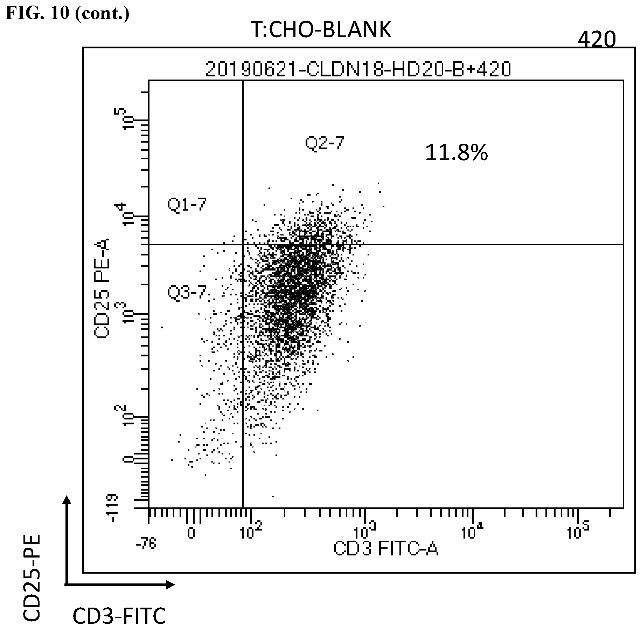

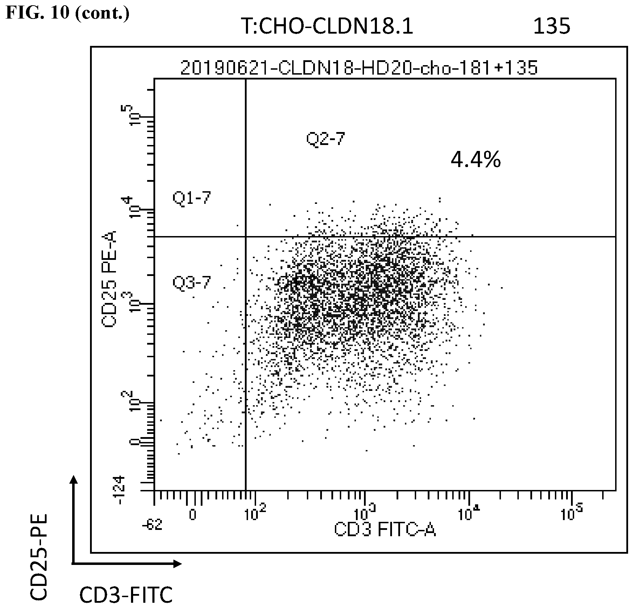

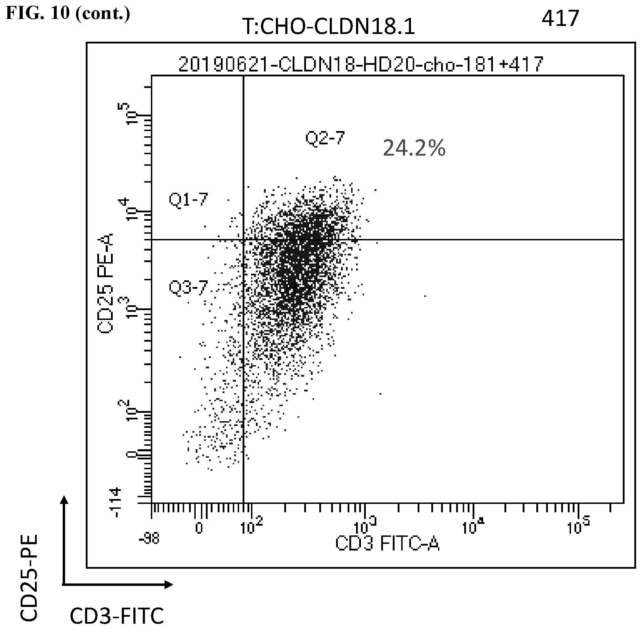

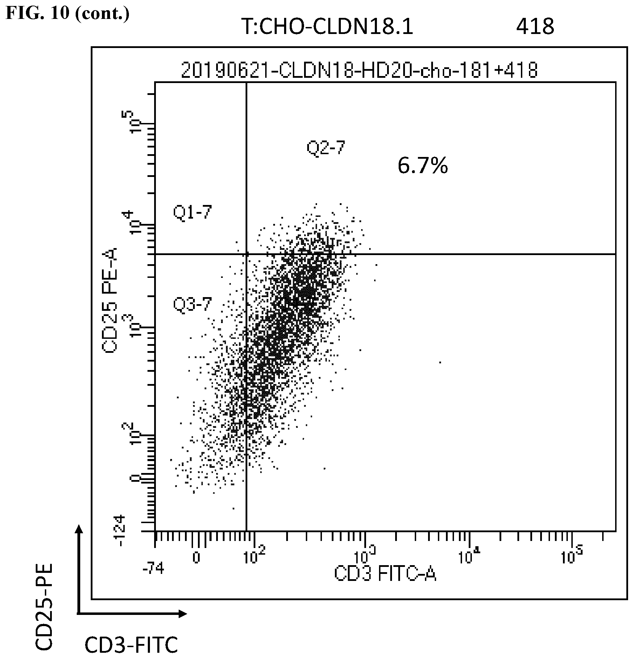

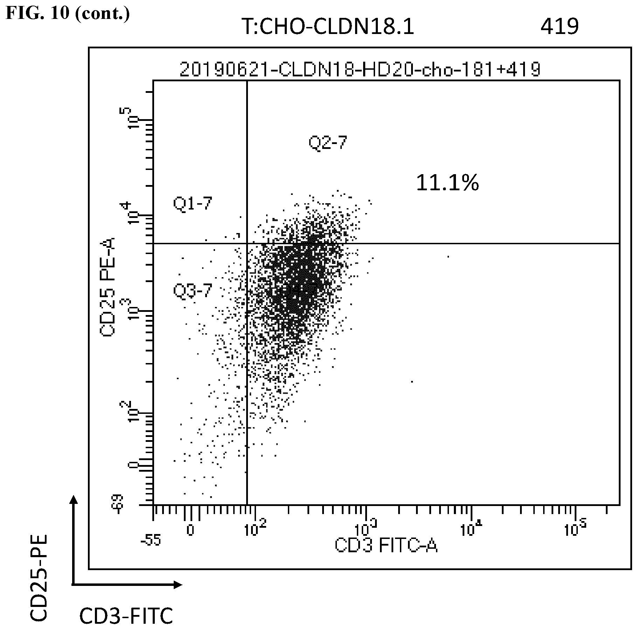

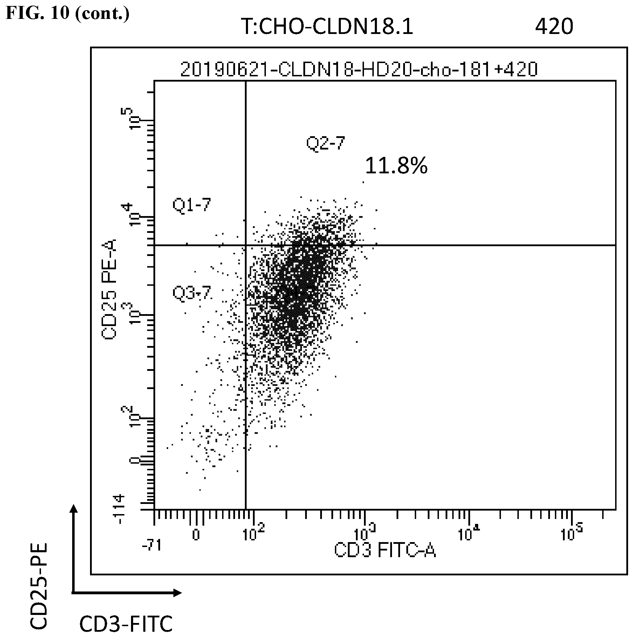

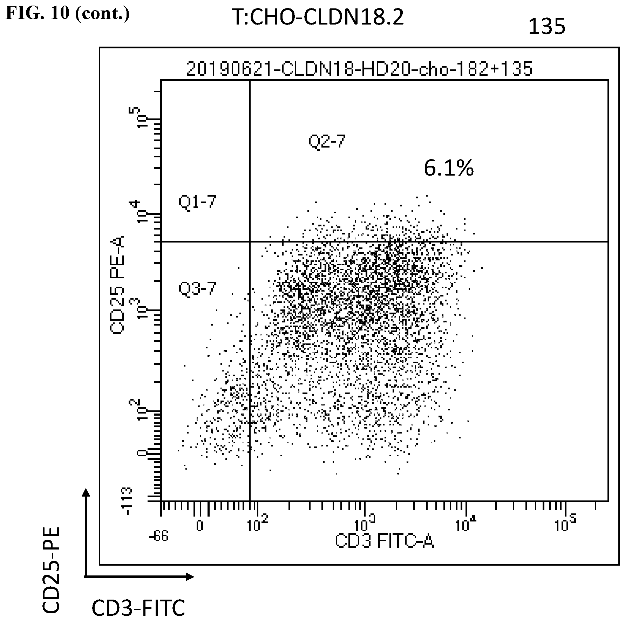

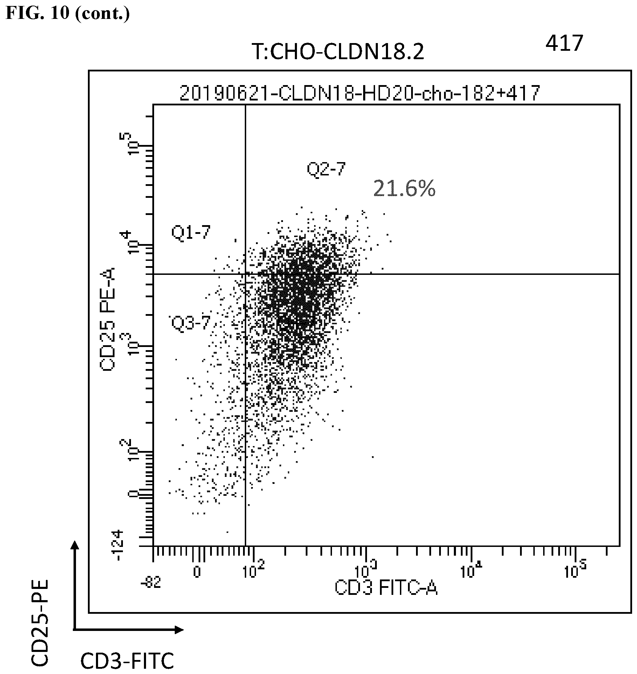

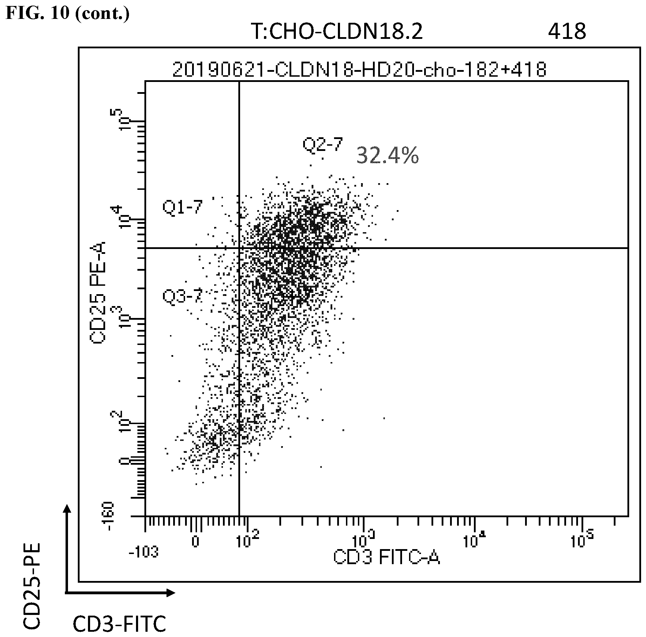

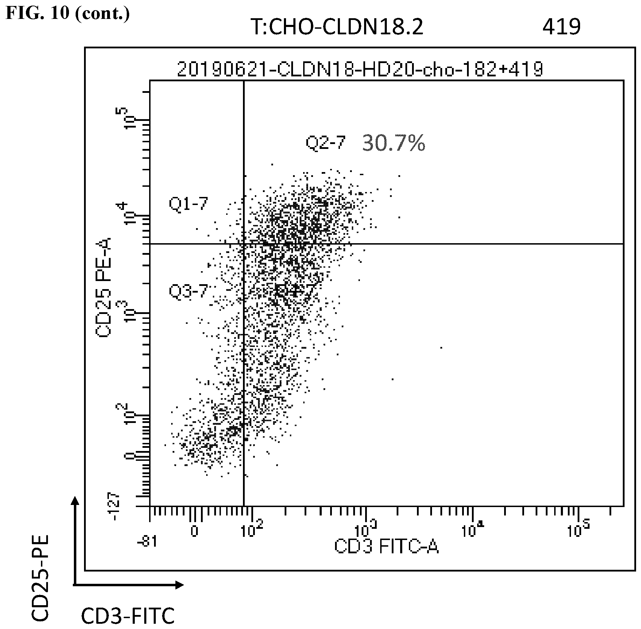

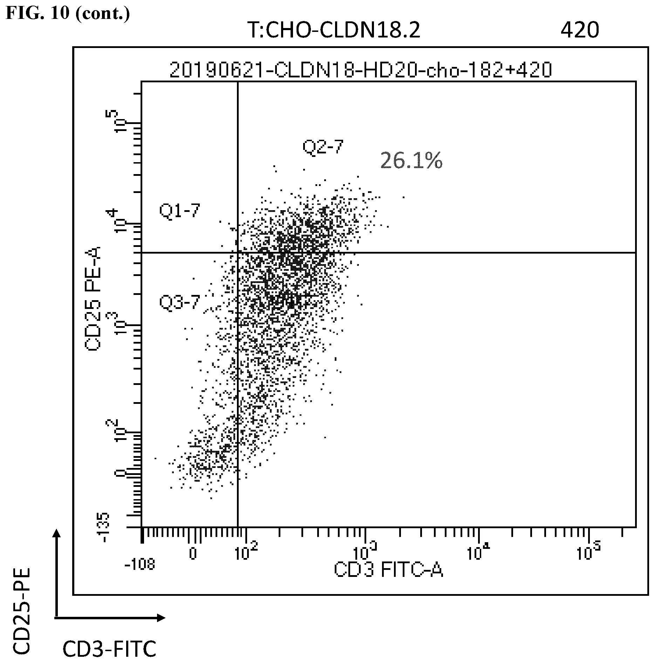

The CAR-T cells from each group were detected for T-cell activation marker proteins CD3/CD25. CD25 expression was found to be up-regulated in CAR-T cells in group 417 after incubation with CHO-CLDN18.1 and CHO-CLDN18.2 and up-regulated in CAR-T cells in groups 418, 419, and 420 CART only after incubation with CHO-CLDN18.2. See FIG. 10.

The above description of the embodiments of the present invention is not intended to limit the present invention, and those skilled in the art may make various changes and modifications to the present invention without departing from the spirit of the present invention, which should fall within the scope of the appended claims.

## Claims

1. A preparation method of an anti-human Claudin 18.2 antibody, the preparation method including steps as follows:
(1) immunizing an animal with cells expressing human Claudin 18.2 protein on surface as an immunogen;
(2) preparing cell clones capable of producing antibodies using the animals immunized in step (1);
(3) using cells expressing human Claudin 18.2 protein on surface as a positive screening antigen, screening antibodies having a binding activity to the positive screening antigen and cells producing the antibodies;
(4) using the cells expressing human Claudin 18.1 protein on surface as a negative screening antigen, excluding antibodies having a binding activity to the negative screening antigen and cells producing the antibody.

2. The preparation method according to claim 1, wherein the cells expressing the human Claudin 18.2 protein in step (1) are derived from the same species as the animal immunized; preferably, the cells expressing the human Claudin 18.2 protein are mouse cells, and the animal immunized is a mouse.

3. The preparation method according to claim 1, wherein the cell clones capable of producing antibodies in step (2) are prepared by a technique selected from the group consisting of hybridoma technique and single cell amplification technique.

4. The preparation method according to claim 1, wherein the positive screening antigen in step (3) is the same as the immunogen in step (1); and the negative screening antigen in step (4) differs from the immunogen in step (1) only in that the protein expressed is human Claudin 18.1 protein.

5. The preparation method according to claim 1, wherein step (3) and step (4) each is conducted by a method selected from the group consisting of Enzyme-Linked Immunosorbent Assay (ELISA) and Fluorescence Resonance Energy Transfer (FRET).

6. An anti-human Claudin 18.2 antibody obtained according to the preparation method of any one of claims 1 to 6.

7. The anti-human Claudin 18.2 antibody according to claim 6, which specifically binds the N-terminus of human Claudin 18.2, preferably extracellular region at the N-terminus of human Claudin 18.2 comprising the first Extracellular Loop (ECL1).

8. The anti-human Claudin 18.2 antibody according to claim 6, which has a specific binding to human CLDN18.2 at nM scale; and which has no significant difference in binding to human CLDN18.1 as compared to an isotype negative antibody (or an unrelated antibody).

9. An antibody or fragment thereof comprising a heavy chain variable region (VH) and a light chain variable region (VL), wherein the heavy chain variable region (VH) and the light chain variable region (VL) comprise a combination of CDRs (VH-CDR1, VH-CDR2, VH-CDR3; and VL-CDR1, VL-CDR2, VL-CDR3) selected from the group consisting of:
(1) VH-CDR1 as shown in SEQ ID NO. 31, VH-CDR2 as shown in SEQ ID NO. 32, and VH-CDR3 as shown in SEQ ID NO. 33; and VL-CDR1 as shown in SEQ ID NO. 34, VL-CDR2 as shown in SEQ ID NO. 35, and VL-CDR3 as shown in SEQ ID NO. 36;
(2) VH-CDR1 as shown in SEQ ID NO. 37, VH-CDR2 as shown in SEQ ID NO. 38, and VH-CDR3 as shown in SEQ ID NO. 39; and VL-CDR1 as shown in SEQ ID NO. 40, VL-CDR2 as shown in SEQ ID NO. 41, and VL-CDR3 as shown in SEQ ID NO. 42;
(3) VH-CDR1 as shown in SEQ ID NO. 43, VH-CDR2 as shown in SEQ ID NO. 44, and VH-CDR3 as shown in SEQ ID NO. 45; and VL-CDR1 as shown in SEQ ID NO. 46, VL-CDR2 as shown in SEQ ID NO. 41, and VL-CDR3 as shown in SEQ ID NO. 47;
(4) VH-CDR1 as shown in SEQ ID NO. 48, VH-CDR2 as shown in SEQ ID NO. 49, and VH-CDR3 as shown in SEQ ID NO. 50; and VL-CDR1 as shown in SEQ ID NO. 40, VL-CDR2 as shown in SEQ ID NO. 41, and VL-CDR3 as shown in SEQ ID NO. 51;
(5) VH-CDR1 as shown in SEQ ID NO. 52, VH-CDR2 as shown in SEQ ID NO. 53, and VH-CDR3 as shown in SEQ ID NO. 54; and VL-CDR1 as shown in SEQ ID NO. 55, VL-CDR2 as shown in SEQ ID NO. 41, and VL-CDR3 as shown in SEQ ID NO. 56;
(6) VH-CDR1 as shown in SEQ ID NO. 57, VH-CDR2 as shown in SEQ ID NO. 58, and VH-CDR3 as shown in SEQ ID NO. 33; and VL-CDR1 as shown in SEQ ID NO. 34, VL-CDR2 as shown in SEQ ID NO. 59, and VL-CDR3 as shown in SEQ ID NO. 60;
(7) VH-CDR1 as shown in SEQ ID NO. 61, VH-CDR2 as shown in SEQ ID NO. 62, and VH-CDR3 as shown in SEQ ID NO. 63; and VL-CDR1 as shown in SEQ ID NO. 46, VL-CDR2 as shown in SEQ ID NO. 41, and VL-CDR3 as shown in SEQ ID NO. 64;
(8) VH-CDR1 as shown in SEQ ID NO. 65, VH-CDR2 as shown in SEQ ID NO. 66, and VH-CDR3 as shown in SEQ ID NO. 67; and VL-CDR1 as shown in SEQ ID NO. 68, VL-CDR2 as shown in SEQ ID NO. 41, and VL-CDR3 as shown in SEQ ID NO. 69;
(9) VH-CDR1 as shown in SEQ ID NO. 65, VH-CDR2 as shown in SEQ ID NO. 70, and VH-CDR3 as shown in SEQ ID NO. 71; and VL-CDR1 as shown in SEQ ID NO. 72, VL-CDR2 as shown in SEQ ID NO. 41, and VL-CDR3 as shown in SEQ ID NO. 73;
(10) VH-CDR1 as shown in SEQ ID NO. 74, VH-CDR2 as shown in SEQ ID NO. 75, and VH-CDR3 as shown in SEQ ID NO. 76; and VL-CDR1 as shown in SEQ ID NO. 77, VL-CDR2 as shown in SEQ ID NO. 41, and VL-CDR3 as shown in SEQ ID NO. 78;
(11) VH-CDR1 as shown in SEQ ID NO. 79, VH-CDR2 as shown in SEQ ID NO. 80, and VH-CDR3 as shown in SEQ ID NO. 81; and VL-CDR1 as shown in SEQ ID NO. 82, VL-CDR2 as shown in SEQ ID NO. 41, and VL-CDR3 as shown in SEQ ID NO. 83;
(12) VH-CDR1 as shown in SEQ ID NO. 37, VH-CDR2 as shown in SEQ ID NO. 38, and VH-CDR3 as shown in SEQ ID NO. 39; and VL-CDR1 as shown in SEQ ID NO. 85, VL-CDR2 as shown in SEQ ID NO. 41, and VL-CDR3 as shown in SEQ ID NO. 42;
(13) VH-CDR1 as shown in SEQ ID NO. 37, VH-CDR2 as shown in SEQ ID NO. 38, and VH-CDR3 as shown in SEQ ID NO. 39; and VL-CDR1 as shown in SEQ ID NO. 85, VL-CDR2 as shown in SEQ ID NO. 41, and VL-CDR3 as shown in SEQ ID NO. 86;
(14) VH-CDR1 as shown in SEQ ID NO. 37, VH-CDR2 as shown in SEQ ID NO. 38, and VH-CDR3 as shown in SEQ ID NO. 39; and VL-CDR1 as shown in SEQ ID NO. 85, VL-CDR2 as shown in SEQ ID NO. 41, and VL-CDR3 as shown in SEQ ID NO. 87;
(15) VH-CDR1 as shown in SEQ ID NO. 74, VH-CDR2 as shown in SEQ ID NO. 75, and VH-CDR3as shown in SEQ ID NO. 76; and VL-CDR1 as shown in SEQ ID NO. 89, VL-CDR2 as shown in SEQ ID NO. 41, and VL-CDR3 as shown in SEQ ID NO. 90, and
(16) VH-CDR1 as shown in SEQ ID NO. 79, VH-CDR2 as shown in SEQ ID NO. 91, and VH-CDR3 as shown in SEQ ID NO. 81; and VL-CDR1 as shown in SEQ ID NO. 92, VL-CDR2 as shown in SEQ ID NO. 41, and VL-CDR3 as shown in SEQ ID NO. 93.

10. The antibody or fragment thereof according to any one of claims 6 to 9, wherein the heavy chain variable region comprises an amino acid sequence as shown in any one of SEQ ID NO. 1, SEQ ID NO. 3, SEQ ID NO. 5, SEQ ID NO. 7, SEQ ID NO. 9, SEQ ID NO. 11, SEQ ID NO. 13, SEQ ID NO. 15, SEQ ID NO. 17, SEQ ID NO. 19, SEQ ID NO. 21, SEQ ID NO. 23, SEQ ID NO. 27, and SEQ ID NO. 29 or an amino acid sequence having at least 75% identity to the amino acid sequence as shown; and/or the light chain variable region comprises an amino acid sequence as shown in any one of SEQ ID NO. 2, SEQ ID NO. 4, SEQ ID NO. 6, SEQ ID NO. 8, SEQ ID NO. 10, SEQ ID NO. 12, SEQ ID NO. 14, SEQ ID NO. 16, SEQ ID NO. 18, SEQ ID NO. 20, SEQ ID NO. 22, SEQ ID NO. 24, SEQ ID NO. 25, SEQ ID NO. 26, SEQ ID NO. 28, and SEQ ID NO. 30 or an amino acid sequence having at least 75% identity to the amino acid sequence as shown

11. The antibody or fragment thereof according to any one of claims 6 to 10, wherein the antibody or fragment thereof comprises a heavy chain variable region and a light chain variable region selected from the group consisting of:
(1) an amino acid sequence as shown in SEQ ID NO. 1 or an amino acid sequence having at least 75% identity to the amino acid sequence as shown in SEQ ID NO. 1; and an amino acid sequence as shown in SEQ ID NO. 2 or an amino acid sequence having at least 75% identity to the amino acid sequence as shown in SEQ ID NO. 2;
(2) an amino acid sequence as shown in SEQ ID NO. 3 or an amino acid sequence having at least 75% identity to the amino acid sequence as shown in SEQ ID NO. 3; and an amino acid sequence as shown in SEQ ID NO. 4 or an amino acid sequence having at least 75% identity to the amino acid sequence as shown in SEQ ID NO. 4;
(3) an amino acid sequence as shown in SEQ ID NO. 5 or an amino acid sequence having at least 75% identity to the amino acid sequence as shown in SEQ ID NO. 5; and an amino acid sequence as shown in SEQ ID NO. 6 or an amino acid sequence having at least 75% identity to the amino acid sequence as shown in SEQ ID NO. 6;
(4) an amino acid sequence as shown in SEQ ID NO. 7 or an amino acid sequence having at least 75% identity to the amino acid sequence as shown in SEQ ID NO. 7; and an amino acid sequence as shown in SEQ ID NO. 8 or an amino acid sequence having at least 75% identity to the amino acid sequence as shown in SEQ ID NO. 8;
(5) an amino acid sequence as shown in SEQ ID NO. 9 or an amino acid sequence having at least 75% identity to the amino acid sequence as shown in SEQ ID NO. 9; and an amino acid sequence as shown in SEQ ID NO. 10 or an amino acid sequence having at least 75% identity to the amino acid sequence as shown in SEQ ID NO. 10;
(6) an amino acid sequence as shown in SEQ ID NO. 11 or an amino acid sequence having at least 75% identity to the amino acid sequence as shown in SEQ ID NO. 11; and an amino acid sequence as shown in SEQ ID NO. 12 or an amino acid sequence having at least 75% identity to the amino acid sequence as shown in SEQ ID NO. 12;
(7) an amino acid sequence as shown in SEQ ID NO. 13 or an amino acid sequence having at least 75% identity to the amino acid sequence as shown in SEQ ID NO. 13; and an amino acid sequence as shown in SEQ ID NO. 14 or an amino acid sequence having at least 75% identity to the amino acid sequence as shown in SEQ ID NO. 14;
(8) an amino acid sequence as shown in SEQ ID NO. 15 or an amino acid sequence having at least 75% identity to the amino acid sequence as shown in SEQ ID NO. 15; and an amino acid sequence as shown in SEQ ID NO. 16 or an amino acid sequence having at least 75% identity to the amino acid sequence as shown in SEQ ID NO. 16;
(9) an amino acid sequence as shown in SEQ ID NO. 17 or an amino acid sequence having at least 75% identity to the amino acid sequence as shown in SEQ ID NO. 17; and an amino acid sequence as shown in SEQ ID NO. 18 or an amino acid sequence having at least 75% identity to the amino acid sequence as shown in SEQ ID NO. 18;
(10) an amino acid sequence as shown in SEQ ID NO. 19 or an amino acid sequence having at least 75% identity to the amino acid sequence as shown in SEQ ID NO. 19; and an amino acid sequence as shown in SEQ ID NO. 20 or an amino acid sequence having at least 75% identity to the amino acid sequence as shown in SEQ ID NO. 20;
(11) an amino acid sequence as shown in SEQ ID NO. 21 or an amino acid sequence having at least 75% identity to the amino acid sequence as shown in SEQ ID NO. 21; and an amino acid sequence as shown in SEQ ID NO. 22 or an amino acid sequence having at least 75% identity to the amino acid sequence as shown in SEQ ID NO. 22;
(12) an amino acid sequence as shown in SEQ ID NO. 23 or an amino acid sequence having at least 75% identity to the amino acid sequence as shown in SEQ ID NO. 23; and an amino acid sequence as shown in SEQ ID NO. 24 or an amino acid sequence having at least 75% identity to the amino acid sequence as shown in SEQ ID NO. 24;
(13) an amino acid sequence as shown in SEQ ID NO. 23 or an amino acid sequence having at least 75% identity to the amino acid sequence as shown in SEQ ID NO. 23; and an amino acid sequence as shown in SEQ ID NO. 25 or an amino acid sequence having at least 75% identity to the amino acid sequence as shown in SEQ ID NO. 25;
(14) an amino acid sequence as shown in SEQ ID NO. 23 or an amino acid sequence having at least 75% identity to the amino acid sequence as shown in SEQ ID NO. 23; and an amino acid sequence as shown in SEQ ID NO. 26 or an amino acid sequence having at least 75% identity to the amino acid sequence as shown in SEQ ID NO. 26;
(15) an amino acid sequence as shown in SEQ ID NO. 27 or an amino acid sequence having at least 75% identity to an amino acid sequence as set forth in SEQ ID NO. 27; and an amino acid sequence as shown in SEQ ID NO. 28 or an amino acid sequence having at least 75% identity to the amino acid sequence as shown in SEQ ID NO. 28;
(16) an amino acid sequence as shown in SEQ ID NO. 29 or an amino acid sequence having at least 75% of identity to the amino acid sequence as shown in SEQ ID NO. 29; and an amino acid sequence as shown in SEQ ID NO. 30 or an amino acid sequence having at least 75% of identity to the amino acid sequence as shown in SEQ ID NO. 30.

12. The antibody or fragment thereof according to any one of claims 6 to 11, wherein the antibody or fragment thereof is in any form, e.g., a monoclonal antibody, a single chain antibody, a diabody, a single domain antibody, a nanobody, a fully or partially humanized antibody, or a chimeric antibody and the like; alternatively, the antibody or fragment thereof is a half-antibody or an antigen-binding fragment of the half-antibody, e.g., scFv, BsFv, dsFv, (dsFv)₂, Fab, Fab', F(ab')₂, or Fv;
preferably, the antibody or fragment thereof further comprises a human or murine constant region, preferably a human or murine light chain constant region (CL) and/or heavy chain constant region (CH);
more preferably, the antibody or fragment thereof comprises a heavy chain constant region selected from the group consisting of IgG, IgA, IgM, IgD and IgE and/or a kappa or lambda type light chain constant region.

13. The antibody or fragment thereof according to any one of claims 6 to 12, wherein the antibody is a monoclonal antibody, preferably a murine, chimeric, or humanized monoclonal antibody; preferably, the heavy chain constant region of the monoclonal antibody is of IgG1 or IgG4 subtype and the light chain constant region of the monoclonal antibody is of kappa type;
preferably, the heavy chain constant region of the monoclonal antibody comprises an amino acid sequence as shown in SEQ ID NO: 124 or an amino acid sequence having at least 75% identity to the amino acid sequence as shown;
preferably, the light chain constant region of the monoclonal antibody comprises an amino acid sequence as shown in SEQ ID NO: 125 or an amino acid sequence having at least 75% identity to the amino acid sequence as shown.

14. A nucleic acid molecule comprising a nucleotide sequence encoding the antibody or fragment thereof according to any one of claims 6 to 13, or encoding a heavy chain CDR, a light chain CDR, a heavy chain variable region, a light chain variable region, a heavy chain or a light chain comprised in the antibody or fragment thereof.

15. A vector comprising the nucleic acid molecule according to claim 14.

16. A host cell comprising the nucleic acid molecule according to claim 14 and/or the vector according to claim 15, or transformed or transfected with the nucleic acid molecule according to claim 14 and/or the vector according to claim 15.

17. A conjugate or fusion protein comprising the antibody or fragment thereof according to any one of claims 6 to 13.

18. A pharmaceutical composition comprising the antibody or fragment thereof according to any one of claims 6 to 13, the nucleic acid molecule according to claim 14, the vector according to claim 15, the host cell according to claim 16, and/or the conjugate or fusion protein according to claim 17, and optionally a pharmaceutically acceptable excipient.

19. A kit comprising the antibody or fragment thereof according to any one of claims 6 to 13, the nucleic acid molecule according to claim 14, the vector according to claim 15, the host cell according to claim 16, the conjugate or fusion protein according to claim 17, and/or the pharmaceutical composition according to claim 18.

20. Use of the antibody or fragment thereof according to any one of claims 6 to 13, the nucleic acid molecule according to claim 14, the vector according to claim 15, the host cell according to claim 16, the pharmaceutical composition according to claim 17, and/or the conjugate or fusion protein according to claim 18 in the manufacture of a medicament for the prevention and/or treatment of cancer.

21. Use of the antibody or fragment thereof according to any one of claims 6 to 13, the nucleic acid molecule according to claim 14, the vector according to claim 15, the host cell according to claim 16, the pharmaceutical composition according to claim 17, and/or the conjugate or fusion protein according to claim 18 in the manufacture of an agent for the diagnosis of cancer.

22. Use of the antibody or fragment thereof according to any one of claims 6 to 13, the nucleic acid molecule according to claim 14, the vector according to claim 15, the host cell according to claim 16, the pharmaceutical composition according to claim 17, and/or the conjugate or fusion protein according to claim 18 in the manufacture of CAR-T cells.

23. A method for preventing and/or treating cancer, the method including administering to a subject in need thereof the antibody or fragment thereof according to any one of claims 6 to 13, the nucleic acid molecule according to claim 14, the vector according to claim 15, the host cell according to claim 16, the pharmaceutical composition according to claim 17, and/or the conjugate or fusion protein according to claim 18, and optionally other drugs or means.

24. A method for diagnosing cancer, the method including contacting the antibody or fragment thereof according to any one of claims 6 to 13, the nucleic acid molecule according to claim 14, the vector according to claim 15, the host cell according to claim 16, the pharmaceutical composition according to claim 17, and/or the conjugate or fusion protein according to claim 18 with a sample from a subject.
